# EUROPEAN PATENT SPECIFICATION

(11) **EP 1 622 867 B1**
(45) Date of publication and mention of the grant of the patent: **19.09.2007**
(21) Application number: 04730833.3
(22) Date of filing: 03.05.2004
(51) Int. Cl.: C07C 307/06, C07D 213/81, C07D 295/26, C07D 333/20, C07D 409/12, C07D 471/04, A61K 31/16, A61P 37/00, A61P 29/00

(54) **N-(2-PHENYLETHYL)SULFAMIDE DERIVATIVES AS INTEGRIN ALPHA4 ANTAGONISTS**
N-(2-PHENYLETHYL)SULFAMID-DERIVATE ALS INTEGRIN-ALPHA4-ANTAGONISTEN
DERIVES DE N-(2-PHENYLETHYL)SULFAMIDE ENTANT QU'ANTAGONISTES DE L'INTEGRINE ALPHA4

(30) Priority: 05.05.2003 ES 3001004
(43) Date of publication of application: 08.02.2006
(73) Proprietor: Laboratorios Almirall, S.A., 08022 Barcelona (ES)
(72) Inventor: JIMENEZ MAYORGA, Juan, Miguel, Abingdon Oxfordshire OX13 5DN (GB); VIDAL GISPERT, Laura, Third Floor, Flat 1a E-08028 Barcelona (ES); WARRELLOW, Graham, Seventh Floor, Flat 1a E-08017 Barcelona (ES)
(74) Representative: Srinivasan, Ravi Chandran
(86) International application number: PCT/EP2004/004670
(87) International publication number: WO 2004/099126

(56) References cited:
- EP-A- 1 323 709
- WO-A-99/64390
- WO-A-02/057242
- DATABASE WPI Section Ch, Week 200135 Derwent Publications Ltd., London, GB; Class B05, AN 2001-335810 XP002263936 -& WO 01/32610 A (KAKEN PHARMACEUTICAL) 10 May 2001 (2001-05-10)
- DATABASE WPI Section Ch, Week 200246 Derwent Publications Ltd., London, GB; Class B05, AN 2002-434874 XP002263935 -& WO 02/14272 A (KAKEN PHARMACEUTICAL) 21 February 2002 (2002-02-21)

## Description

The present invention relates to new therapeutically useful N-(2-phenylethyl)sulfamide derivatives, to processes for their preparation and to pharmaceutical compositions containing them. These compounds are antagonists of the α4 integrins, both the α4β1 integrin (VLA-4, "Very Late Antigen-4" or CD49d/CD29) and/or the α4β7 integrin (LPAM-1 and α4βp), thereby blocking the binding of α4β1 to its various ligands, such as VCAM-1, osteopontin and regions of fibronectin and/or the binding of α4β7 to its various ligands, such as MadCAM-1, VCAM-1 and fibronectin.

Through this mechanism of action the compounds of the invention inhibit cell (e.g. leukocyte) adhesion, activation, migration, proliferation and differentiation and are useful therefore in the treatment, prevention and suppression of immune or inflammatory disorders and, of other diseases mediated by α4β1 and/or α4β7 binding and/or by cell adhesion and activation, such as multiple sclerosis, asthma, allergic rhinitis, allergic conjunctivitis, inflammatory lung diseases, rheumatoid arthritis, septic arthritis, type I diabetes, rejection following organ transplantation, restenosis, rejection following autologous bone marrow transplantation, inflammatory sequelae of viral infections, atopic dermatitis, myocarditis, inflammatory bowel disease including ulcerative colitis and Crohn's disease, certain types of toxic and immune-based nephritis, contact dermal hypersensitivity, psoriasis, tumor metastasis, atherosclerosis and cerebral ischemia.

This invention also relates to compositions containing such compounds, to processes for their preparation, and to methods of treatment using such compounds. According to one aspect of the present invention we provide a particular group of compounds which are potent inhibitors of the binding of α4β1 and/or α4β7 integrins to their ligands.

Many physiological processes require that cells come into close contact with other cells and/or extracellular matrix. Such adhesion events may be required for cell activation, migration, proliferation and differentiation. Cell-cell and cell-matrix interactions are mediated through several families of Cell Adhesion Molecules (CAMs) including the selectins, integrins, cadherins and the immunoglobulins superfamily. CAMs play an essential role in both normal and pathophysiological processes. Therefore, the targeting of specific and relevant CAMs in certain disease conditions without interfering with normal cellular functions is essential for an effective and safe therapeutic agent that inhibits cell-cell and cell-matrix interactions. One family of adhesion molecules that is believed to play a particularly important role in regulating immune and inflammatory responses is the integrin family.

The integrin family is made up of structurally and functionally related glycoproteins consisting of α and β heterodimeric; transmembrane receptor molecules found in various combinations on nearly every mammalian cell type. (for reviews see: E.C. Butcher, Cell, 67, 1033 (1991); T.A. Springer, Cell, 76, 301 (1994); D. Cox et al., "The Pharmacology of Integrins", Medicinal Research Rev., 14, 195 (1995) and V.W. Engleman et al., "Cell Adhesion Integrins as Pharmaceutical Targets" in Ann. Repts. In Medicinal Chemistry, Vol. 31, J.A. Bristol, Ed.; Acad. Press, NY, 1996, p. 191). At least 14 different integrin α chains and 8 different integrin β chains have been identified (A. Sonnenberg, Current Topics in Microbiology and Immunology, 184, 7, (1993)). The members of the family are typically named according to their heterodimer composition although trivial nomenclature is widespread in this field. Thus the integrin termed α4β1 consists of the integrin α4 chain associated with the integrin β1 chain, and the integrin termed α4β7 consists of the integrin α4 chain associated with the integrin β7 chain. Not all the potential pairings of integrin α and β chains have yet been observed in nature and the integrin family has been subdivided based on' the pairings that have been recognised (A. Sonnenberg, ibid; S.A. Mousa et al., Drugs Discovery Today, 2, 187 (1997)).

One particular integrin subgroup of interest involves the a4 chain, which can pair with two different β chains, β1 and β7. α4β1 (VLA-4, "very late antigen-4"; or CD49d/CD29) is an integrin expressed on all leukocytes, except platelets, including dendritic cells and macrophage-like cells and is a key mediator of the cell-cell and cell-matrix interactions of these cell types (see M.E. "VLA Proteins in the Integrin Family: Structures, Functions, and their Role on Leukocytes." Ann. Rev. Immunol., 8, 365 (1990)). The ligands for α4β1 include vascular cell adhesion molecule-1 (VCAM-1), the CS-1 domain of fibronectin (FN) and osteopontin. VCAM-1 is a member of the Ig superfamily and is expressed in vivo on endothelial cells at sites of inflammation. (See R. Lobb et al., "Vascular Cell Adhesion Molecule-1" in Cellular and Molecular Mechanisms of Inflammation, C.G. Cochrane and M.A. Gimbrone, Eds.; Acad. Press, San Diego, 1993, p. 151). VCAM-1 is produced by vascular endothelial cells in response to pro-inflammatory cytokynes (see A.J. H. Gearing and W. Newman, "Circulating adhesion molecules in disease.", Immunol. Today, 14, 506 (1993)). The CS-1 domain is a 25 aminoacid sequence that arises by alternative splicing within a region of fibronectin. (For a review, see R.O. Hynes "Fibronectins", Springer-Verlag, NY, 1990). A role for α4β1/CS-1 interactions in inflammatory conditions has been proposed (see M.J. Elices, "The integrin α4β1 (VLA-4) as a therapeutic target" in Cell Adhesion and Human disease, Ciba Found. Symp., John Wiley & Sons, NY, 1995, p. 79). Osteopontin is expressed by a number of cell types including osteoclasts, osteoblasts, macrophages, activated T-cells, smooth muscle cells and epithelial cells (C.M. Giachelli et al., "Molecular and cellular biology of osteopontin: Potential role in cardiovascular disease", Trends Card. Med., 5, 88 (1995)).

α4β7 (also referred to as LPAM-1 and α4βp) is an integrin expressed on leukocytes and is a key mediator of leukocyte trafficking and homing in the gastrointestinal tract (see C.M. Parker et al., Proc. Nat. Acad. Sci. USA, 89, 1924 (1992)). The ligands for α4β7 include mucosal addressing cell adhesion molecule-1 (MadCAM-1) and, upon activation of α4β7, VCAM-1 and fibronectin (Fn). MadCAM-1 is a member of the lg superfamily and is expressed *in vivo* on endothelial cells of gut-associated mucosal tissues of the small and large intestine ("Peyer's Patches") and lactating mammary glands. (See M.J. Briskin et al., Nature, 363, 461 (1993); A. Hammann et al., J. Immunol., 152, 3282 (1994)). MadCAM-1 can be induced *in vitro* by proinflammatory stimuli (See E.E. Sikarosky et al., J. Immunol., 151, 5239 (1993)). MadCAM-1 is selectively expressed at sites of lymphocyte extravasation and specifically binds to the integrin α4β7.

Neutralising anti-α4 antibodies or blocking peptides that inhibit the interaction between α4β1 and/or α4β7 and their ligands have proven efficacious both prophylactically and therapeutically in several animal models of inflammation and in humans (X.-D. Yang et al., Proc. Nat. Acad. Sci. USA, 90, 10494 (1993), P.L. Chisholm et al., Eur. J. Immunol., 23, 682 (1993), T.A. Yednock et al., Nature, 356, 63 (1992), R.R. Lobb et al., J. Clin. Invest., 94, 1722 (1994), J. Relton, Drug News Perspect., 14, 346 (2001), N. Turbridy et al., Neurology, 53, 466 (1999)). The primary mechanism of action of such antibodies appears to be the inhibition of lymphocyte and monocyte interactions with CAMs associated with components of the extracellular matrix and vascular endothelium, thereby limiting leukocyte migration to extravascular sites of injury or inflammation and/or limiting the priming and/or activation of leukocytes.

Since the discovery of their key role in mediating inflammatory pathophysiology, α4β1 and α4β7 have received considerable attention as drug design targets. Important advances have been made in identifying potent and selective candidates for further development strongly suggesting that α4β1 and α4β7 should be tractable small molecule targets (S.P. Adams et al., "Inhibitors of Integrin Alpha 4 Beta 1 (VLA-4)" in Ann. Repts. In Medicinal Chemistry, Vol. 34, W.K. Hagmann, Ed.; Acad. Press, NY, 1999, p. 179).

There still remains a need for low molecular weight, specific inhibitors of α4β1 and α4β7-dependent cell adhesion that have improved pharmacokinetic and pharmacodynamic properties such as oral bioavailability and significant duration of action. Such compounds would be useful for the treatment, prevention or suppression of various pathologies mediated by α4β1 and α4β7 binding and cell adhesion and activation.

Compounds with related structures have been described as metalloprotease inhibitors.

The PCT patent applications number WO 00/67746, WO 00/51974, WO 00/43415, WO 00/73260, WO 98/58902, WO 98/04247, WO 99/26921, WO 98/53818 and WO 00/71572 disclose compounds that inhibit the binding of α4β1 and/or α4β7 integrins to their receptors and their use in the treatment or prevention of diseases mediated by α4β1 and/or α4β7 binding and/or by cell adhesion and activation, such as multiple sclerosis, asthma, allergic rhinitis, allergic conjunctivitis, inflammatory lung diseases, rheumatoid arthritis, septic arthritis, type I diabetes, rejection following organ transplantation, restenosis, rejection following autologous bone marrow transplantation, inflammatory sequelae of viral infections, atopic dermatitis, myocarditis, inflammatory bowel disease including ulcerative colitis and Crohn's disease, certain types of toxic and immune-based nephritis, contact dermal hypersensitivity, psoriasis, tumor metastasis, atherosclerosis and cerebral ischemia. WO 02/057242 discloses urea derivatives as integrin α₄β₁ and/or α₄β₇ antagonists.

We have now found that a novel series of N-(2-phenylethyl)sulfamide derivatives are potent and selective antagonists of the α4 integrins, both the α4β1 integrin and/or the α4β7 integrin and are therefore useful in the treatment or prevention of these pathological conditions, diseases and disorders.

The compounds of the present invention can also be used in combination with other drugs known to be effective in the treatment of these diseases. For example, they can be used in combination with retinoids, vitamin D analogues, steroids, PUVA/UVB, cyclosporine, methotrexate, anti-TNF-α or phosphodiesterase 4 inhibitors in the treatment of psoriasis, since these compounds have complementary mechanisms of action to α4β1 integrin antagonist.

The present invention provides a compound according to Formula I: 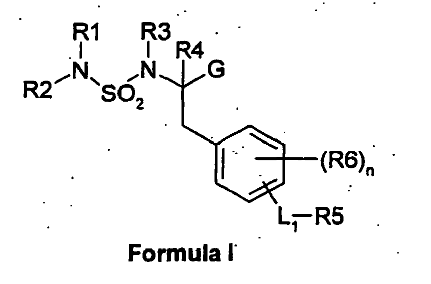 wherein:
- G is a COOH group or a tetrazolyl group
- R1 and R2 are independently selected from hydrogen atoms and alkyl, alkenyl, alkynyl, cycloalkyl, cycloalkylalkyl, cycloalkylalkenyl, cycloalkylalkynyl, cycloalkenyl, cycloalkenylalkyl, cycloalkenylalkenyl, cycloalkenylalkynyl, heterocyclyl, heterocyclylalkyl, heterocyclylalkenyl, heterocyclylalkynyl, aryl, arylalkyl, - arylalkenyl, arylalkynyl, heteroaryl, heteroarylalkyl, heteroarylalkenyl, or heteroarylalkynyl groups;
   or R1_{.} and R2 form, together with the nitrogen atom to which they are attached, either a 3- to 14- membered monocyclic or polycyclic heterocyclic ring system or a 5- to 14-membered heteroaryl group wherein said groups comprise from 1 to 5 heteroatoms selected from nitrogen, oxygen and sulphur;
   wherein said alkyl, alkenyl, and alkynyl groups or moieties are unsubstituted or substituted with one to four substituents, which may be the same or different and are independently selected from Ra;
   and wherein said cycloalkyl, heterocyclyl, aryl and heteroaryl groups or moieties are unsubstituted or substituted with one to four substituents, which may be the same or different and are independently selected from Rb;
- R3 and R4 are independently selected from hydrogen atoms and alkyl groups having from 1 to 6 carbon atoms;
- R5 is selected from the group consisting of 6- to 14- membered monocyclic or polycyclic aryl groups and 5- to 14- membered monocyclic or polycyclic heteroaryl groups comprising from 1 to 5 heteroatoms selected from nitrogen, oxygen and sulphur,
   wherein said aryl and heteroaryl groups or moieties are unsubstituted or substituted with one to four substituents, which may be the same or different and are independently selected from Rb;
- R6 is a group selected from -OH, -ORc, -NO₂, halogen, -S(O)Rc, -S(O)₂Rc, -SRc,-S(O)₂ORc, -S(O)NRcRc -S(O)₂NRcRc, -NRcRc, -O(CRcRc)mNRcRc, -C(O)Rc,-CO₂Rc, -CO₂(CRcRc)mCONRcRc, -OC(O)Rc, -CN, -C(O)NRcRc, -NRcC(O)Rc,-OC(O)NRcRc, -NRcC(O)ORc, -NRcC(O)NRcRc, -CRc(N-ORc), -CFH₂, -CF₂H, -Ra,-CF₃, alkyl, alkenyl and alkynyl;
- n is an integer from 0 to 3;
- Ra is a group selected from alkyl, -OH,-ORc,-NO₂, halogen,-S(O)Rc, -S(O)₂Rc, -SRc,-S(O)₂ORc, S(O) NRcRc, -S(O)₂NRcRc, -NRcRc, -O(CRcRc)mNRcRc, -C(O)Rc,-CO₂Rc, -CO₂ (CRcRc)mCONRcRc, -OC(O)Rc, -CN, -C(O)NRcRc, -NRcC(O)Rc,-OC(O) NRcRc, -NRcC(O)ORc, -NRcC(O)NRcRc, -CRc(N-ORc), -CFH₂, - CF₂H, Ra, or -CF₃ ; wherein if two or more Rc groups are present these may be the same or different;
- Rb is a group selected from -OH, -ORd,-NO₂, halogen, -S(O)Rd, -S(O)₂Rd ,- SRd,-S(O)₂ORd,- S(O)NRdRd, -S(O)₂NRdRd, -NRdRd,- O(CRdRd)mNRdRd, -C(O)Rd,-CO₂Rd, -CO2(CRdRd)mCONRdRd, -OC(O)Rd, -CN, -C(O)NRdRd, -NRdC(O)Rd,-OC(O)NRdRd, -NRdC(O)ORd, -NRdC(O)NRdRd, -CRd(N-ORd), -CFH₂, -CF₂H, Ra,-CF₃, alkyl, alkenyl, C₂₋₄alkynyl, cycloalkyl, cycloalkylalkyl, heterocyclyl, heterocyclylalkyl, aryl, arylalkyl, heteroaryl or heteroarylalkyl; wherein said alkyl, alkenyl, alkynyl, cycloalkyl, heterocyclyl, aryl and heteroaryl groups or moieties are unsubstituted or substituted with one to four substituents which may be the same or different and are independently selected from Ra;
- L1 is either a direct bond or a group selected from the group consisting of -N(Rc)-, -O-, -N(Rc)CO-, -CON(Rc)-, -O(CO)N(Rc)- and -N(Rc)(CO)O-;
- Rc is a hydrogen atom or an alkyl group having from 1 to 4 carbon atoms;
- Rd is alkyl, alkenyl, alkynyl, cycloalkyl, cycloalkylalkyl, cycloalkylalkenyl, cycloalkylalkynyl, cycloalkenyl, cycloalkenylalkyl, cycloalkenylalkenyl, cycloalkenylalkynyl, heterocyclyl, heterocyclylalkyl, heterocyclylalkenyl, heterocyclylalkynyl, aryl, arylalkyl, arylalkenyl, arylalkynyl, heteroaryl, heteroarylalkyl, heteroarylalkenyl, or heteroarylalkynyl;
   wherein said alkyl, alkenyl, alkynyl, cycloalkyl, cycloalkenyl, heterocyclyl, aryl and heteroaryl groups are unsubstituted or substituted with one to four substituents, which may be the same or different and are independently selected from Re;
- Re is a group selected from alkyl, -OH, -ORc, -NO₂, halogen, -S(O)Rc, -S(O)₂Rc,-SRc, -S(O)₂ORc, -S(O)NRcRc, -S(O)₂NRcRc, -NRcRc, -O(CRcRc)mNRcRc, -C(O)Rc, -CO₂Rc, -CO₂(CRcRc)mCONRcRc; -OC(O)Rc, -CN, -C(O)NRcRc, -NRcC(O)Rc,-OC(O)NRcRc, -NRcC(O)ORc, -NRcC(O)NRcRc, -CRc(N-ORc), -CFH₂, -CF₂H, -Ra, or -CF₃; wherein if two or more Rc groups are present these may be the same or different;
and any pharmaceutically acceptable salt thereof as well as any compound resulting from the esterification, with any alcohol, of the carboxylic group in the case where G is such a carboxylic group and any pharmaceutically acceptable salt thereof.

Further objectives of the present invention are to provide processes for preparing said compounds; pharmaceutical compositions comprising an effective amount of said compounds; the use of the compounds in the manufacture of a medicament for the treatment of diseases susceptible of being improved by inhibition of the binding of α4β1 and/or α4β7 integrins to their receptors; and methods of treatment of diseases susceptible to amelioration by inhibition of the binding of α4β1 and/or α4β7 integrins to their receptors, which methods comprise the administration of the compounds of the invention to a subject in need of treatment.

As used herein (either alone or within other terms such as cycloalkylalkyl, cycloalkenylalkyl, heterocyclylalkyl, arylalkyl and heteroarylalkyl), the term alkyl embraces optionally substituted, linear or branched radicals having 1 to 20 carbon atoms or, preferably 1 to 12 carbon atoms. More preferably alkyl radicals are "lower alkyl" radicals having 1 to 8, preferably 1 to 6 and more preferably 1 to 4 carbon atoms.

Examples include methyl, ethyl, n-propyl, i-propyl, n-butyl, sec-butyl and tert-butyl, n-pentyl, 1-methylbutyl, 2-methylbutyl, isopentyl, 1-ethylpropyl, 1,1-dimethylpropyl, 1,2-dimethylpropyl, n-hexyl or 1-ethylbutyl, 2-ethylbutyl, 1,1-dimethylbutyl, 1,2-dimethylbutyl, 1,3-dimethylbutyl, 2,2-dimethylbutyl, 2,3-dimethylbutyl, 2-methylpentyl, 3-methylpentyl, iso-hexyl radicals.

As used herein (either alone or within other terms such as cycloalkylalkenyl, cycloalkenylalkenyl, heterocyclylalkenyl, arylalkenyl and heteroarylalkenyl), the term alkenyl embraces optionally substituted, linear or branched, mono or polyunsaturated radicals having 2 to 20 carbon atoms or, preferably, 2 to 12 carbon atoms. More preferably alkenyl radicals are "lower alkenyl" radicals having 2 to 8, preferably 2 to 6 and more preferably 2 to 4 carbon atoms. In particular it is preferred that the alkenyl radicals are mono or diunsaturated.

Examples include vinyl, allyl, 1-propenyl, isopropenyl, 1-butenyl, 2-butenyl, 3-butenyl, 1-pentenyl, 2-pentenyl, 3-pentenyl and 4-pentenyl radicals.

As used herein (either alone or within other terms such as cycloalkylalkynyl, cycloalkenylalkynyl, heterocyclylalkynyl, arylalkynyl and heteroarylalkynyl), the term alkynyl embraces optionally substituted, linear or branched, mono or polyunsaturated radicals having 2 to 20 carbon atoms or, preferably, 2 to 12 carbon atoms. More preferably, alkynyl radicals are "lower alkynyl" radicals having 2 to 8, preferably 2 to 6 and more preferably 2 to 4 carbon atoms. In particular, it is preferred that the alkynyl radicals are mono or diunsubstituted.

Examples include 1-propynyl, 2-propynyl, 1-butynyl, 2-butynyl and 3-butynyl radicals.

As used herein (either alone or within other terms such as arylalkyl, arylalkenyl and arylalkynyl), the term aryl radical embraces typically a C₆-C₁₄ monocyclic or polycyclic aryl radical such as phenyl or naphthyl, anthranyl or phenanthryl. Phenyl is preferred. When an aryl radical carries 2 or more substituents, the substituents may be the same or different.

As used herein, the term heteroaryl (either alone or within other terms such as heteroarylalkyl, heteroarylalkenyl and heteroarylalkynyl), radical embraces typically a 5- to 14- membered ring system comprising, at least one heteroaromatic ring and containing at least one heteroatom selected from O, S and N. A heteroaryl radical may be a single ring or two or more fused rings wherein at least one ring contains a heteroatom.

Examples include pyridyl, pyrazinyl, pyrimidinyl, pyridazinyl, furyl, oxadiazolyl, oxazolyl, imidazolyl, thiazolyl, thiadiazolyl, thienyl, pyrrolyl, pyridinyl, benzothiazolyl, indolyl, indazolyl, purinyl, quinolyl, isoquinolyl, phthalazinyl, naphthyridinyl, quinoxalinyl, quinazolinyl, quinolizinyl, cinnolinyl, triazolyl, indolizinyl, indolinyl, isoindolinyl, isoindolyl, indolyl, indazolyl, purinyl, imidazolidinyl, pteridinyl and pyrazolyl radicals.

Oxadiazolyl, oxazolyl, pyridyl, pyrrolyl, imidazolyl, thiazolyl, thiadiazolyl, thienyl, furanyl, pyrazinyl and pyrimidinyl radicals are preferred.

When a heteroaryl radical carries 2 or more substituents, the substituents may be the same or different.

As used herein (either alone or within other terms such as cycloalkylalkyl, cycloalkylalkenyl and cycloalkylalkynyl), the term cycloalkyl embraces saturated carbocyclic radicals and, unless otherwise specified, a cycloalkyl radical typically has from 3 to 7 carbon atoms.

Examples include cyclopropyl, cyclobutyl, cyclopentyl, cyclohexyl and cycloheptyl. It is preferably cyclopropyl, cyclopentyl or cyclohexyl. When a cycloalkyl radical carries 2 or more substituents, the substituents may be the same or different.

As used herein (either alone or within other terms such as cycloalkenylalkyl, cycloalkenylalkenyl and cycloalkenylalkynyl), the term cycloalkenyl embraces partially unsaturated carbocyclic radicals and, unless otherwise specified, a cycloalkenyl radical typically has from 4 to 7 carbon atoms.

Examples include cyclobutenyl, cyclopentenyl, cyclohexenyl and cycloheptenyl. It is preferably cyclopentenyl or cyclohexenyl. When a cycloalkenyl radical carries 2 or more substituents, the substituents may be the same or different.

As used herein (either alone or within other terms such as heterocyclylalkyl, heterocyclylalkenyl and heterocyclylalkynyl), the term heterocyclyl radical embraces typically a non-aromatic, saturated or unsaturated, monocyclyc or polycyclyc, C₃-C₁₄ carbocyclic ring system, such as a 5, 6 or 7 membered radical, in which one or more, for example 1, 2, 3 or 4 of the carbon atoms preferably 1 or 2 of the carbon atoms are replaced by a heteroatom selected from N, O and S. Saturated heterocyclyl radicals are preferred. A heterocyclic radical may be a single ring or two or more fused rings wherein at least one ring contains a heteroatom. When a heterocyclyl radical carries 2 or more substituents, the substituents may be the same or different.

Examples of heterocyclic radicals include piperidyl, pyrrolidyl, pyrrolinyl, piperazinyl, morpholinyl, thiomorpholinyl, pyrrolyl, azepanyl, pyrazolinyl, pirazolidinyl, quinuclidinyl, triazolyl, pyrazolyl, tetrazolyl, cromanyl, isocromanyl, imidazolidinyl, imidazolyl, oxiranyl, azaridinyl, 4,5-dihydro-oxazolyl and 3-aza-tetrahydrofuranyl. Where a heterocyclyl radical carries 2 or more substituents, the substituents may be the same or different.

In one embodiment of the present invention G is a COOH group as well as any compound resuming from the esterification, with an alcohol, of the COOH group, preferably a free COOH group and salts thereof.

In another embodiment of the present invention R3 and R4 are hydrogen atoms.

Typically, R1 and R2 are independently selected from hydrogen atoms and alkyl, cycloalkyl, heterocyclylalkyl, aryl, arylalkyl, heteroarylalkyl groups, wherein said alkyl; cycloalkyl, heterocyclyl, aryl and heteroaryl groups or moieties are unsubstituted or substituted.

Preferably, R1 and R2 form, together with the nitrogen atom to which they are attached, either a 5- to 8- membered monocyclic heterocyclic ring system, wherein said ring system comprise from 1 to 4 heteroatoms selected from nitrogen, oxygen and sulphur and is unsubstituted or substituted..

In another embodiment of the present invention R5 is selected from the group consisting of 6- to 14- membered monocyclic or polycyclic aryl and 5- to 14- membered monocyclic or polycyclic heteroaryl groups comprising from 1 to 5 heteroatoms selected from nitrogen, oxygen and sulphur wherein said aryl and heteroaryl groups or moieties are unsubstituted or substituted; said aryl or heteroaryl groups being preferably unsubstituted or substituted by one or more halogen atoms.

In another embodiment of the present invention L1 is a group selected from -NH-, -O- and -NHCO-.

In still another embodiment of the present invention R5-L1- is selected from the group comprising benzamide, isonicotinamide, 2,6-naphthyridin-1-ylamino, 2,7-naphthyridin-1-ylamino; 2,6-naphthyridin-1-yloxy and 2,7-naphthyridin-1-yloxy wherein said groups are unsubstituted or substituted.

In another embodiment of the present invention n is zero.

Preferred compounds of formula I have an S-configuration at the carbon atom alpha to the G group.

Particularly preferred compounds of formula I include:
- (2*S*)-2-([(tert-butylamino)sulfonyl]amino}-3-{4-[(3,5-dichloroisonicotinoyl)amino]phenyl}propionic acid
- Methyl (2*S*)-2-(*N*-benzylaminosulfonylamino)-3-[4-(2,6-dichlorobenzoylamino)phenyl]propionate
- (2*S*)-2-(*N*-benzylaminosulfonylamino)-3-[4-(2,6-dichlorobenzoylamino)phenyl]propionic acid
- Methyl (2*S*)-3-{4-[(2,6-dichlorobenzoyl)amino]phenyl}-2-{[(dimethylamino)sulfonyl]amino} propionate
- (2*S*)-3-{4-[(2,6-dichlorobenzoyl)amino]phenyl)-2-{[(dimethylamino)sulfonyl]amino) propionic acid
- Methyl (2*S*)-3-{4-[(3,5-dichloroisonicotinoyl)amino]phenyl}-2-{[(dimethylamino)sulfonyl]amino}propionate
- (2*S*)-3-{4-[(3,5-dichloroisonicotinoyl)amino]phenyl}-2-{[(dimethylamino)sulfonyl]amino}propionic acid
- Methyl (2*S*)-3-(4-{[1-(2,6-Dichlorophenyl)methanoyl]amino)phenyl)-2-(piperidine-1-sulfonylamino)propionate
- (2*S*)-3-(4-{[1-(2,6-Dichlorophenyl)methanoyl]amino)phenyl)-2-(piperidine-1-sulfonylamino)propionic acid
- Methyl (2*S*)-3-{4-[(3,5-dichloroisonicotinoyl)amino]phenyl}-2-{[(diisobutylamino)sulfonyl]amino}propionate
- (2*S*)-3-{4-[(3,5-dichloroisonicotinoyl)amino]phenyl}-2-{[(diisobutylamino)sulfonyl]amino}propionic acid
- Methyl (2*S*)-2-({[benzyl(ethyl)amino]sulfonyl}amino)-3-{4-[(3,5-dichloroisonicotinoyl)amino]phenyl}propionate
- (2*S*)-2-{[(benzylethylamino)sulfonyl]amino}-3-{4-[(3,5-dichloroisonicotinoyl)amino]phenyl}propionic acid
- Methyl (2*S*)-3-{4-[(3,5-dichloroisonicotinoyl)amino]phenyl)-2-{[(dibutylamino)sulfonyl]amino}propionate
- (2*S*)-3-(4-[(3,5-dichloroisonicotinoyl)amino]phenyl)-2-{[(dibutylamino)sulfonyl]amino}propionic acid
- Methyl (2*S*)-3-{4-[(3,5-dichloroisonicotinoyl)amino]phenyl)-({[[2-(3,4-dimethoxyphenyl)ethyl]isobutylamino]sulfonyl}amino)propionate
- (2*S*)-3-{4-[(3,5-dichloroisonicotinoyl)amino]phenyl)-2-({[[2-(3,4-dimethoxyphenyl)ethyl]isobutylamino]sulfonyl}amino)propionic acid
- Methyl (2*S*)-2-({[bis(thien-2-ylmethyl)amino]sulfonyl}amino)-3-{4-[(3,5-dichloroisonicotinoyl)amino]phenyl}propionate
- (2*S*)-2-({[bis(thien-2-ylmethyl)amino]sulfonyl}amino)-3-{4-[(3,5-dichloroisonicotinoyl)amino]phenyl}propionic acid
- Methyl (*2S*)-3-{4-[(3,5-dichloroisonicotinoyl)amino]phenyl}-2-({[methyl(2-pyridin-2-ylethyl)amino]sulfonyl}amino)propionate
- (*2S*)-3-{4-[(3,5-dichloroisonicotinoyl)amino]phenyl}-2-({[methyl(2-pyridin-2-ylethyl)amino]sulfonyl}amino)propionic acid
- Methyl (2*S*)-2-{[(cyclohexylmethylamino)sulfonyl]amino}-3-[4-[(3,5-dichloroisonicotinoyl)amino]phenyl}propionate
- (2*S*)-2-{[(cyclohexylmethylamino)sulfonyl]amino}-3-{4-[(3,5-dichloroisonicotinoyl)amino]phenyl}propionic acid
- Methyl (2*S*)-3-{4-[(3,5-dichloroisonicotinoyl)amino]phenyl}-2-({[(3-methylbutyl)(thien-2-ylmethyl)amino]sulfonyl}amino)propionate
- (2*S*)-3-{4-[(3,5-dichloroisonicotinoyl)amino]phenyl}-2-({[(3-methylbutyl)(thien-2-ylmethyl)amino]sulfonyl}amino)propionic acid
- Methyl (2*S*)-3-{4-[(3,5-dichloroisonicotinoyl)amino]phenyl}-2-[(piperidin-1-ylsulfonyl)amino]propionate
- (2*S*)-3-{4-[(3,5-dichloroisonicotinoyl)amino]phenyl}-2-[(piperidin-1-ylsulfonyl)amino]propionic acid
- Methyl (2*S*)-2-[(azepan-1-ylsulfonyl)amino]-3-{4-[(3,5-dichloroisonicotinoyl)amino]phenyl}propionate
- (2*S*)-2-[(azepan-1-ylsulfonyl)amino]-3-{4-[(3,5-dichloroisonicotinoyl)amino]phenyl}propionic acid
- Methyl (2*S*)-3-{4-[(3,5-dichloroisonicotinoyl)amino]phenyl}-2-[(morpholin-4-ylsulfonyl)amino]propionate
- (*2S*)-3-{4-[(3,5-dichloroisonicotinoyl)amino]phenyl}-2-[(morpholin-4-ylsulfonyl)amino]propionic acid
- Methyl (2*S*)-3-{4-[(3,5-dichloroisonicotinoyl)amino]phenyl}-2-[(thiomorpholin-4-ylsulfonyl)amino]propionate
- (2*S*)-3-{4-[(3,5-dichloroisonicotinoyl)amino]phenyl}-2-[(thiomorpholin-4-ylsulfonyl)amino]propionic acid
- Methyl (2*S*)-2-{[(dimethylamino)sulfonyl]amino}-3-[4-(2,6-naphthyridin-1-yloxy)phenyl]propionate
- (2*S*)-2-{[(dimethylamino)sulfonyl]amino}-3-[4-(2,6-naphthyridin-1-yloxy)phenyl]propionic acid
- Methyl (2*S*)-2-{[(diisobutylamino)sulfonyl]amino}-3-[4-(2,6-naphthyridin-1-yloxy)phenyl]propionate
- (2*S*)-2-{[(diisobutylamino)sulfonyl]amino}-3-[4-(2,6-naphthyridin-1-yloxy)phenyl]propionic acid
- Methyl-(2*S*)-2-([(diisobutylamino)sulfonyl]amino}-3-[4-(2,6-naphthyridin-1-ylamino)phenyl]propionate
- (2*S*)-2-{[(diisobutylamino)sulfonyl]amino}-3-[4-(2,6-naphthyridin-1-ylamino)phenyl]propiortic acid
- Methyl (2*S*)-3-{4-[(2,6-dichlorobenzoyl)amino]phenyl}-2-{[(2,6-dimethylpiperidin-1-yl)sulfonyl]amino}propionate
- (2*S*)-3-(4-[(2,6-dichlorobenzoyl)amino]phenyl}-2-{[(2,6-dimethylpiperidin-1-yl)sulfonyl]amino}propionic acid
- Methyl (2*S*)-3-{4-[(3,5-dichloroisonicotinoyl)amino]phenyl}-2-{[(diisopropylamino)sulfonyl]amino}propionate
- (2*S*)-3-{4-[(3,5-dichloroisonicotinoyl)amino]phenyl}-2-{[(diisopropylamino)sulfonyl]amino}propionic acid
- Methyl (2*S*)-3-{4-[(3,5-dichloroisonicotinoyl)amino]phenyl}-2-{[(2,6-dimethylpiperidin-1-yl)sulfonyl]amino}propionate
- (2*S*)-3-{4-[(3,5-dichloroisonicotinoyl)amino]phenyl}-2-{[(2,6-dimethylpiperidin-1-yl)sulfonyl]amino}propionic acid
- Methyl (2*S*)-2-{[(dimethylamino)sulfonyl]amino}-3-(4-(2,6-naphthyridin-1-ylamino)phenyl]propionate
- (2*S*)-2-{[(dimethylamino)sulfonyl]amino}-3-[4-(2,6-naphthyridin-1-ylamino)phenyl]propionic acid
- Methyl (2*S*)-2-{[(diisopropylamino)sulfonyl]amino}-3-[4-(2,6-naphthyridin-1-ylamino)phenyl]propionate
- (2*S*)-2-{((diisopropylamino)sulfonyl]amino}-3-[4-(2,6-naphthyridin-1-ylamino)phenyl]propionic acid
- Methyl (2*S*)-2-({[cyclohexyl(isopropyl)amino]sulfonyl)amino)-3-[4-(2,6-naphthyridin-1-ylamino)phenyl]propionate
- (2*S*)-2-({[cyclohexyl(isopropyl)amino]sulfonyl}amino)-3-[4-(2,6-naphthyridin-1-ylamino)phenyl]propionic acid
- Methyl (2*S*)-2-{[(diisopropylamino)sulfonyl]amino}-3-[4-(2,6-naphthyridin-1-yloxy)phenyl]propionate
- (2*S*)-2-{[(diisopropylamino)sulfonyl]amino}-3-[4-(2,6-naphthyridin-1-yloxy)phenyl]propioriic acid
- Methyl (2S)-2-{[(diisopropylamino)sulfonyl]amino}-3-[4-(2,6-naphthyridin-1-ylamino)phenyl]propionate
- (2*S*)-2-{((diisopropylamino)sulfonyl]amino}-3-[4-(2,6-naphthyridin-1-ylamino)phenyl]propionic acid
- Methyl (2*S*)-2-{[(2,6-dimethylpiperidin-1-yl)sulfonyl]amino}-3-[4-(2,6-naphthyridin-1-y)amino)pheny)]pr6pionate
- (2*S*)-2-{[(2,6-dimethylpiperidin-1-yl)sulfonyl]amino}-3-[4-(2,6-naphthyridin-1-ylamino)phenyl]propionic acid
- Methyl (2*S*)-2-{[(2,6-dimethylpiperidin-1-yl)sulfonyl]amino}-3-[4-(2,6-naphthyridin-1-yloxy)phenyl]propionate
- (2*S*)-2-{[(2,6-dimethylpiperidin-1-yl)sulfonyl]amino}-3-[4-(2,6-naphthyridin-1-yloxy)phenyl]propionic acid
- Methyl (2*S*)-2-({[benzyl(isopropyl)amino]sulfonyl}amino)-3-{4-[(2,6-dichlorobenzoyl)amino]phenyl}propionate
- (2*S*)-2-({[benzyl(isopropyl)amino]sulfonyl}amino)-3-{4-[(2,6-dichlorobenzoyl)amino]phenyl}propionic acid
- Methyl (2*S*)-2-({[benzyl(isopropyl)amino]sulfonyl}amino)-3-{4-[(3;5-dichloroisonicotinoyl)amino]phenyl}propionate
- (2*S*)-2-({[benzyl(isopropyl)amino]sulfonyl}amino)-3-{4-[(3,5-dichloroisonicotinoyl)amino]phenyl}propionic acid
- Methyl (2*S*)-2-({[isopropyl(thien-2-ylmethyl)amino]sutfonyl}amino)-3-{4-[(3,5-dichloroisonicotinoyl)amino]phenyl}propionate
- (2*S*)-2-({[isopropyl(thien-2-ylmethyl)amino]sulfonyl}amino)-3-{4-[(3,5-dichloroisonicotinoyl)amino]phenyl}propionic acid
- Methyl (2*S*)-2-({[isopropy)(thien-2-ylmethyl)amino]sulfonyl}amino)-3-{4-[(3,5-dichlorobenzoyl)amino]phenyl}propionate
- (2*S*)-2-({[isopropyl(thien-2-ylmethyl)amino]sulfonyl}amino)-3-{4-[(3,5-dichlorobenzoyl)amino]phenyl}propionic acid
- Methyl (2S)-3-{4-[(2,6-dichloroisonicotinoyl)amino]phenyl}-2-[({isobutyl[(1*S*)-1-phenylethyl]amino}sulfonyl)amino]propionate
- (2*S*)-3-{4-[(2,6-dichloroisonicotinoyl)amino]phenyl}-2-[({isobutyl[(1*S*)-1-phenylethyl]amino}sulfonyl)amino]propionic acid
- Methyl (2*S*)-2-({[cyclopentyl(isopropyl)amino]sulfonyl}amino)-3-{4-[(3,5-dichloroisonicotinoyl)amino]phenyl}propionate
- (2*S*)-2-({[cyclopentyl(isopropyl)amino]suffonyl}amino)-3-{4-[(3,5-dichloroisonicotinoyl)amino]phenyl}propionic acid
- Methyl (2S)-3-{4-[(3,5-dichloroisonicotinoyl)amino]phenyl}-2-({[isobutyl(isopropyl)amino]sulfonyl}amino)propionate
- (2*S*)-3-{4-[(3,5-dichloroisonicotinoyl)amino]phenyl}-2-({[isobutyl(isopropyl)amino]sulfonyl}amino)propionic acid
- Methyl (2S)-2-({[cyclohexyl(isopropyl)amino]sulfonyl}amino)-3-[4-(2,6-naphthyridin-1-yloxy)phenyl]propionate
- (2*S*)-2-({[cyclohexyl(isopropyl)amino]sulfonyl}amino)-3-(4-(2,6-naphthyridin-1-yloxy)phenyl]propionic acid
- Methyl (2*S*)-3-(4-[(3,5-dichloroisonicotinoyl)amino]phenyl}-2-[({isobutyl[(1R)-1-phenylethyl]amino}sulfonyl)amino]propionate .
- (2*S*)-3-{4-[(3,5-dichtoroisonicotinoyl)amino]phenyl}-2-(({isobutyl[(1R)-1-phenylethyl]amino}sulfonyl)amino]propionic acid
- Methyl (2*S*)-2-({[methyl(phenyl)amino]sulfonyl}amino)-3-{4-[(2,6-dichlorobenzoyl)amino]phenyl}propionate
- (2*S*)-2-({[methyl(phenyl)amino]sulfonyl}amino)-3-{4-[(2,6-dichlorobenzoyl)amino]phenyl}propionic acid
- Methyl (2*S*)-({[2-(phenylsulfonyl)phenyl]amino}sulfonyl)amino]-3-{4-[(3,5-dichloroisonicotinoyl)amino]phenyl}propionate
- (2*S*)-({[2-(phenylsulfonyl)phenyl]amino}sulfonyl)amino]-3-{4-[(3,5-dichloroisonicotinoyl)amino]phenyl}propionic acid

The present invention also provides processes for preparing a compound of the invention. Thus a compound of Formula I in which Z is a -COOH group may be obtained by hydrolysis of an ester of formula (II): 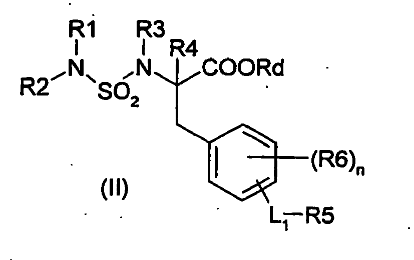 where Rd is selected from the group comprising C₁₋₆alkyl and aryl-C₁₋₄alkyl, preferably methyl, ethyl, isopropyl, tert-butyl and benzyl.

The hydrolysis may be performed using either an acid or a base depending on the nature of Rd, for example a base such as lithium, sodium or potassium hydroxide in an aqueous organic solvent mixture such as methanol, ethanol, tetrahydrofuran, diethyl ether, dioxane at a temperature of from 20°C to 100°C. In the case of acid hydrolysis in an acid such as trifluoroacetic acid the reaction is performed at room temperature.

Additionally, compounds of Formula I in which Z is a tetrazole group may be obtained by standard conditions treating the corresponding nitrile derivative of fomula (XV): 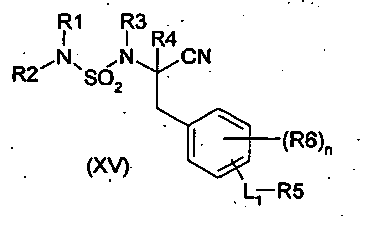 with sodium azide or tributyltinazide in an inert organic solvent such as dimethylformamide, toluene, xylene, tetrahydrofuran, e.g. in the presence, in some cases, of an acid such as ammonium chloride, e.g. at a temperature of from room temperature to 140°C.

The nitrile derivatives of formula (XV) may be prepared from the corresponding primary amide by methods known *per se,* e.g. Z. Groznka, et al. Roczniki Chemii Ann. Soc. Chim. Polonorum (1971), 45, 967. The primary amides may be obtained by standard conditions. By way of illustration, an ester of formula (II) can be treated with saturated solution of ammonia in methanol, ethanol or dioxane, e.g. at room temperature to provide the corresponding primary amide.

A number of different synthetic schemes are available for the preparation of the esters of formula (II). Five of these schemes are explained in detail under the headings Scheme 1 to Scheme 5.

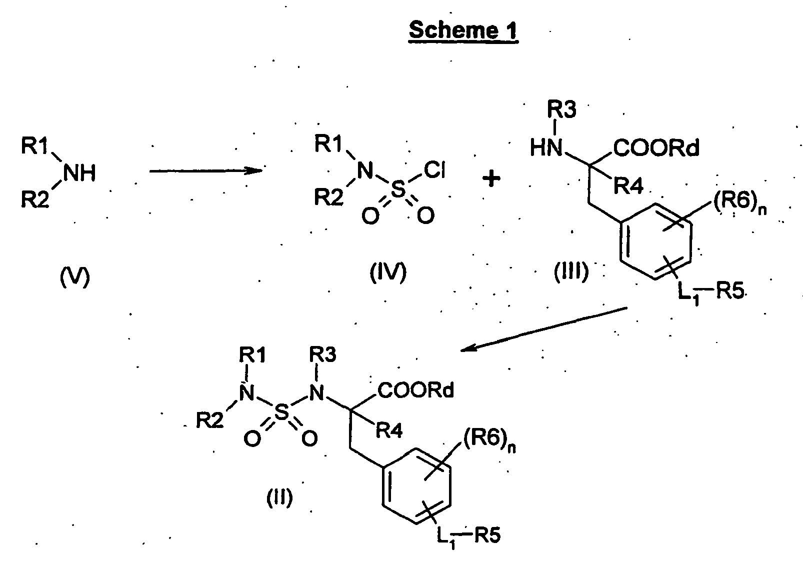

Esters of formula (II), wherein the R1, R2 and R3 are as described above, may be prepared by reaction of the corresponding amine of formula (III) or a salt thereof with a corresponding sulfamoyl chloride, of formula (IV).

The reaction may be carried out under standard conditions for this type of reaction in the presence of a base such as triethylamine, diisopropylethyl amine, DBU, e. g. in an inert organic solvent such as dichloromethane, tetrahydrofuran, dioxane at a temperature of from 0°C to 70°C.

When the sulfamoyl chloride (IV) is not commercially available, these compounds could be prepared by standard conditions treating the corresponding amine (V) with sulfuryl chloride, fuming sulfuric acid or chlorosulfonic acid in the presence of a base such as pyridine, triethylamine, diisopropylethyl amine, e. g. followed by treatment with phosphorous pentachloride in an inert organic solvent such as dichloromethane, chloroform, benzene, toluene, e.g. at a temperature of from 0°C to 80°C.

Amines of formula (V) that are not commercially available, wherein R1 and R2 are different from H, could be prepared from the corresponding primary amines by a reductive alkylation process employing the corresponding aldehyde and a borohydride, for example sodium triacetoxyborohydride or sodium cyanoborohydride, in a solvent such as dichloromethane, acetone, ethanol, methanol, trimethylorthoformate, in the presence of an acid, where necessary, such as acetic acid at a temperature of from room temperature to 80°C. Alternatively, an alkylation process could be performed using the corresponding halide, sulphonate, sulphate derivative, e.g. preferably in an inert organic solvent such as toluene, dioxane, tetrahydrofuran, acetone, methyl isobutyl ketone, dimethylformamide, e.g. and in the presence of a base such as triethylamine, diisopropylethylamine, DBU, potassium carbonate, sodium hydroxide, cesium hydroxide, e.g. at a temperature of from room temperature to 130°C.

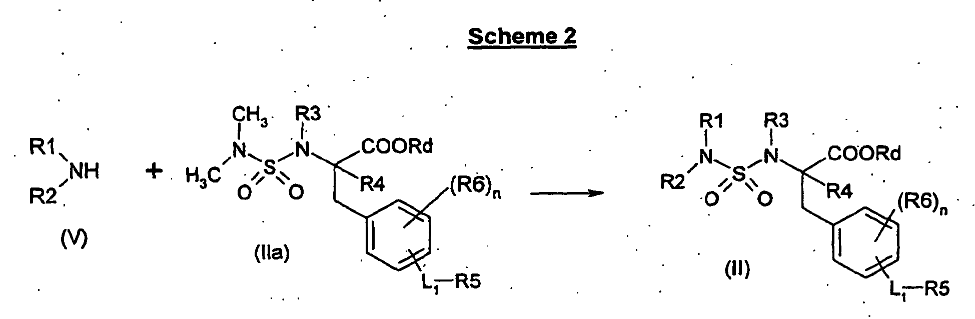

Alternatively, esters of formula (II) wherein R1, R2 and R3 is as described above, may be prepared by reaction of the corresponding amine (V) with a dimethyl sulfamide of formula (IIa).

The reaction between the amine (V) and sulfamide (IIa) may be carried out in an inert organic solvent such as pyridine, acetonitrile, dioxane, tetrahydrofuran, toluene, 1,1,2-trichloroethane e.g. at a temperature of from 50 °C to 130°C.

The sulfamide of formula (IIa) may be obtained by reacting a corresponding amine (III) with dimethylsulfamoyl chloride in the presence of a base such as pyridine, triethylamine, diisopropylethyl amine, DBU, e.g. in an inert organic solvent such as pyridine, dichloromethane, tetrahydrofuran, dioxane at a temperature of from 0°C to room temperature.

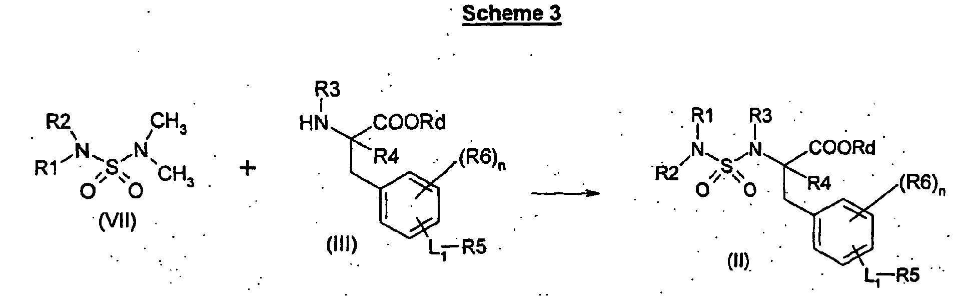

Additionally, ester of formula (II) wherein R1, R2 and R3 is as described above, may be prepared by reaction of the corresponding amine of formula (III) or a salt thereof with a corresponding sulfamide of formula (VII).

The reaction between the amine (III) and sulfamide (VII) may be carried out in an inert organic solvent such as pyridine, acetonitrile, dioxane, tetrahydrofuran, toluene, 1,1,2-trichloroethane e.g. at a temperature of from 50 °C to 130°C.

The sulfamide of formula (VII) may be obtained by reacting a corresponding amine (V) with dimethylsulfamoyl chloride in the presence of a base such as pyridine, triethylamine, diisopropylethyl amine, DBU, e. g. in an inert organic solvent such as pyridine, dichloromethane, tetrahydrofuran, dioxane at a temperature of from 0°C to room temperature.

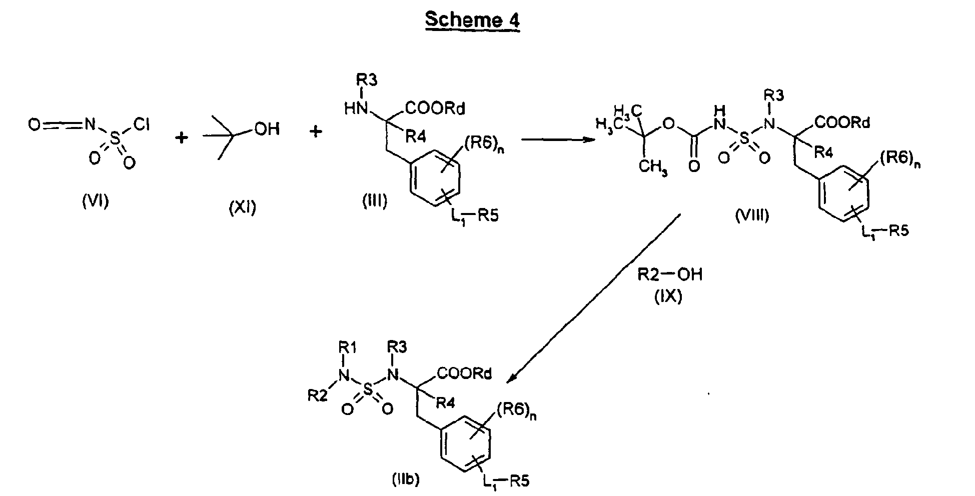

Esters of formula (II), wherein the R1 is hydrogen and R2 and R3 are as described above, may be prepared by reaction of the corresponding sulfamide of formula (VIII) with a corresponding alcohol of formula (IX):

The reaction may be carried out under standard Mitsunobu conditions in the presence of a phosphine such as triphenylphosphine, tributylphosphine, and an azodicarbonyl derivative such as DEAD, DIAD, ADDP, e.g. in an inert organic solvent such as tetrahydrofuran, dioxane, diethylether at a temperature of from 0°C to 100°C.

The sulfamide of formula (VIII) may be obtained by reacting a corresponding amine (III) with *N*-(*tert*-butoxycarbonyl)-*N*-[4-(dimethylazaniumylidene)-1,4-dihydropyridin-1-ylsulfonyl]azanide (prepared as described by Jean-Yves Winum et al. in Organic Letters 2001, 3, 2241) in the presence of a base such as pyridine, triethylamine, diisopropylethylamine, DBU, e. g. in an inert organic solvent such as tetrahydrofuran, dioxane, diethylether, e. g. at a temperature of from 0°C to 100°C.

Additionally, the reaction may be performed by reacting the amine (III) with chlorosulfonyl isocyanate and *tert*-butanol in the presence of a base such as pyridine, triethylamine, diisopropylamine, DBU, e. g. in an inert organic solvent such as dichloromethane, tetrahydrofuran, dioxane at a temperature of from -20°C to room temperature.

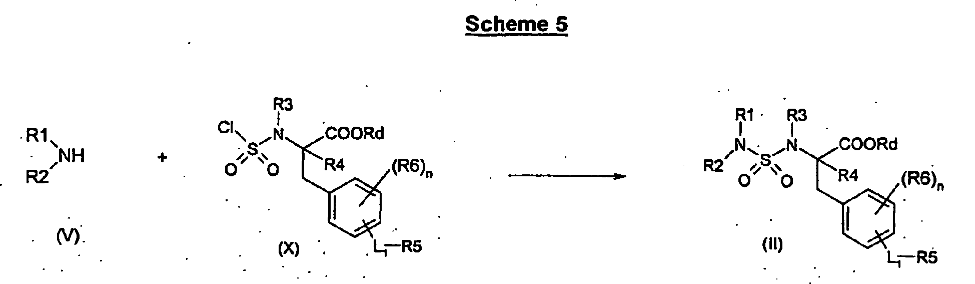

Alternatively, esters of formula (II) wherein R1, R2 and R3 is as described above, may be prepared by reaction of the corresponding amine (V) with sulfamoyl chloride of formula (X).

The reaction may be carried out under standard conditions for this type of reaction in the presence of a base such as triethylamine, diisopropylethylamine, DBU, e. g. in an inert organic solvent such as dichloromethane, tetrahydrofuran, dioxane at a temperature of from 0°C to 70°C.

The sulfamoyl chloride of formula (X) may be obtained by reacting a corresponding amine (III) with sulfuryl chloride, fuming sulfuric acid or chlorosulfonic acid in the presence of a base such as pyridine, triethylamine, diisopropylethyl amine, e. g. followed by treatment with phosphorous pentachloride in an inert organic solvent such as dichloromethane, chloroform, benzene, toluene, e.g. at a temperature of from 0°C to 80°C.

The aminoesters of formula (III) where R3 is not hydrogen can be prepared under reductive amination reaction conditions by reacting a corresponding alpha-amino acetate (XI) 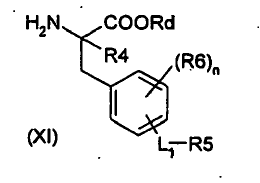 with an aldehyde or ketone in the presence of a reducing agent (e.g. sodium cyanoborohydride, sodium triacetoxyborohydride, and the like) and an organic acid (e.g., glacial acetic acid, trifluoroacetic acid, and the like) at room temperature. Suitable solvents for the reaction are halogenated hydrocarbons (e.g, ,1,2-dichloroethane, chloroform, and the like)

The intermediates of formula (III) and amines of formula (V) are known compounds or may be prepared from known starting materials by methods well known in the field of organic chemistry and described in particular in the following publications: WO 98/58902, WO99/36393, WO99/43642, WO00/43372, WO00/73260, WO01/79173, WO01/32610 and WO02/20522.

In any of the heregoing general description of the synthesis of compounds of Formula I, intermediate compounds at any stage may contain protecting groups to protect functionalities which would otherwise react under the conditions described. Such protecting groups are added and removed at appropiate stages during the synthesis of compounds of Formula I and the chemistries of such protections and deprotections are well described in the prior art (for example: T.W. Green and P.G.M. Wuts, "Protecting Groups in Organic Synthesis"; John Wiley and Sons, Inc.; Third Edition, 1999).

Where it is desired to obtain a particular enantiomer of a compound of Formula I this may be produced from the corresponding mixture of enantiomers using a suitable conventional procedure for resolving enantiomers. Thus for example, diastereomeric derivatives, e.g. salts, may be produced by reaction of a mixture of enantiomers of Formula I e.g. a racemate, and an appropiate chiral compound, e.g. a chiral base. The diastereomers may then be separated by any convenient means, for example by crystallisation and the desired enantiomer recovered, e.g. by treatment with an acid in the instance where the diastereomer is a salt. In another resolution process a racemate of Formula I may be separated using chiral High Performance Liquid Chromatography.

Alternatively, if desired, a particular enantiomer may be obtained by using an appropiate chiral intermediate in one of the processes described above.

The compounds of Formula I can be converted by methods known per se into pharmaceutically acceptable salts by reaction with an alkali metal hydroxide such as sodium or potassium hydroxide or an organic base. The acid or alkali addition salts so formed may be interchanged with suitable pharmaceutically acceptable counterions using processes known per se.

Also compounds of Formula I in which there is the presence of an basic group may be converted into pharmacologically acceptable salts, preferably acid addition salts by treatment with organic or inorganic acids such as fumaric, tartaric, citric, succinic or hydrochloric acid

### Pharmacological action

The compounds of the present invention are useful in treating a subject afflicted with a pathological condition susceptible to amelioration by antagonism of α4β1 and/or α4β7 integrins, which comprises administering to the subject an effective amount of a compound of the invention, or a pharmaceutically acceptable salt thereof, as well as in the manufacture of a medicament for the treatment of a pathological condition susceptible of being improved or prevented by antagonism of α4β1 and/or α4β7 integrins. Such pathological conditions include multiple sclerosis, asthma, allergic rhinitis, allergic conjunctivitis, an inflammatory lung disease, rheumatoid arthritis, polydermatomyositis, septic arthritis, type 1 diabetes, rejection following organ transplantation, restenosis, rejection following autologous bone marrow transplantation, inflammatory sequelae of viral infections, atopic dermatitis, myocarditis, inflammatory bowel disease including ulcerative colitis and Chron's disease, contact dermal hypersensitivity, psoriasis, tumor metastasis, atherosclerosis or cerebral ischemia.

Those of skill in the art are well aware of the pathological conditions susceptible to amerlioration by antagonism of α4β1 and/or α4β7 integrins. Such conditions include, for example, conditions susceptible to amelioration by administration of a known anti-α4 antibody. The compounds of the invention can therefore be used to ameliorate any pathological condition susceptible to amelioration by an anti-α4 antibody.

The following assays demonstrate the activity of the compounds.

### U-937 cell adhesion to human VCAM-1 (α4β1 binding assay)

Recombinant human soluble VCAM-1 (R&D Systems Ltd., UK) at 2 µg/ml in PBS was immobilized overnight onto microtiter plates. Unbound VCAM-1 was washed away and VCAM-1 coated plates were blocked with bovine serum albumin (BSA) 2,5 % in PBS for 2h at room temperature. U-937 cells were labelled with 5-carboxyfluorescein diacetate (5-CFDA) in order to detect bound cells to the wells. Test compounds were added to the wells followed by U-937 cells and the adhesion assay was performed for 1h at 37°C. Following incubation, the wells were emptied and washed. Inhibition of binding was measured by the quantity of fluorescence bound to the plate for each of the various concentrations of test compound, as well as for controls containing no test compound, with a Cytofluor 2300 fluorescence measurement system.

### RPMI 8866 cell adhesion to mouse MAdCAM-1 (α4β7 binding assay)

Recombinant mouse MAdCAM-1 was coated on a 96-well, plate overnight. Unbound MAdCAM-1 was washed away and plates were blocked with 0,5 % BSA. Cells were labelled with BCECF-AM and added to ligand-coated wells. Test compounds were added to the wells followed by RPMI 8866 and the adhesion assay was performed for 45 min at room temperature. Following incubation, the wells were emptied and washed. Inhibition of binding was measured by the quantity of fluorescence bound to the plate for each of the various concentrations of test compound, as well as for controls containing no test compound, with a Cytofluor 2300 fluorescence measurement system.

Compounds of the invention generally have IC50 values in the α4β1 assays below 10 µM. The compounds of the Examples typically had IC50 values of 1 µM. and below. Most preferred compounds of the current invention displayed IC50 values of below 100 nM in one or both of the adhesion assays.

The following Examples have IC50 values in the α4β1 assays between 100 nM and 1 µM: 1, 7, 11, 13, 15, 17, 19, 21, 25, 27, 29, 31, 33, 41, 43, 45, 49, 53, 55, 57, 59, 61, 63, 65 and 73

The following Examples have IC50 values in the α4β31 assays below 100 nM: 3, 51, 69, 71, 77 and 81.

The ability of the compounds of Formula I to antagonize the actions of α4β1 and/or α4β7 integrins make them useful for inhibiting cell (e.g. leukocyte) adhesion processes, including cell activation, migration, proliferation and differentiation; thus preventing or reversing the symptoms of immune or inflammatory disorders and of other pathological conditions known to be mediated by the binding of α4β1 and/or α4β7 to their various respective ligands. The subject in need of treatment is typicallly a mammal, in particular a human.

Preferably, said pathological condition, disease or disorder is selected from multiple sclerosis, asthma, allergic rhinitis, allergic conjunctivitis, inflammatory lung diseases, rheumatoid arthritis, polydermatomyositis, septic arthritis, type I diabetes, rejection following organ transplantation, restenosis, rejection following autologous bone marrow transplantation, inflammatory sequelae of viral infections, atopic dermatitis, myocarditis, inflammatory bowel disease including ulcerative colitis and Chron's disease, contact dermal hypersensitivity, psoriasis, tumor metastasis, atherosclerosis and cerebral ischemia.

The magnitude of prophylactic or therapeutic dose of a compound of Formula I will, of course, vary with the nature of the severity of the condition to be treated and with the particular compound of Formula I and its route of administration. It will also vary according to the age, weight and response of the individual patient. In general, the daily dose range lie within the range of from about 0.001 mg to about 100 mg per kg body weight of a mammal, preferably 0.01 mg to about 50 mg per Kg, and most preferably 0.1 to 10 mg per kg, in single or divided doses. On the other hand, it may be necessary to use dosages outside these limits in some cases.

Any suitable route of administration may be employed for providing a mammal, especially a human, with an effective dosage of a compound of the present invention. For example, oral, rectal, topical, parenteral, ocular, pulmonary, nasal, and the like may be employed. Dosage forms include tablets, troches, dispersions, suspensions, solutions, capsules, creams, ointments, aerosols, and the like.

Another aspect of the present invention provides pharmaceutical compositions comprising a a compound of the invention, or a pharmaceutically acceptable salt thereof. Accordingly, the use of compounds of the present invention may also involve pharmaceutical compositions which oomprise any compound of the invention, or a pharmaceutically acceptable salt thereof.

The term "composition", as in pharmaceutical composition, is intended to encompass a product comprising the active ingredient(s), and the inert ingredient(s) (pharmaceutically acceptable excipients) that make up the carrier, as well as any product which results, directly or indirectly, from combination, complexation or aggregation of any two or more of the ingredients, or from dissociation of one or more of the ingredients. Accordingly, the pharmaceutical compositions of the present invention encompass any composition made by admixing a compound of the invention additional active ingredient(s), and pharmaceutically acceptable excipients.

The expression "pharmaceutical acceptable salts" refers to salts prepared from pharmaceutically acceptable non-toxic bases or acids including inorganic bases or acids and organic bases or acids.

The compositions include compositions suitable for oral, rectal, topical, parenteral (including subcutaneous, intramuscular, and intravenous), ocular (opthalmic), pulmonary (aerosol inhalation), or nasal administration, although the most suitable route in any given case will depend on the nature and severity of the conditions being treated and on the nature of the active ingredient. They may be conveniently presented in unit dosage form and prepared by any of the methods well-known in the art of pharmacy.

In practical use, the compounds of the invention can be combined as the active ingredient in intimate admixture with a pharmaceutical carrier according to conventional pharmaceutical compounding techniques. The carrier may take a wide variety of forms depending on the form of preparation desired for administration, e.g., oral or parenteral (including intravenous). In preparing the compositions for oral dosage form, any of the usual pharmaceutical media may be employed, such as, for example, water, glycols, oils, alcohols, flavouring agents, preservatives, coloring agents and the like in the case of oral liquid preparations, such as, for example, suspensions, elixirs and solutions; or carriers such as starches, sugars, microcrystalline cellulose, diluents, granulating agents, lubricants, binders, disintegrating agents and the like in the case of oral solid preparations such as, for example, powders, capsules and tablets, with the solid oral, preparations being preferred over the liquid preparations. Because of their ease of administration, tablets and capsules represent the most advantageous oral dose unit form in which case solid pharmaceutical carriers are obviously employed. If desired, tablets may be coated by standard aqueous or nonaqueous techniques.

Compositions for parenteral injection may be prepared from soluble salts, which may be or may not be freeze-dried and which may be dissolved in pyrogen free aqueous media or other appropriate parenteral injection fluid.

For administration by inhalation, the compounds of the present invention are conveniently delivered in the form of an aerosol spray presentation from pressurized packs of nebulisers. The compounds may also be delivered as powders, which may be formulated and the powder composition may be inhaled with the aid of an insufflation powder inhaler device. The preferred delivery systems for inhalation are metered dose inhalation (MDI) aerosol, which may be formulated as a suspension or solution of compound of Formula, I in suitable propellants, such as fluorocarbons or hydrocarbons and dry powder inhalation (DPI) aerosol, which may be formulated as a dry powder of a compound of Formula I with or without additional excipients.

Suitable topical formulations of a compound of the invention include transdermal devices, aerosols, creams, gels, ointments, lotions, dusting powders, and the like.

### EXAMPLES

The syntheses of the compounds of the invention and of the intermediates for use therein are illustrated by the following . Examples, including Preparation Examples (Preparations 1 and 2), which do not limit the scope of the invention in any way.

### PREPARATION 1:

### Methyl (2S)-2-({[(tert-butoxycarbonyl)amino]sulfonyl}amino)-3-{4-[(2,6-dichlorobenzoyl)amino]phenyl}propionate

To a suspension of methyl (2S)-2-amino-3-{4-[(2,6-dichlorobenzoyl)amino]phenyl} propionate hydrochloride and *N*-(*tert-*butoxycarbonyl)-*N*-[4-(dimethylazaniumylidene)-1,4-dihydropyridin-1-ylsulfonyl]azanide in tetrahydrofuran (10 mL) was added triethylamine (0.25 g, 2.48 mmoL) and the mixture was heated at 60°C overnight. The solvent was removed, the crude was dissolved in ehtyl acetate and washed with hydrochloric acid 1N (25 mL) and brine (25 mL). The organic extracts were dried over sodium sulphate, filtered and evaporated. The residue was purified by flash chromatography (hexanes:ethyl acetate, 1:1) to afford the title compound (0.63 g, 40%) as a colourless oil.

δ (DMSO-d₆): 10.90 (s, 1H), 10.70 (s, 1H), 8.40 (d, 1H), 7.55 (m, 5H), 7.20 (d, 2H), 4.15 (m, 1 H), 3.60 (s, 3H), 2.90 (m, 2H), 1.40 (s, 9H).

### PREPARATION 2

### Methyl (2S)-2-{[(dimethylamino)sulfonyl]amino}-3-(4-nitrophenyl)propionate

To a solution of dimethylsulfamoyl chloride (10.9. g, 76.16 mmoL) in pyridine (55 mL) under nitrogen atmosphere was added a solution of methyl (2S)-2-amino-3-(4-nitrophenyl)propionate (4.27 g, 19.04, mmoL) in pyridine (55 mL) dropwise at 0°C. The mixture was stirred at room temperature for 16h. The solvent was removed, the crude was dissolved in ethyl acetate and washed with hydrochloric acid 0.5 N (100 mL) and brine (100 mL). The organic extracts were dried over sodium sulphate, filtered and evaporated. The residue was purified by flash chromatography (hexanes:ethyl acetate, 1:1) to afford the title compound (3.51 g, 56%) as a white solid.

δ (DMSO-d₆): 8.15 (d, 2H), 7.95 (s, 1H), 7.50 (d, 2H), 3.95 (m, 1H), 3.60 (s, 3H), 3.00 (m, 2H), 2.30 (s, 6H).

### EXAMPLE 1

### (2S)-2-{[(tert-butylamino)sulfonyl]amino}-3-{4-[(3,5-dichloroisonicotinoyl)amino]phenyl}propionic acid

To a solution of methyl (2*S*)-2-amino-3-{4-[(3,5-dichloroisonicotinoyl)amino] phenyl}propionate (0.25 g, 0.62 mmoL) and triethylamine (0.31 g, 3.09 mmoL) in methylene chloride (5 mL) under nitrogen atmosphere was added a solution of 2-methylpropane-2-sulfonyl chloride (0.32g, 1.85 mmoL) (prepared as described in J. A. Kloek and K. L. Leschinsky J. Org. Chem. 1976, 41, 4028) in methylene chloride (4 mL) dropwise. The mixture was stirred at room temperature for 16h. The volatiles, were removed *in vacuo* and the residue was partitioned between ethyl acetate (50 mL) and water (100 mL). The organic extracts were dried over sodium sulphate, filtered and evaporated. The residue was purified by flash chromatography (methylene chloride:ethyl acetate, 1:1) to afford the title compound (0.05 g, 16%) as a white solid.

A solution of the solid above (0.05 g, 0.1. mmoL) and LiOH.H₂O (6 mg, .0.24 mmoL) in tetrahydrofuran (2 mL) and H₂O (2 mL) was stirred at room temperature for 2h. The organic solvent was removed under reduced pressure and the resulting aqueous solution was acidified with hydrochloric acid until pH 6. The precipitate was collected by filtration to obtain the title compound (0.04 g, 82%) as a white solid.

δ (CDCl₃): 8.62 (bs, 1H), 7.95 (s, 1H), 7.56 (d, 2H), 7.18 (d, 2H), 4.34 (m, 1H), 3.15 (m, 2H), 1.26 (s, 9H).

### EXAMPLE 2

### Methyl (2S)-2-(N-benzylaminosulfonylamino)-3-[4-(2,6dichlorobenzoylamino)phenyl] propionate

To a solution of Preparation 1 (1 g, 1.59 mmoL), benzyl alcohol (0.99 g, 9.52 mmoL) and tributylphosphine (0.79 mL, 3.18 mmoL) in THF (5 mL) at 0° under an argon atmosphere was added a solution of 1,1'-(azodicarbonyl)dipiperidine (0.80 g, 3.18 mL) in THF (4 mL) dropwise. The mixture was stirred at room temperature for 24h and at 60° for an additional 24h, and concentrated under reduced pressure. The residue was purified by flash chromatography (hexanes:ethyl acetate, 3:2) to afford the title compound (0.12g, 14%) as a colourless oil.

δ (DMSO-d₆): 11.31 (s, 1H), 10.74 (s, 1H), 8.62 (bs, 1H), 7.60 (m, 5H), 7.38 (m, 5H), 7.19 (d, 2H), 5.05 (s, 2H), 4.08 (m, 1 H), 3.54 (s, 3H); 2.91 (m, 2H).

### EXAMPLE 3

### (2S)-2-(N-benzylaminosulfonylamino)-3-[4-(2,6dichlorobenzoylamino)phenyl] propionic acid

A solution of the crude above (0.19 g, 0.36 mmoL) and LiOH.H₂O (36 mg, 0.85 mmoL) in tetrahydrofuran (4 mL) and H₂O (4 mL) was stirred at room temperature for 2h. The organic solvent was removed under reduced pressure and the resulting aqueous solution was acidified with citric acid until pH 2. The precipitate was collected by filtration to obtain the title compound (0.08 g, 43%) as a white solid.

m.p.: 157°C

δ (DMSO-d₆): 10.68 (s, 1 H), 7.49 (m, 5H), 7.33 (m, 5H), 7.18 (d, 2H), 4.96 (s, 2H), 3.95 (m, 1H), 3.00 (m, 1 H), 2.80 (m, 1H).

### EXAMPLE 4

### Methyl (2S)-3-{4-[(2,6-dichlorobenzoyl)amino]phenyl}-2-{[(dimethylamino)sulfonyl] amino}propionate

To a solution of dimethylsulfamoyl chloride (0.7 g, 4.91 mmoL) in pyridine (5 mL) under nitrogen atmosphere was added a solution of methyl (2S)-2-amino-3-{4-[(2,6-dichlorobenzoyl)aminolphenyllpropionati (0.45 g, 1.23 mmoL) in pyridine (5 mL) dropwise at 0°C. The mixture was stirred at room temperature for 16h. The solvent was removed, the crude was dissolved in ehtyl acetate and washed with hydrochloric acid 0.5 N (100 mL) and brine (100 mL). The organic extracts were dried' over sodium sulphate, filtered and evaporated. The residue was purified by flash chromatography (hexanes:ethyl acetate, 1:1) to afford the title compound (0.33 g, 28%) as a white solid.

δ (DMSO-d₆): 10.64 (s, 1 H), 7.85 (bs, 1 H), 7.52 (m, 5H), 7.18 (d, 2H), 3.85 (m, 1H), 3.52 (s, 3H), 2.78 (m,' 2H), 2.34 (s, 6H).

### EXAMPLE 5

### (2S)-3-{4-[(2,6-dichlorobenzoyl)amino]phenyl}-2-{{(dimethylamino)sulfonyl]amino} propionic acid

To a solution of the solid above (0.072 g, 0.015 mmoL) in tetrahydrofuran (2 mL) was added NaOH 2N (2 mL) and stirred at room temperature for 2h. The organic solvent was removed under reduced pressure and the resulting aqueous solution was acidified with citric acid until pH 2. The precipitate was collected by filtration to obtain the title compound (0.07 g, 70%) as a white solid.

m.p.: 186°C

δ (DMSO-d₆): 10.70 (s, 1H), 7.72 (d, 1H), 7.58 (m, 5H), 7.26 (d, 2H), 3.84 (m, 1H), 2.93 (m, 1 H), 2.80 (m, 1H), 2.43 (s, 6H).

### EXAMPLE 6

### Methyl (2S)-3-{4-[(3,5-dichloroisonicotinoyl)amino]phenyl)-2-([(dimethylamino) sulfonyl]amino}propionate

The title compound was obtained as a white solid from methyl (2S)-2-amino-3-{4-[(3,5-dichloroisonicotinoyl)amino]phenyl}propionate following the same procedure described in Example 4.

δ (CDCl₃): 8.59 (s, 2H), 7.85 (s, 1H), 7.54 (d, 2H), 7.18 (d, 2H), 4.20 (d, 1H), 4.24 (m, 1H), 3.78 (s, 3H), 3.14 (m, 2H), 2.64 (s, 6H).

### EXAMPLE 7

### (2S)-3-{4-[(3,5-dichloroisonicotinoyl)amino}phenyl}-2-{[(dimethylamino)sulfonyl] amino}propionic acid

The title compound (81%) was prepared from the compound of Example 6 by hydrolysis in a similar manner to Example 5.

m.p.: 197°C

δ (DMSO-d₆): 12.85 (bs, 1H), 10.89 (s, 1H), 8.80 (s, 2H), 7.72 (d, 1H), 7.58 (d, 2H), 7.28 (d, 2H), 3.84 (m, 1 H), 2.97 (m, 1 H), 2.80 (m, 1H), 2.43 (s, 6H).

### EXAMPLE 8

### Methyl (2S)-3-(4-{[1-(2,6-Dichlorophenyl)methanoyl]amino}phenyl)-2-(piperidine-1-sulfonylamino)propionate

A mixture of compound from Example 4 (0.1 g, 0.211 mmoL) and piperidine (0.18 g, 2.11 mmoL) in dioxan (1 mL) was heated under reflux 20h. The solvent was removed, the crude was dissolved in ehtyl acetate and washed with hydrochloric acid 2 N (10 mL) and brine (10 mL). The organic extracts were dried over sodium sulphate, filtered and evaporated. The residue was purified by flash chromatography (methylene chloride:ethyl acetate, 10:1) to afford the title compound (0.03 g, 26%) as a white solid.

δ (DMSO-d₆): 10.72 (s; 1H), 7.94 (d, 1H), 7.58 (m, 5H), 7.24 (d, 2H), 3.84 (m, 1H), 3.64 (s, 3H), 2.85 (m, 2H), 2.48 (m, 4H), 1.24 (m, 6H).

### EXAMPLE 9

### (2S)-3-(4-{[1-(2,6-dichlorophenyl)methanoyl]amino}phenyl)-2-(piperidine-1-sulfonylamino)propionic acid

The title compound (95%) was prepared from the compound of Example 8 by hydrolysis in a similar manner to Example 5.

δ (DMSO-d₆): 10.76 (s, 1H), 7.92 (d, 1H), 7.54 (m, 5H), 7.26 (d, 2H), 3.86 (m, 1H), 2.92 (m, 2H), 2.50 (m, 4H), 1.27 (m, 6H).

### EXAMPLE 10

### Methyl (2S)-3-{4-[(3,5-dichloroisonicotinoyl)amino]phenyl}-2-[{(diisobutylamino) sulfonyl]amino}propionate

The title compound was obtained as a white solid from the compoud of Example 6 and diisobutylamine following the same procedure described in Example 8.

δ (CDCl₃): 8.53 (s, 2H); 8.00 (s, 1H), 7.54 (d, 2H), 7.18 (d, 2H), 5.02 (d, 1H), 4.22 (m, 1H), 3.79 (s, 3H), 3.05 (m, 2H), 2.82 (d, 4H), 1.94 (m, 2H), 0.86 (d, 12H).

### EXAMPLE 11

### (2S)-3-{4-[(3,5-dichloroisonicotinoyl)amino]phenyl}-2-{[(diisobutylamino)sulfonyl] amino}propionic acid

The title compound (65%) was prepared from the compound of Example 10 by hydrolysis in a similar manner to Example 5.

m.p.: 209°C

δ (DMSO-d₆): 12.81 (bs, 1H), 10.89 (s, 1H), 8.80 (s, 2H), 7.58 (d, 2H), 7.48 (d, 1H), 7.28 (d, 2H), 3.76 (m, 1H), 2.97 (m, 1H), 2.74 (m, 1H), 2.44 (d, 4H), 1.67 (m, 1H), 0.80 (d, 12H).

### EXAMPLE 12

### Methyl (2S)-2-({[benzyl(ethyl)amino]sulfonyl)amino)-3-{4-[(3,5-dichloroisonicotinoyl) amino]phenyl}propionate

The title compound was obtained as a white solid from the compoud of Example 6 and benzylethylamine following the same procedure described in Example 8.

δ (DMSO-d₆): 8.80 (s, 2H), 7.79 (d, 1H), 7.59 (d, 2H), 7.24 (m, 7H), 3.89 (s, 2H), 3.81 (m, 1H), 3.57 (s, 3H), 2.97 (m,1H), 2.79 (m, 3H), 0.80 (t, 3H).

### EXAMPLE 13

### (2S)-2-{[(benzylethylamino)sulfonyl]amino}-3-{4-[(3,5-dichloroisonicotinoyl)amino] phenyl}propionic acid

The title compound (94%) was prepared from the compound of Example 12 by hydrolysis in a similar manner to Example 5.

m.p.: 190°C

δ (DMSO-d₆): 12.82 (bs, 1H), 10.91 (s, 1H), 8.81 (s, 2H), 7.81 (d, 1H), 7.59 (d, 2H), 7.26 (m, 7H), 3.90 (s, 2H), 3.83 (m, 1H), 2.97 (m, 1H), 2.80 (m, 3H), 0.803 (t, 3H).

### EXAMPLE 14

### Methyl (2S)-3-{4-[(3,5-dichloroisonicotinoyl)amino]phenyl}-2-{[(dibutylamino) sulfonyl]amino}propionate

The title compound was obtained as a white solid from the compoud of Example 6 and dipentylamine following the same procedure described in Example 8.

δ (CDCl₃): 8.50 (s, 2H), 8.10 (s, 1H). 7.55 (d, 2H), 7.19 (d, 2H), 5.05 (d, 1H). 4.19 (m, 1H), 3.80 (s, 3H), 3.20 (m, 2H), 3.05 (m, 4H), 1:45 (m, 4H), 1.25 (m, 4H), 0.90 (m, 6H).

### EXAMPLE 15

### (2S)-3-(4-[(3,5-dichloroisonicotinoyl}amino]phenyl}-2-([(dibutylamino)sulfonyl]. amino}propionic acid

The title compound (89%) was prepared from the compound of Example 14 by hydrolysis in a similar manner to Example 5.

m.p.: 167°C

δ (DMSO-d₆): 12.78 (bs, 1H), 10.88 (s, 1H), 8.80 (s, 2H), 7.55 (m, 3H), 7.28 (d, 2H), 3.72 (m, 1H), 2.96 (m, 1H), 2.73 (m, 5H), 1.28 (m, 8H), 0.85 (m, 6H).

### EXAMPLE 16

### Methyl (2S)-3-{4-[(3,5-dichloroisonicotinoyl)amino]phenyl}-2-({[[2-(3,4-dimethoxyphenyl)ethyl]isobutylamino]sulfonyl}amino)propionate

The title compound was obtained as a white solid from the compoud of Example 6 and *N-*[2-(3,4-dimethoxyphenyl)ethyl]-*N*-isobutylamine following the same procedure described in Example 8.

δ (CDCl₃): 8.55 (s, 2H), 7.65 (s, 1H), 7.50 (d, 2H), 7.20 (d, 2H), 6.65 (m, 3H), 4.90 (d, 1H), 4.20 (m, 1H), 3.90 (s, 3H), 3.85 (s, 3H), 3.75 (s, 3H), 3.20 (m, 4H), 2.83 (m, 4H), 1.87 (m, 1 H), 0.90 (d, 6H).

### EXAMPLE 17

### (2S)-3-{4-[(3,5-dichloroisonicotinoyl)amino]phenyl}-2-({[[2-(3,4-dimethoxyphenyl) ethyl]isobutylamino]sulfonyl}amino)propionic acid

The title compound (85%) was prepared from the compound of Example 16 by hydrolysis in a similar manner to Example 5.

m.p.: 192°C

δ (DMSO-d₆): 12.82 (bs, 1H), 10.89 (s, 1H), 8.80 (s, 2H), 7.65 (d, 1H), 7.58 (d, 2H), 7.27 (d, 2H), 6.86 (d, 1H), 6.78 (s, 1H), 6.68 (d, 2H), 3.80 (m, 1H), 3.74 (s, 3H), 3.71 (s, 3H), 3.01 (m, 3H), 2.73 (m, 3H), 2.57 (m, 2H), 1.70 (m, 1H), 0.76 (d, 6H).

### EXAMPLE 18

### Methyl (2S)-2-({[bis(thien-2-ylmethyl)amino]sulfonyl}amino)-3-{4-[(3,5-dichloroisonicotinoyl)amino]phenyl}propionate

The title compound was obtained as a white solid from the compoud of Example 6 and *N,N*-bis(thien-2-ylmethyl)amine following the same procedure described in Example 8.

δ (CDCl₃): 8.52 (s, 2H), 7.95 (s, 1H), 7.45 (d, 2H), 7.30 (dd, 2H), 7.07 (d, 2H), 6.99 (m, 4H), 5.43 (d, 1H), 4.39 (d, 4H), 4.28 (m, 1H), 3.77 (s, 3H), 3.07 (m. 2H).

### EXAMPLE 19

### (2S)-2-({[bis(thien-2-ylmethyl)amino]sulfonyl}amino)-3-(4-[(3,5-dichloroisonicotinoyl)amino]phenyl}propionic acid

The title compound (89%) was prepared from the compound of Example 18 by hydrolysis in a similar manner to Example 5.

δ (DMSO-d₆): 10.90 (s, 1H), 8.80 (s, 2H), 8.04 (d, 1H), 7.56 (d, 2H), 7.47 (dd, 2H), 7.20 (d, 2H), 6.95 (m, 2H), 6.86 (d, 2H), 4.06 (dd, 4H), 3.86 (m, 1 H), 2.88 (m, 2H).

### EXAMPLE 20

### Methyl (2S)-3-{4-[(3,5-dichloroisonicotinoyl)amino]phenyl}-2-({[methyl(2-pyridin-2-ylethyl)amino]sulfonyl}amino)propionate

The title compound was obtained as a white solid from the compoud of Example 6 and N-methyl-*N*-(2-pyridin-2-ylethyl)amine following the same procedure described in Example 8.

δ (CDCl₃): 8.52 (s, 2H), 8.49 (d, 1H), 8.28 (s, 1H), 7.63 (ddd, 1H), 7.51 (d, 2H), 7.17 (m, 4H), 5.46 (d, 1 H), 4.15 (m, 1 H), 3.76 (s, 3H), 3.46 (m, 2H), 3.03 (m, 4H), 2.64 (s, 3H).

### EXAMPLE 21

### (2S)-3-{4-[(3,5-dichloroisonicotinoyl)amino]phenyl}-2-({[methyl(2-pyridin-2-ylethyl) amino]sulfonyl}amino)propionic acid

The title compound (70%) was prepared from the compound of Example 20 by hydrolysis in a similar manner to Example 5.

δ (DMSO-d₆): 10.89 (s, 1H), 8.80 (s, 2H), 8.50 (d, 1H), 8.28 (s, 1H), 7.75 (m, 2H), 7.57 (d, 2H), 7.26 (m, 4H), 3.79 (m, 1H). 3.39 (m, 2H), 2.77 (m, 4H), 2.46 (s, 3H).

### EXAMPLE 22

### Methyl (2S)-2-{[(cyclohexylmethylamino)sulfonyl]amino}-3-{4-[(3,5-dichloro)sonicotinoyl)amino]phenyl)propionate

The title compound was obtained as a white solid from the compoud of Example 6 and cyclohexylmethylamine following the same procedure described in Example 8.

δ (CDCl₃): 8.56 (s, 2H), 7.73 (s, 1 H), 7.54 (d, 2H), 7.19 (d, 2H), 4.98 (d, 1 H), 4.14 (m, 1 H), 3.77 (s, 3H), 3.53 (m, 1H), 3.06 (m, 2H), 2.59 (s, 3H), 1.71 (m, 5H), 1.31 (m, 5H).

### EXAMPLE 23

### (2S)-2-{[(cyclohexylmethylamino)sulfonyl]amino}-3-{4-[(3,5-dichloroisonicotinoyl)amino]phenyl}propionic acid

The title compound (91%) was prepared from the compound of Example 22 by hydrolysis in a similar manner to Example 5.

m.p.: 196°C

δ (DMSO-d₆): 12:59 (bs, 1H), 10.74 (s, 1H), 8.66 (s, 2H), 7.45 (m, 3H), 7.12 (d, 2H), 3.54 (m, 1 H), 3.19 (m, 1H), 2.69 (m, 2H), 2.25 (s, 3H), 1.43 (m, 5H), 1.06 (m, 5H).

### EXAMPLE 24

### Methyl (2S)-3-{4-[(3,5-dichloroisonicotinoyl)amino]phenyl}-2-({[(3-methylbutyl) (thien-2-ylmethyl)amino]sulfonyl}amino)propionate

The title compound was obtained as a white solid from the compoud of Example 6 and *N-*(3-methylbutyl)-*N*-(thien-2-ylmethyl)amine following the same procedure described in Example 8.

δ (CDCl₃-d6): 8.55 (s, 2H), 7.80 (s, 1H), 7.50 (d, 2H). 7.28 (m, 1H), 7.14 (d, 2H), 6.97 (m, 2H), 5.12 (d, 1H), 4.44 (s, 2H), 4.21 (m, 1H), 3.76 (s, 3H), 3.06 (m, 4H), 1.42 (m, 3H), 0.88 (m, 6H).

### EXAMPLE 25

### (2S)-3-{4-[(3,5-dichloroisonicotinoyl)amino]phenyl}-2-({[(3-methylbutyl)(thien-2-ylmethyl)amino]sulfonyl}amino)propionic acid

The title compound (95%) was prepared from the compound of Example 24 by hydrolysis in a similar manner to Example 5.

m.p.: 187°C

δ (DMSO-d₆): 12.83 (bs, 1H), 10.91 (s, 1H), 8.80 (s, 2H), 7.82 (s, 1H), 7.60 (d, 2H), 7.47 (m, 1 H), 7.28 (d, 2H), 6.95 (m, 2H), 4.10 (s, 2H), 3.81 (m, 1H), 2.99 (m, 1H), 2.72 (m, 3H), 1.15 (m, 1H), 0.84 (m, 2H), 0.71 (d, 6H).

### EXAMPLE 26

### Methyl (2S)-3-{4-[(3,5-dichloroisonicotinoyl)amino]phenyl)-2-[(piperidin-1-ylsulfonyl)amino]propionate

A mixture of Preparation 2 (0.25 g, 0.75 mmoL) and piperidine (0.64 g, 7.5 mmoL) In dioxan (3 mL) was heated under reflux 20h. The solvent was removed, the crude was dissolved in ethyl acetate and washed with hydrochloric acid 2 N (10 mL) and brine (10 mL). The organic extracts were dried over sodium sulphate, filtered and evaporated. The resulting crude oil (0.17 g, 60%) was used in the next reaction without further purification.

To a solution of the crude above (0.17 g, 0.45 mmoL) in methanol (7 mL) were added zinc powder (0.29 g, 4.5 mmoL) and ammonium chloride (0.36 g, 6.7 mmoL) in portions. Then, water (3.5 mL) was added dropwise, after the mildly exothermic reaction subsided, the solution was stirred for 1h at room temperature. The mixture was filtered, and the filtrate was concentrated *in vacuo* until a yellow precipitate appeared. The precipitate was collected by filtration to yield methyl (2S)-3-(4-aminophenyl)-2-{[(dimethylamino)sulfonyl]amino}propionate (0.13 g, 87%) as a yellow solid.

A solution of 3,5-dichloroisonicotinoyl chloride (0.12 g, 0.58 mmoL) in methylene chloride (1 mL) was added dropwise to a stirred solution of (2*S*)-3-(4-aminophenyl)-2-{[(dimethylamino)sulfonyl]amino}propionate (0.133 g, 0.4 mmoL) and *N*-methylmorpholine (0.07 g, 0.72 mmoL) in methylene chloride (5 mL) at 0°C. The reaction mixture was allowed to warm to room temperature and stirred overnight. The reaction mixture was diluted with methylene chloride and washed with hydrochloric acid 1 N (10 mL) and brine (10 mL). The organic layer was dried (MgSO₄) and concentrated. The residue was purified by flash chromatography (methylene chloride:ethyl acetate, 3:1) to afford the title compound (0.06 g, 28%) as a white solid.

δ (CDCl₃): 8.59 (s, 2H), 7.8 (s, 1H), 7.52 (d, 2H), 7.20 (d, 2H), 5.10 (d, 1H), 4.22 (m, 1 H), 3.80 (s, 3H), 3.05 (m, 6H), 1.50 (m, 6H).

### EXAMPLE 27

### (2S)-3-{4-[(3,5-dichloroisonicotinoyl)amino]phenyl}-2-[(piperidin-1-ylsulfonyl)amino] propionic acid

The title compound (81 %) was prepared from the compound of Example 26 by hydrolysis in a similar manner to Example 5.

m.p.; 208°C

δ (DMSO-d₆): 12.82 (bs, 1H), 10.89 (s, 1H), 8.79 (s, 2H), 7.65 (d, 1H), 7.59 (d, 2H), 7.30 (d, 2H), 3.74 (m, 1H), 2.98 (m, 1H), 2.67 (m, 5H), 1.26 (m, 6H).

### EXAMPLE 28

### Methyl (2S)-2-[(azepan-1-ylsulfonyl)amino]-3-{4-[(3,5-chloroisonicotinoyl)amino] phenyl}propionate

The title compound was obtained as a white solid from the compoud of Preparation 2 and azepane following the same procedure described in Example 26.

δ (CDCl₃): 8.55 (s, 2H), 7.90 (s, 1 H), 7.55 (d, 2H), 7.20 (d, 2H), 5.05 (d, 1H), 4.18 (m, 1 H), 3.80 (s, 3H), 3.18 (m, 4H), 3.10 (m, 2H), 1.60 (m, 8H).

### EXAMPLE 29

### (2S)-2-[(azepan-1-ylsulfonyl)amino]-3-{4-[(3,5-dichloroisonicotinoyl)amino] phenyl}propionic acid

The title compound (93%) was prepared from the compound of Example 28 by hydrolysis in a similar manner to Example 5.

m.p.: 185°C

δ (DMSO-d₆): 12.80 (bs, 1H), 10.90 (s, 1H), 8.80 (s, 2H), 7.58 (m, 3H); 7.29 (d, 2H), 3.78 (m, 1 H), 2.81 (m, 6H), 1.42 (m, 8H).

### EXAMPLE 30

### Methyl (2S)-3-{4-[(3,5-dichloroisonicotinoyl)amino]phenyl}-2-[(morpholin-4-ylsulfonyl}amino]propionate

The title compound was obtained as a white solid from the compoud of Preparation 2 and morpholine following the same procedure described in Example 26.

δ (DMSO-d₆): 10.80 (s, 1H), 8.62 (s, 2H), 8.01 (d, 1H), 7.40 (d, 2H), 7.10 (d, 2H), 3.75 (m, 1H), 3.50 (s, 3H), 3.25 (m, 4H), 2.80 (m, 1 H), 2.50 (m, 5H).

### EXAMPLE 31

### (2S)-3-{4-[(3,5-dichloroisonicotinoyl)amino]phenyl}-2-[(morpholin-4-ylsulfonyl) amino]propionic acid

The title compound (83%) was prepared from the compound of Example 30 by hydrolysis in a similar manner to Example 5.

m.p.: 152°C

δ (DMSO-d₆): 12.90 (bs, 1H), 10.90 (s, 1H), 8.80 (s, 2H), 7.96 (d, 1H), 7.59 (d, 2H), 7.30 (d, 2H), 3.80 (m, 1H), 3.27 (m, 4H), 3.01 (m, 1H), 2.70 (m, 5H).

### EXAMPLE 32

### Methyl (2S)-3-{4-[(3,5-dichioroisonicotinoyl)amino]phenyl}-2-[(thiomorpholin-4-ylsulfonyl)amino]propionate

The title compound was obtained as a white solid from the compoud of Preparation 2 and thiomorpholine following the same procedure described in Example 26.

δ (DMSO-d₆): 10.90 (s, 1H), 8.80 (s, 2H), 8.15 (d, 1H), 7.60 (d, 2H), 7.24 (d, 2H), 3.85 (m, 1H), 3.63 (s, 3H), 3.05 (m, 6H), 2.50 (m, 4H).

### EXAMPLE 33

### (2S)-3-{4-[(3,5-dichloroisonicotinoyl)amino]phenyl}-2-[(thiomorpholin-4-ylsulfonyl) amino]propionic acid

The title compound (58%) was prepared from the compound of Example 32 by hydrolysis in a similar manner to Example 5.

m.p.: 173°C

δ (DMSO-d₆): 12.91 (bs, 1H), 10.91 (s, 1H), 8.80 (s, 2H), 7.93 (d, 1 H), 7.59 (d, 2H), 7.29 (d, 2H), 3.76 (m, 1H), 3.06 (m, 6H), 2.45 (m, 4H).

### EXAMPLE 34

### Methyl (2S)-2-{[(dimethylamino)sulfonyl]amino}-3-[4-(2,6-naphthyridin-1-yloxy) phenyl]propionate

To a solution of dimethylsulfamoyl chloride (4.07 g, 28.34 mmoL) in pyridine (20 mL) under nitrogen atmosphere was added a solution of methyl (2*S*)-2-amino-3-[4-(2,6-naphthyridin-1-yloxy)phenyl]propionate (2.29 g, 7.08 mmoL) in pyridine (20 mL) dropwise at 0°C. The mixture was stirred at room temperature for 16h. The solvent was removed, the crude was dissolved in ethyl acetate and washed with hydrochloric acid 0.5 N (200 mL) and brine (200 mL). The organic extracts were dried over sodium sulphate, filtered and evaporated. The residue was purified by flash chromatography (hexanes:ethyl acetate, 1:1) to afford the title compound (0.9 g, 38%) as a white solid.

δ (CDCl₃): 9.30 (s, 1H), 8.79 (d, 1H), 8.19 (d, 1H), 8.10 (d, 1H), 7.44 (d, 1 H), 7.26 (dd, 4H), 4.92 (d, 2H), 4.28 (m, 1H), 3.81 (s, 3H), 3.13 (m, 2H), 2.70 (s, 6H).

### EXAMPLE 35

### (2S)-2-{[(dimethylamino)sulfonyl]amino}-3-[4-(2,6-naphthyridin-1-yloxy)phenyl] propionic acid

To a solution of the solid above (0.09 g, 0.21 mmoL) in tetrahydrofuran (2 mL) was added NaOH 2N (2 mL) and stirred at room temperature for 2h. The organic solvent was removed under reduced pressure and the resulting aqueous solution was acidified with citric acid until pH 2. The precipitate was collected by filtration to obtain the title compound (0.05 g, 56%) as a white solid.

m.p.: 202°C

δ (DMSO-d₆): 12.88 (bs, 1 H), 9.44 (s, 1H), 8.79 (d, 1H), 8.19 (d, 1H), 8.10 (d, 1H), 7.71 (m, 2H), 7.40 (d, 2H), 7.23 (d, 2H), 3.87 (m, 1H), 3.07 (m, 1H), 2.82 (m, 1 H), 2.40 (s, 6H).

### EXAMPLE 36

### Methyl (2S)-2-{[(diisobutylamino)sulfonyl]amino}-3-[4-(2,6-naphthyridin-1-yloxy) phenyl]propionate

The title compound was obtained as a white solid from the compoud of Example 34 and diisobutylamine following the same procedure described in Example 8.

δ (CDCl₃): 9.30 (s, 1H), 8.78 (d, 1H). 8.17 (d, 1H), 8.09 (d, 1H), 7.44 (d, 1H), 7.25 (dd, 4H), 4.78 (d, 1H), 4.27 (m, 1H), 3.77 (s, 3H), 3.13 (m. 2H), 2.86 (d, 4H), 1.88 (m, 2H), 0.91 (d, 12H).

### EXAMPLE 37

### (2S)-2-{[(diisobutylamino)sulfonyl]amino}-3-[4-(2,6-naphthyridin-1-yloxy)phenyl] propionic acid

The title compound (93%) was prepared from the compound of Example 36 by hydrolysis in a similar manner to Example 35.

m.p.: 88°C

δ (DMSO-d₆): 12.82 (bs, 1H), 9.44 (s, 1H), 8.80 (d, 1H), 8.15 (d, 1 H), 8:08 (d, 1H), 7.70 (d, 1H), 7.55 (d, 1H), 7.39 (d, 2H), 7.23 (d, 2H), 3.92 (m, 1H), 3.04 (m, 2H), 2.75 (m, 4H), 1.72 (m, 2H), 0.78 (d, 12H).

### EXAMPLE 38

### Methyl (2S)-2-{[(diisobutylamino)sulfonyl]amino}-3-[4-(2,6-naphthyridin-1-ylamino) phenyl]propionate

The title compound was obtained as a white solid from the compoud of Example 46 and diisobutylamine following the same procedure described in Example 8.

δ (CDCl₃): 9.17 (s, 1H), 8.65 (d, 1H), 8.20 (d, 1H). 7.68 (m, 3H), 7.19 (m, 4H), 4.91 (bs, 1H), 4.25 (bs, 1H), 3.75 (s, 3H), 3.09 (m, 2H), 2.88 (d, 4H), 1.91 (m, 2H), 0.90 (d, 12H).

### EXAMPLE 39

### (2S)-2-{[(diisobutylamino)sulfonyl]amino}-3-[4-(2,6-naphthyridin-1-ylamino)phenyl] propionic acid

The title compound (79%) was prepared from the compound of Example 38 by hydrolysis in a similar manner to Example 35.

δ (DMSO-d₆): 9.34 (s. 1H), 9.24 (s, 1H), 8.70 (d, 1H), 8.42 (d, 1H), 8.15 (d, 1H), 7.83 (d, 2H), 7.47 (d, 1H), 7.28 (m, 3H), 3.80 (m, 1H), 2.87 (m, 2H), 1.69 (m, 2H), 0.77 (d, 12H).

### EXAMPLE 40

### Methyl (2S)-3-{4-[(2,6-dichlorobenzoyl)amino]phenyl}-2-{[(2,6-dimethylpiperidin-1-yl) sulfonyl]amino}propionate

To a solution of methyl, (2*S*)-2-amino-3-{4-[(2,6-dichlorobenzoyl)amino]phenyl}propionate hydrochloride (0.79 g, 1.97 mmoL) and 2,6-dimethylpiperidine-1-sulfonyl chloride (0.38 g, 1.79 mmoL) (prepared as described in J. A. Kloek and K. L. Leschinsky J. Org. Chem. 1976, 41, 4028) in tetrahydrofuran (14 mL) was added triethylamine (0.4 g, 3.94 mmoL) under nitrogen atmosphere. The mixture was refluxed for 16h. The solvent was removed, the crude was dissolved in dichloromethane and washed with citric acid 5% solution (100 mL) and brine (100 mL). The organic extracts were dried over sodium sulphate, filtered and evaporated. The residue was purified by flash chromatography (hexanes:ethyl acetate, 3:2) to afford the title compound (0.27 g, 28%) as a white solid.

δ (CDCl₃): 7.55 (d, 2H), 7.48 (s, 1H), 7.30 (m, 3H), 7.20 (d, 2H), 4.75 (d, 1H), 4.00 (m, 3H), 3.73 (s, 3H), 3.08 (m, 2H), 1.48 (m, 6H), 1.29 (d, 6H).

### EXAMPLE 41

### (2S)-3-{4-[(2,6-dichlorobenzoyl)amino]phenyl}-2-{[(2,6-dimethylpiperidin-1-yl) sulfonyl]amino}propionic acid

The title compound (95%) was prepared from the compound of Example 40 by hydrolysis in a similar manner to Example 35.

m.p.: 159°C

δ (DMSO-d₆): 12.74 (bs, 1H), 10.69 (s, 1H),7.53 (m, 6H), 7.23.(d, 2H), 3.77 (m, 1H), 3.67 (m, 1H), 3.52 (m, 1H), 2.85 (m, 2H), 1.35 (m, 6H), 1.17 (d, 6H).

### EXAMPLE 42

### Methyl (2S)-3-{4-[(3,5-dichloroisonicotinoyl)amino]phenyl}-2-{[(diisopropylamino) sulfonyl]amino}propionate

The title compound was obtained as a white solid from methyl (2*S*)-2-amino-3-{4-[(3,5-dichloroisonicotinoyl)aminolphenyl}propionate hydrochloride and diisopropylamine following the same procedure described in Example 40.

δ (CDCl₃): 8.55 (s, 2H), 7.84 (s, 1H), 7.54 (d, 2H), 7.19 (d, 2H), 5.01 (d, 1H), 4.18 (m, 1H), 3.76 (s, 3H), 3.59 (m, 2H), 3.11 (d, 2H), 1.23 (dd, 12H).

### EXAMPLE 43

### (2S)-3-(4-[(3,5-dichloroisonicotinoyl)amino]phenyl)-2-([(diisopropylamino)sulfonyl] amino}propionic acid

The title compound (70%) was prepared from the compound of Example 34 by hydrolysis in a similar manner to Example 35.

m.p.: 151°C

δ (DMSO-d6): 12.70 (bs, 1H), 10.88 (s, 1H), 8.80 (s, 2H); 7.56 (d, 2H), 7.40 (bs, 1H), 7.25 (d, 2H), 3.66 (m, 1H), 3.44 (m, 2H), 2.87 (m, 2H), 1.11 (d, 6H), 1.01 (d, 6H).

### EXAMPLE 44

### Methyl (2S)-3-{4-[(3,5-dichloroisonicotinoyl)amino]phenyl)-2-{[(2,6-dimethyl piperidin-1-yl)sulfonyl]amino}propionate

The title compound was obtained as a white solid from methyl (2S)-2-amino-3-{4-[(3,5-dichloroisonicotinoyl)amino]phenyl}propionate hydrochloride and 2,6-dimethylpiperidine following the same procedure described in Example 40.

δ (CDCl₃): 8.56 (s, 2H), 7.83 (s, 1 H), 7.54 (d, 2H), 7.20 (d, 2H), 5.00 (d, 1H), 4.02 (m, 3H), 3.77 (s, 3H), 3.07 (m, 2H), 1.47 (m, 6H), 1.29 (d, 6H).

### EXAMPLE 45

### (2S)-3-{4-[(3,5-dichlorolsonicotinoyl)amino]phenyl)-2-{{1(2,6-dimethylpiperidin-1-yl)sulfonyl]amino}propionic acid

The title compound (64%) was prepared from the compound of Example 34 by hydrolysis in a similar manner to Example 35.

m.p.: 162°C

δ (DMSO-d₆): 12.72 (bs, 1H), 10.88 (s, 1H), 8.80 (s, 2H), 7.56 (d, 2H), 7.36 (bs, 1H). 7.26 (d, 2H), 3.74 (m, 2H), 3.58 (m, 1H), 2.86 (m, 2H), 1.35 (m, 6H), 1.15 (d, 6H).

### EXAMPLE 46

### Methyl (2S)-2-{[(dimethylamino)sulfonyl]amino}-3-[4-(2,6-naphthyridin-1-ylamino) phenyl]propionate

The title compound was obtained as a white solid from methyl (2S)-2-amino-3-[4-(2,6-naphthyridin-1-ylamino)phenyl]propionate following the same procedure described in Example 4.

δ (DMSO-d₆): 9.37 (s, 1H), 9.24 (s, 1H). 8.70 (d, 1H), 8.43 (d, 1H); 8.16 (d, 1H), 7.97 (d, 1H). 7.84 (d, 2H), 7.28 (m, 3H), 3.95 (m, 1H), 3.66 (s, 3H), 2.90 (m, 2H), 2.42 (s, 6H).

### EXAMPLE 47

### (2S)-2-{[(dimethylamino)sulfonyl]amino}-3-[4-(2,6-naphthyridin-1-ylamino)phenyl]propionic acid

The title compound (51 %) was prepared from the compound of Example 46 by hydrolysis in a similar manner to Example 35.

m.p.:211°C

δ (DMSO-d₆): 12.72 (bs, 1H), 10.88 (s, 1H), 8.80 (s, 2H), 7.56 (d, 2H), 7.36 (bs, 1H), 7.26 (d, 2H), 3.74 (m, 2H), 3.58 (m, 1 H), 2.86 (m, 2H), 1.35 (m, 6H), 1.15 (d, 6H).

### EXAMPLE 48

### Methyl (2S)-2-{[(diisopropylainino)sulfonyl]amino}-3-[4-(2,6-naphthyridin-1-ylamino) phenyl]propionate

The title compound was obtained as a white solid from methyl (2S)-2-amino-3-{4-[(2,6-dichlorobenzoyl)amino]phenyl}propionate hydrochloride and diisopropylamine following the same procedure described in Example 40.

δ (CDCl₃): 7.58 (d, 2H), 7.46 (s, 1H), 7.35 (m, 3H), 7.19 (d, 2H), 4.74 (d, 1H), 4.17 (m, 1H), 3.75 (s, 3H), 3.59 (m, 2H), 3.08 (d, 2H), 1.23 (dd, 12H).

### EXAMPLE 49

### (2S)-2-{[(diisopropylamino)sulfonyl]amino}-3-[4-(2,6-naphthyridin-1-ylamino)phenyl] propionic acid

The title compound (77%) was prepared from the compound of Example 48 by hydrolysis in a similar manner to Example 35.

m.p.: 168°C

δ (DMSO-d₆): 12.65 (bs, 1H), 10.68 (s, 1H), 7.56 (m, 5H), 7.32 (bs, 1H), 7.22 (s, 2H), 3.64 (m, 1H), 3.43 (m, 2H), 2.86 (m, 2H), 1.11 (d, 6H), 1.01 (d, 6H).

### EXAMPLE 50

### Methyl (2S)-2({[cyclohexyl(isopropyl)amino]sulfonyl}amino)-3-[4-(2,6-naphthyridin-1-ylamino)phenyl]propionate

The title compound was obtained as a white solid from methyl (2S)-2-amino-3-{4-[(3,5-dichloroisonicotinoyl)amino]phenyl}propionate hydrochloride and cyclohexyl(isopropyl)amine following the same procedure described in Example 40.

δ (CDCl₃): 8.56 (s, 2H), 7.84 (s, 1H), 7.55 (d, 2H), 7.19 (d, 2H), 4.94 (d, 1H), 4.14 (m, 1H), 3.77 (s, 3H), 3.62 (m, 2H), 3.08 (m, 2H), 1.69 (m, 6H), 1.24 (m, 10H).

### EXAMPLE 51

### (2S)-2-({[cyclohexyl(isopropyl)amino]sulfonyl}amino)-3-[4-(2,6-naphthyridin-1-ylamino)phenyl]prooloni.c acid

The title compound (85%) was prepared from the compound of Example 50 by hydrolysis in a similar manner to Example 35.

m.p.: 162°C

δ (DMSO-d₆): 10.86 (s, 1H), 8.80 (s, 2H), 7.55 (d, 2H), 7.24 (d, 2H), 7.10 (bs, 1H), 3.45 (m, 1 H), 2.87 (m, 4H), 3.08 (m, 2H), 1.58 (m, 6H), 1.11 (m, 10H).

### EXAMPLE 52

### Methyl (2S)-2-{[(diisopropylamino)sulfonyl]amino }-3-[4-(2,6-naphthyridin-1-yloxy) phenyl]propionate

The title compound was obtained as a white solid from methyl (2S)-2-amino-3-[4-(2,6-naphthyridin-1-yloxy)phenyl]propionate and diisopropylamine following the same procedure described in Example 40.

δ (CDCl₃): 9.29 (s, 1H), 8.78 (d, 1H), 8.17 (d, 1H), 8.10 (d, 1H), 7.43 (d, 2H), 7.22 (m, 4H), 4.81 (d, 1H), 4.21 (m, 2H), 3.77 .(s, 3H), 3.62 (m, 2H), 3.13 (d, 2H), 1.27 (d, 6H), 1.24 (d, 6H).

### EXAMPLE 53

### (2S)-2-{[(diisopropylamino)sulfonyl]amino}-3-[4-(2,6-naphthyridin-1-yloxy)phenyl] propionic acid

The title compound (70%) was prepared from the compound of Example 52 by hydrolysis in a similar manner to Example 35.

m.p.: 112°C

δ (DMSO-d₆): 9.44 (s, 1H), 8.79 (d, 1H), 8.17 (d, 1H), 8.10 (d, 1H), 7.69 (d, 1H), 7.79 (d, 1H), 7.36 (d, 2H), 7.22 (d, 2H), 3.74 (m, 1H), 3.46 (m, 2H), 2.96 (m, 2H), 1.14 (d, 6H), 1.04 (d, 6H).

### EXAMPLE 54

### Methyl (2S)-2-{[(diisopropylamino)sulfonyl]amino}-3-[4-(2,6-naphthyridin-1-ylamino) phenyl]propionate

The title compound was obtained as a white solid from methyl (2S)-2-amino-3-[4-(2,6-naphthyridin-1-ylamino)phenyl]propionate and diisopropylamine following the same procedure described in Example 40.

δ (CDCl₃): 9.20 (s. 1H). 8.68 (d, 1H), 8.24 (d, 1H), 7.69 (m, 1H), 7.19 (d, 4H), 4.78 (d, 1H), 4.19 (m, 1H); 3.75 (s, 3H), 3.63 (m, 2H), 3.09 (d, 2H), 1.27 (d, 6H), 1.23 (d, 6H).

### EXAMPLE 55

### (2S)-2-{[(diisopropylamino)sulfonyl]amino}-3-[4-(2,6-naphthyridin-1-ylamino)phenyl] propionic acid

The title compound (64%) was prepared from the compound of Example 54 by hydrolysis in a similar manner to Example 35.

m.p.: 195°C

δ (DMSO-d₆): 12.70 (bs, 1H), 9.33 (s, 1H), 9.23 (s, 1H), 8.68 (d, 1H), 8.41 (d, 1H), 8.15 (d, 1H), 7.80 (d, 2H), 7.49 (d, 1H), 7.29 (d, 1H), 7.22 (d, 2H), 3.70 (m, 1H), 3.46 (m, 2H), 2.87 (m, 2H), 1.12 (d, 6H), 1.02 (d, 6H).

### EXAMPLE 56

### Methyl (2S)-2-{[(2,6-dimethylpiperidin-1-yl)sulfonyl]amino}-3-[4-(2,6-naphthyridin-1-ylamino)phenyl]propionate

The title compound was obtained as a white solid from methyl (2*S*)-2-amino-3-[4-(2,6-naphthyridin-1-ylamino)phenyl]propionate and 2,6-dimethylpiperidine following the same procedure described in Example 40.

δ (DMSO-d₆): 9.34 (s, 1H), 9.24 (s, 1H), 8.69 (d, 1H), 8.42 (d, 1H), 8.15 (d, 1H), 7.79 (m, 3H), 7.30 (d, 1H), 7.20 (d, 2H), 3.77 (m, 3H), 3.61 (s, 3H), 2.85 (m, 2H), 1.36 (m, 6H), 1.16 (d, 6H).

### EXAMPLE 57

### (2S)-2-{[(2,6-dimethylpiperidin-1-yl)sulfonyl]amino}-3-[4-(2,6-naphthyridin-1-ylamino)phenyl]propionic acid

The title compound (80%) was prepared from the compound of Example 56 by hydrolysis in a similar manner to Example 35.

m.p.: 204°C.

δ (DMSO-d₆): 9.32 (s, 1H), 9.23 (s, 1H), 8.69 (d, 1H). 8.41 (d, 1H), 8.15 (d, 1H), 7.78 (d, 2H), 7.29 (d, 2H), 7.21 (d, 2H), 3.80 (m, 2H), 3.61 (m, 1H), 2.86 (m, 2H), 1.37 (m, 6H), 1.17 (d, 6H).

### EXAMPLE 58

### Methyl (2S)-2-{[(2,6-dimethylpiperidin-1-yl)sulfonyl]amino}-3-[4-(2,6-naphthyridin-1-yloxy)phenyl]propionate

The title compound was obtained as a white solid from methyl (2S)-2-amino-3-[4-(2,6-naphthyridin-1-yloxy)phenyl]propionate and 2,6-dimethylpiperidine following the same procedure described in Example 40.

δ (DMSO-d₆): 9.44 (s, 1H), 8.80 (d, 1H), 8.18 (d, 1H), 8.09 (d, 1H), 7.80 (d, 1H), 7.71 (d, 1H), 7.35 (d, 2H), 7.23 (d, 2H), 3.76 (m, 3H), 3.64 (s, 3H), 2.93 (m, 2H), 1.38 (m, 6H), 1.17 (d, 6H).

### EXAMPLE 59

### (2S)-2-{[(2,6-dimethylpiperidin-1-yl)sulfonyl]amino}-3-[4-(2,6-naphthyridin-1-yloxy)phenyl]propionic acid

The title compound (76%) was prepared from the compound of Example 58 by hydrolysis in a similar manner to Example 35.

m.p.: 151°C

δ (DMSO-d₆): 9.44 (s, 1H), 8.79 (d, 1H), 8.18 (d, 1H), 8:10 (d, 1H), 7.70 (d, 1H), 7.49 (d, 1 H), 7.36 (d, 2H), 7.21 (d, 2H), 3.74 (m, 3H), 2.92 (m, 2H), 1.37 (m; 6H), 1.18 (d, 6H).

### EXAMPLE 60

### Methyl (2S)-2-({[benzyl(isopropyl)amino]sulfonyl}amino)-3-{4-[(2,6-dichlorobenzoyl) amino]phenyl}propionate

The title compound was obtained as a white solid from methyl (2S)-2-amino-3-{4-[(2,6-dichlorobenzoyl)amino]phenyl}propionate hydrochloride and *N*-benzyl-*N*-isopropylamine following the same procedure described in Example 40.

δ (CDCl₃): 7.57 (d, 2H), 7.34 (in, 9H), 7.13 (d, 2H), 4.72.(d, 1H), 4.22 (s, 2H), 4.11 (m. 1H), 3.94 (m, 1 H), 3.72 (s, 3H), 2.99 (m, 2H), 1.15 (d, 3H); 1.12 .(d, 3H).

### EXAMPLE 61

### (2S)-2-({[benzyl(isopropyl)amino]sulfonyl)amino)-3-(4-[(2,6-dichlorobenzoyl)amino] phenyl}propionic acid

The title compound (93%) was prepared from the compound of Example 60 by hydrolysis in a similar manner to Example 35.

m.p.: 159°C

δ (DMSO-d₆): 10.68 (s, 1H), 7.54 (m, 6H), 7.26 (m, 8H), 4.06 (m, 2H), 3.72 (m, 2H), 2.89 (m, 2H), 0.95 (d, 3H), 0.98 (d, 3H).

### EXAMPLE 62

### Methyl (2S)-2-({[benzyl(isopropyl)amino]sulfonyl}amino)-3-{4-[(3,5-dichloroisonicotinoyl)amino]phenyl}propionate

The title compound was obtained as a white solid from methyl (2S)-2-amino-3-{4-[(3,5-dichloroisonicotinoyl)amino]phenyl}propionate hydrochloride and *N*-benzyl-*N-*isopropylamine following the same procedure described in Example 40.

δ (CDCl₃): 8.56 (s, 2H), 7.65 (s, 1H), 7.50 (d, 2H), 7.33 (m, 5H), 7.12 (d, 2H), 4,96 (d, 1H), 4.23 (s, 2H), 4.11 (m, 1H), 3.94. (m, 1H), 3.71 (s, 3H), 3.00 (m, 2H), 1.14 (d, 3H), 1.12(d,3H).

### EXAMPLE 63

### (2S)-2-({[benzyl(isopropyl)amino]sulfonyl}amino)-3-{4-[(3,5-dichloroisonicotinoyl) amino]phenyl}propionic acid

The title compound (84%) was prepared from the compound of Example 62 by hydrolysis in a similar manner to Example 35.

m.p.: 182°C

δ (DMSO-d₆): 12.82 (bs, 1H), 10.89 (s, 1H), 8.80 (s, 2H), 7.57 (m, 3H), 7.27 (m, 8H), 4.02 (m, 2H), 3.69 (m, 2H), 2.87 (m, 2H), 0.94 (d,3H), 0.87 (d, 3H).

### EXAMPLE 64

### Methyl (2S)-2-({[isopropyl(thien-2-ylmethyl)amino]sulfonyl}amino)-3-{4-[(3,5-dichloroisonicotinoyl)amino]phenyl}propionate

The title compound was obtained as a white solid from methyl (2*S*)-2-amino-3-{4-[(3,5-dichloroisonicotinoyl)amino]phenyl}propionate hydrochloride and *N*-(thien-2-ylmethyl)propan-2-amine following the same procedure described in Example 40.

δ (CDCl₃): 8.52 (s, 2H), 8.22 (s, 1H), 7.50 (d, 2H), 7.23 (m, 1H), 7.11 (d, 2H), 6.95 (m, 2H), 5.18 (d, 1H), 4.42 (s, 2H), 4.11 (m, 1H), 3.90 (m, 1H). 3.72 (s, 3H), 3.01 (m, 2H), 1.25 (d, 6H).

### EXAMPLE 65

### (2S)-2-({[isopropyl(thien-2-ylmethyl)amino]sulfonyl}amino)-3-{4-[(3,5-dichloroisonicotinoyl)amino]phenyl}propionic acid

The title compound (98%) was prepared from the compound of Example 64 by hydrolysis in a similar manner to Example 35.

δ (DMSO-d₆): 12.75 (bs, 1H), 10.89 (s, 1H), 8.80 (s, 2H), 7.69 (d, 1H), 7.59 (d, 2H), 7.50 (d, 2H), 7.40 (d, 1 H), 7.24 (d, 2H), 6.98 (m, 1 H), 6.93 (m, 1H), 4.22 (s, 2H), 3.76 (m, 1H), 3.65 (m, 1 H), 2.93 (m, 1 H), 2.78 (m, 1 H), 1.01 (d, 3H), 0.90 (d, 3H).

### EXAMPLE 66

### Methyl (2S)-2-({[isopropyl(thien-2-ylmethyl)amino]sulfonyl}amino)-3-{4-[(3,5-dichlorobenzoyl)amino]phenyl}propionate

The title compound was obtained as a white solid from methyl (2*S*)-2-amino-3-{4-((2,6-dichlorobenzoyl)amino]phenyl}propionate hydrochloride and *N*-(thien-2-ylmethyl)propan-2-amine following the same procedure described in Example 40.

δ (CDCl₃): 7.56 (d, 2H), 7.35 (m, 5H), 7.12 (d, 2H), 6.97 (m, 2H), 4.78 (d, 1H), 1.48 (s, 2H), 4.08 (m, 1 H), 3.90 (m, 1H), 3.71 (s, 3H), 3.00 (m, 2H); 1.24 (d, 6H), 1.18 (d, 6H).

### EXAMPLE 67

### (2S)-2-({[isopropyl(thien-2-ylmethyl)amino]sulfonyl}amino)-3-{4-[(3,5-dichlorobenzoyl)amino]phenyl}propionic acid

The title compound (72%) was prepared from the compound of Example 66 by hydrolysis in a similar manner to Example 35.

m.p.: 122°C

δ (DMSO-d₆): 10.69 (s, 1H), 7.54 (m, 6H), 7.41 (d, 1 H), 7.21 (d, 2H), 6.95 (d, 2H), 4.23 (s, 2H), 3.69 (m, 2H), 2.87 (m, 2H); 1.01 (d, 6H), 0.92 (d, 6H).

### EXAMPLE 68

### Methyl (2S)-3-{4-[(2,6-dichloroisonicotinoyl)amino]phenyl}-2-[({isobutyl[(1S)-1-phenylethyl]amino}sulfonyl)amino]propionate

The title compound was obtained as a white solid from methyl (2*S*)-2-amino-3-{4-[(3,5-dichloroisonicotinoyl)amino]phenyl}propionate hydrochloride and *N*-isobutyl-*N*-[(1*S*)-1-phenylethyl]amine following the same procedure described in Example 40.

δ (CDCl₃): 8.60 (s, 2H), 7.57 (d, 2H), 7.35 (m, 5H), 7.20 (d, 2H), 5.02 (m, 1H), 4.73 (d, 1H), 4.25 (m, 1H), 3.78 (s, 3H), 3.10 (m, 2H), 2.77 (d, 2H), 2.07 (m, 1H), 1.61 (d, 3H), 0.76 (d, 3H), 0.63 (d, 3H).

### EXAMPLE 69

### (2S)-3-{4-[(2,6-dichloroisonicotinoyl)amino]phenyl}-2-[({isobutyl[(1S)-1-phenylethyl] amino}sulfonyl)amino]propionic acid

The title compound (70%) was prepared from the compound of Exampple 68 by hydrolysis in a similar manner to Example 35.

m.p.: 182°C

δ (DMSO-d₆): 12.81 (bs, 1H), 10.88 (s, 1H), 8.80 (s, 2H), 7.55 (d, 2H), 7.29 (m, 8H), 4.83 (d, 1H), 3.81 (m, 1H), 2.87 (m, 2H), 2.38 (d, 2H), 1.56 (m, 1H), 1.33 (d, 3H), 0.53 (d, 3H), 0.39 (d, 3H).

### EXAMPLE 70

### Methyl (2S)-2-({[cyclopentyl(isopropyl)amino]sulfonyl}amino)-3-(4-[(3,5-dichloroisonicotinoyl)amino]phenyl}propionate

The title compound was obtained as a white solid from methyl (2S)-2-amino-3-{4-[(3,5-dichloroisonicotinoyl)amino]phenyl}propionate hydrochloride and *N*-cyclopentyl-*N-*isopropylamine following the same procedure described in Example 40.

δ (DMSO-d₆): 10.92 (s, 1H). 8.80 (s, 2H), 7.88 (d, 1H), 7.60 (d, 2H), 7.27 (d, 2H), 3.73 (m, 1H). 3.62 (s, 3H), 3.40 (m, 2H), 2.90 (m, 2H), 1.60 (m, 5H), 1.31 (m, 3H), 1.10 (d, 3H), 1.00 (d, 3H).

### EXAMPLE 71

### (2S)-2-({[cyclopentyl(isopropyl)amino]sulfonyl)amino)-3-(4-[(3,6-dichlbrolsonicotinoyl)amino]phenyl}propionic acid

The title compound (55%) was prepared from the compound of Example 70 by hydrolysis in a similar manner to Example 35.

m.p.:157°C

δ (DMSO-d₆): 10.90 (s, 1H). 8.80 (s, 2H), 7.58 (m, 3H), 7.27 (d, 2H), 3.63 (m, 1H), 3.42 (m, 2H), 2.85 (m, 2H), 1.56 (m, 5H), 1.31 (m, 3H), 1.10 (d, 3H), 0.97 (d, 3H).

### EXAMPLE 72

### Methyl (2S)-3-{4-[(3,5-dichloroisonicotinoyl)amino]phenyl}-2-({[isobutyl (isopropyl)amino]sulfonyl}amino)propionate

The title compound was obtained as a white solid from methyl (2S)-2-amino-3-{4-[(3,5-dichloroisonicotinoyl)amino]phenyl}propionate hydrochloride and *N*-isobutyl-*N-*isopropylamine following the same procedure described in Example 40.

δ (DMSO-d₆): 10.90 (s, 1H). 8.80 (s, 2H), 7.80 (d, 1H), 7.60 (d, 2H), 7.25 (d, 2H), 3.80 (m, 1H), 3.63 (s, 3H), 3.58 (m, 1H), 2.90 (m, 4H), 1.80 (m, 1H). 1.00 (d, 3H), 0.90 (d, 3H), 0.78 (d, 6H).

### EXAMPLE 73

### (2S)-3-(4-[(3,5-dichloroisonicotinoyl)aminqlphenyl)-2-((risobutyl(isopropyl)amino] ulfonyl}amino)propionic acid

The title compound (91 %) was prepared from the compound of Example 72 by hydrolysis in a similar manner to Example 35.

m.p.: >300°C

δ (DMSO-d₆): 9.43 (s, 1H), 8.79 (d, 1H), 8.16 (d, 1H), 8.11 (d, 1H), 7.69 (d, 1H), 7.25 (dd, 5H), 3.66 (m, 1H), 3.51 (m, 1H), 3.35 (m, 1H), 2.95 (m, 3H), 1.61 (m, 6H), 1.12 (m, 10H).

### EXAMPLE 74

### Methyl (2S)-2-({[cyclohexyl(isopropyl)amino]sulfonyl}amino)-3-[4-(2,6-naphthyridin-1-yloxy)phenyl]propionate

The title compound was obtained as a white solid from methyl (2S)-2-amino-3-[4-(2,6-naphthyridin-1-yloxy)phenyl]propionate and *N*-cyclohexyl-*N*-isopropylamine following the same procedure described in Example 40.

δ (CDCl₃): 9.30 (s, 1H), 8.80 (d, 1 H), 8.18 (d, 1H), 8.10 (d, 1H), 7.42 (d, 1H), 7.23 (m, 5H), 4.68 (d, H), 4.22 (m, 1H), 3.78 (s, 3H), 3.70 (m, 1H), 3.10 (m, 3H), 1.70 (m, 6H), 1.20 (m, 10H).

### EXAMPLE 75

### (2S)-2-({[cyclohexyl(isopropyl)amino]sulfonyl}amino)-3-[4-(2,6-naphthyridin-1-yloxy) phenyl]propionic acid

The title compound (81%) was prepared from the compound of example 74 by hydrolysis in a similar manner to Example 35.

m.p.: 138°C

δ (DMSO-d₆): 10.92 (s, 1H), 8.80 (s, 2H), 7.58 (m, 3H), 7.27 (d, 2H), 3.69 (m, 1H), 3.57 (m, 1H), 2.86 (m, 4H), 1.79 (m, 1H), 1.00 (d, 3H), 0.88 (d, 3H), 0.77 (d, 6H).

### EXAMPLE 76

### Methyl (2S)-3-{4-[(3,5-dichloroisonicotinoyl)amino]phenyl}-2-[({Isobutyl[(1R)-1-phenylethyl]amino}sulfonyl)amino]propionate

The title compound was obtained as a white solid from methyl (2S)-2-amino-3-{4-[(3,5-dichloroisonicotinoyl)amino]phenyl}propionate hydrochloride and *N*-isobutyl-*N*-[(1*R*)-1-phenylethyl]amine following the same procedure described in Example 40.

δ (CDCl₃): 8.55 (s, 2H), 7.70 (s, 1 H), 7.50 (d, 2H), 7.30 (m, 5H), 7.10 (d, 2H), 5.10 (d, 1H), 4.78 (m, 1H), 4.23 (m, 1H), 3.79 (s, 3H), 3.10 (m, 2H), 2.80 (m, 2H), 1.30 (m, 3H), 0.90 (m, 1H), 0.70 (d, 3H), 0.60 (d, 3H).

### EXAMPLE 77

### (2S)-3-{4-[(3,5-dichloroisonicotinoyl)amino]phenyl}-2-[({isobutyl[(1R)-1-phenylethyl] amino}sulfonyl)amino]propionic acid

The title compound (76%) was prepared from the compound of Example 76 by hydrolysis in a similar manner to Example 35.

m.p.: 171°C

δ (DMSO-d₆): 8.79 (s, 2H), 7.58 (d, 2H); 7.43 (bs, 1H), 7.29 (m, 7H), 4.80 (m, 1H), 3.79 (m, 1H), 2.96 (m, 2H), 2.54 (m, 2H), 1.43 (m, 4H), 0.56 (d, 3H), 0.0.42, (d, 3H).

### EXAMPLE 78

### Methyl (2S)-2-({[methyl(phenyl)amino]sulfonyl}amino)-3-(4-[(2,6-dichlorobenzoyl) amino]phenyl}propionate

To a solution of methyl (2S)-2-amino-3-{4-[(2,6-dichlorobenzoyl)amino]phenyl}propionate hydrochloride (0.45 g, 1.24 mmoL) in methylene chloride (2 mL) chlorosulfonic acid (0.14 g, 1.24 mmoL) was added dropwise at 0°C in the presence of triethylamine (0.25 g, 2.48 mmoL). After the addition was completed the reaction mixture was allowed to reach room temperature and was stirred for an additional 2h. The solvent was removed under reduced pressure and the crude was dissolved in benzene (2 mL), PCl₅ was added and the mixture was heated under reflux for 1h. The solvent was again removed under reduced pressure and the resulting crude was treated with ethyl ether. The solid was separated by filtration and the resulting crude oil was used in the next reaction without further purification.

To a solution of *N*-methylaniline (0.133 g, 1.24 mmoL) in tetrahydrofuran (10 mL) in the presence of triethylamine (0.5 g, 4.96 mmoL) the crude sulfamoyl chloride was added at 0°C and the mixture was allowed to stir at this temperature for 2h. The solvent was removed, the crude was dissolved in ethyl acetate and washed with ammonium chloride 0.5 M (50 mL) and brine (50 mL). The organic extracts were dried over sodium sulphate, filtered and evaporated. The residue was purified by flash chromatography (hexanes:ethyl acetate, 3:2) to afford the title compound (0.08 g, 14%) as a white solid.

δ (CDCl₃): 7.59 (d, 2H), 7.32 (m, 9H), 7.14 (d, 2H), 4.94 (d, 1H), 4.18 (m, 1H), 3.74 (s, 3H), 3.16 (s, 3H), 3.03 (m, 2H).

### EXAMPLE 79

### (2S)-2-({[methyl(phenyl)amino]sulfonyl}amino)-3-{4-[(2,6-dichlorobenzoyl) amino]phenyl}propionic acid

The title compound (89%) was prepared from the compound of Example 78 by hydrolysis in a similar manner to Example 35.

m.p.:117°C

δ (DMSO-d₆): 12.85 (bs, 1H), 1.0.73 (s, 1H), 7.56 (m, 5H), 7.25 (m, 6H), 7.07 (d, 2H), 3.79 (m, 1H), 2.92 (s, 3H), 2.86 (m, 2H).

### EXAMPLE 80

### Methyl (2S)-({[2-(phenylsulfonyl)phenyl]amino}sulfonyl)amino]-3-{4-[(3,5-dichloroisonicotinoyl)amino]phenyl}propionate

The title compound was obtained as a white solid from methyl (2S)-2-amino-3-{4-[(3,5-dichloroisonicotinoyl)amino]phenyl}propionate hydrochloride and 2-(phenylsulfonyl)aniline following the same procedure described in Example 78.

δ (DMSO-d₆): 10.85 (s, 1H), 8.98 (m, 2H), 8.88 (s, 2H), 7.97 (m, 3H), 7.75 (m, 3H), 7.41 (m, 3H), 7.25 (m, 2H), 7.02 (d, 2H), 4.02 (m, 1H), 3.52 (s, 3H), 3.10 (m, 2H).

### EXAMPLE 81

### (2S)-({[2-{phenylsulfonyl)phenyl]amino}sulfonyl)amino]-3-{4-[(3,5-dichloroisonicotinoyl)amino]phenyl}propionic acid

The title compound (83%) was prepared from the compound of Example 80 by hydrolysis in a similar manner to Example 35.

m.p.: 206°C

δ (DMSO-d₆): 10.81 (s, 1H), 8.88 (m, 4H), 7.96 (m, 3H), 7.77 (m, 3H), 7.38 (m, 3H), 7.23 (m, 2H), 7.03 (d, 2H), 4.04 (m, 1H), 2.81 (m, 2H).

The following examples illustrate pharmaceutical compositions according to the present invention and procedure for their preparation.

### EXAMPLE 82

### Preparation of a pharmaceutical composition: tablets

| Formulation: | |
|---|---|
| Compound of the present invention | 5.0 mg |
| Lactose | 113.6 mg |
| Microcrystalline cellulose | 28.4 mg |
| Light silicic anhydride | 1.5 mg |
| Magnesium stearate | 1.5 mg |

Using a mixer machine, 15 g of the compound of the present invention was mixed with 340.8 g of lactose and 85.2 g of microcrystalline cellulose. The mixture was subjected to compression moulding using a roller compactor to give a flake-like compressed material. The flake-like, compressed material was pulverized using a hammer mill, arid the pulverized material was screened through a 20 mesh screen. A 4.5 g portion of light silicic anhydride and 4.5 g of magnesium stearate were added to the screened material and mixed. The mixer product was subjected to a tablets making machine equipped with a die/punch system of 7.5 mm in diameter, thereby obtaining 3,000 tablets each having 150 mg in weight.

### EXAMPLE 83

### Preparation of a pharmaceutical composition: tablets coated

| Formulation: | |
|---|---|
| Compound of the present invention | 5.0 mg |
| Lactose | 95.2 mg |
| Com starch | 40.8 mg |
| Polyvinylpyrrolidone | 7.5 mg |
| Magnesium stearate | 1.5 mg |
| Hydroxypropylcellulose | 2.3 mg |
| Polyethylene glycol | 0.4 mg |
| Titanium dioxide | 1.1 mg |
| Purified talc | 0.7 mg |

Using a fluidized bed granulating machine, 15 g of the compound of the present invention was mixed with 285.6 g of lactose and 122.4 g of com starch. Separately, 22.5 g of polyvinylpyrrolidone was dissolved in 127.5 g of water to prepare a binding solution. Using a fluidized bed granulating machine, the binding solution was sprayed on the above mixture to give granulates. A 4.5 g portion of magnesium stearate was added to the obtained granulates and mixed. The obtained mixture was subjected to a tablet making machine equipped with a die/punch biconcave system of 6.5 mm in diameter, thereby obtaining 3,000 tablets, each having 150 mg in weight.
Separately, a coating solution : was prepared by suspending .6.9 g of hydroxypropylmethylcellulose 2910, 1.2 g. of polyethylene glycol 6000, 3.3 g of titanium dioxide and 2.1 g of purified talc in 72.6 g of water. Using a High Coated, the 3,000 tablets prepared above were coated with the coating solution to give film-coated tablets, each having 154.5 mg in weight.

### EXAMPLE 84

### Preparation of a pharmaceutical composition: liquid inhalant

| Permutation: | |
|---|---|
| Compound of the present invention | 400 µg |
| Physiological saline | 1 ml |

A 40 mg portion of the compound of the present invention was dissolved in 90 ml of physiological saline, and the solution was adjusted to a total volume of 100 ml with the same saline solution, dispensed in 1 ml portions into 1 ml capacity ampoule and then sterilized at 115° for 30 minutes to give liquid inhalant.

### EXAMPLE 85

### Preparation of a pharmaceutical composition: powder inhalant

| Formulation: | |
|---|---|
| Compound of the present invention | 200 µg |
| Lactose | 4,000 µg |

A 20 g portion of the compound of the present invention was uniformly mixed with 400 g of lactose, and a 200 mg portion of the mixture was packed in a powder inhaler for exclusive use to produce a powder inhalant.

### EXAMPLE 86

### Preparation of a pharmaceutical composition: inhalation aerosol.

| Formulation: | |
|---|---|
| Compound of the present invention | 200 µg |
| Dehydrated (Absolute) ethyl alcohol USP | 8,400 µg |
| 1,1,1,2-Tetrafluoroethane (HFC-134A) | 46,810 µg |

The active ingredient concentrate is prepared by dissolving 0.0480 g of the compound of the present invention in 2.0160 g of ethyl alcohol. The concentrate is added to an appropriate filling apparatus. The active ingredient concentrate is dispensed into aerosol container, the headspace of the container is purged with Nitrogen or HFC-134A vapor (purging ingredients should not contain more than 1 ppm oxygen) and is sealed with valve. 11.2344 g of HFC-1 34A propellant is then pressure filled into the sealed container.

### EXAMPLE 87

### Preparation of a pharmaceutical composition: Gel.

| Formulation: | |
|---|---|
| Compound of the present invention | 0,03% |
| Carbomer 980NF | 1,00 % |
| Glycerin | 10.00 % |
| Diethanolamine to | pH: 5.5 |
| Purified water | to 100,00% |

### EXAMPLE 88

### Preparation of a pharmaceutical composition: Pomade.

| Formulation: | |
|---|---|
| Compound of the present invention | 0,03% |
| Glyceryl monolaurate | 5,00% |
| Hydrogenated Coco-glycerides | 15.00% |
| Glycerine | 15,00 % |
| Light mineral oil | 5,00% |
| White petrolatum | to 100,00% |

## Claims

1. A compound of formula (I): 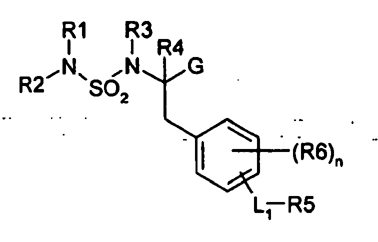 wherein:
• G is a COOH group or a tetrazolyl group;
• R1 and R2 are independently selected from hydrogen atoms and alkyl, alkenyl, alkynyl, cycloalkyl, cycloalkylalkyl, cycloalkylalkenyl, cycloalkylalkynyl, cycloalkenyl, cycloalkenylalkyl, cycloalkenylalkenyl, cycloalkenylalkynyl, heterocyclyl, heterocyclylalkyl, heterocyclylalkenyl, heterocyclylalkynyl, aryl, arylalkyl, arylalkenyl, arylalkynyl, heteroaryl, heteroarylalkyl, heteroarylalkenyl, or heteroarylalkynyl groups; or R1 and R2 form, together with the nitrogen atom to which they are attached, either a 3- to 14- membered monocyclic or polycyclic heterocyclic ring system or a 5- to 14-membered heteroaryl group wherein said groups comprise from 1 to 5 heteroatoms selected from nitrogen, oxygen and sulphur;
wherein said alkyl, alkenyl, and alkynyl groups or moieties are unsubstituted or substituted with one to four substituents, which may be the same or different and are independently selected from Ra;
and wherein said cycloalkyl, heterocyclyl, aryl and heteroaryl groups or moieties are unsubstituted or substituted with one to four substituents, which may be the same or different and are independently selected from Rb;
• R3 and R4 are independently selected from hydrogen atoms and alkyl groups having from 1 to 6 carbon atoms;
• R5 is selected from the group consisting of 6- to 14- membered monocyclic or polycyclic aryl groups and 5- to 14- membered monocyclic or polycyclic heteroaryl groups comprising from 1 to 5 heteroatoms selected from nitrogen, oxygen and sulphur;
wherein said aryl and heteroaryl groups or moieties are unsubstituted or substituted with one to four substituents, which may be the same or different and are independently selected from Rb;
• R6 is a group selected from -OH, -ORc, -NO₂, halogen, -S(O)Rc, -S(O)₂Rc, -SRc,-S(O)₂ORc, -S(O)NRcRc -S(O)₂NRcRc, -NRcRc, -O(CRcRc)mNRcRc, -C(O)Rc,-CO₂Rc, -CO₂(CRcRc)mCONRcRc, -OC(O)Rc, -CN, -C(O)NRcRc, -NRcC(O)Rc,-OC(O)NRcRc, -NRcC(O)ORc, -NRcC(O)NRcRc, -CRc(N-ORc), -CFH₂, -CF₂H, -Ra,-CF₃, alkyl, alkenyl and alkynyl;
• n is an integer from 0 to 3
• Ra is a group selected from alkyl, -OH, -ORc, -NO₂, halogen, -S(O)Rc, -S(O)₂Rc,-SRc, -S(O)₂ORc, S(O)NRcRc, -S(O)₂NRcRc, -NRcRc, -O(CRcRc)mNRcRc, -C(O)Rc, -CO₂Rc, -CO₂(CRcRc)mCONRcRc, -OC(O)Rc, -CN, -C(O)NRcRc. -NRcC(O)Rc,-OC(O)NRcRc, -NRcC(O)ORc, -NRcC(O)NRcRc, -CRc(N-ORc), -CFH₂, - CF₂H, -Ra, or -CF₃ ; wherein if two or more Rc groups are present these may be the same or different;
• Rb is a group selected from -OH, -ORd, -NO₂, halogen, -S(O)Rd, -S(O)₂Rd, -SRd,-S(O)₂ORd, -S(O)NRdRd, -S(O)₂NRdRd, -NRdRd,- O(CRdRd)mNRdRd, -C(O)Rd,-CO₂Rd, -CO₂(CRdRd)mCONRdRd, -OC(O)Rd, -CN, -C(O)NRdRd, -NRdC(O)Rd,-OC(O)NRdRd, -NRdC(O)ORd, -NRdC(O)NRdRd, -CRd(N-ORd), -CFH₂, -CF₂H, -Ra,-CF₃, alkyl, alkenyl, C₂₋₄alkynyl, cycloalkyl, cycloalkylalkyl, heterocyclyl, heterocyclylalkyl, aryl, arylalkyl, heteroaryl or heteroarylalkyl; wherein said alkyl, alkenyl, alkynyl, cycloalkyl, heterocyclyl, aryl and heteroaryl groups or moieties are unsubstituted or substituted with one to four substituents which may be the same or different and are independently selected from Ra;
• L1 is either a direct bond or a group selected from the group consisting of -N(Rc)-,-O-, -N(Rc)CO-, -CON(Rc)-, -O(CO)N(Rc)- and -N(Rc)(CO)O-;
• Rc is a hydrogen atom or an alkyl group having from 1 to 4 carbon atoms;
• Rd is alkyl, alkenyl, alkynyl, cycloalkyl, cycloalkylalkyl, cycloalkylalkenyl, cycloalkylalkynyl, cycloalkenyl, cycloalkenylalkyl, cycloalkenylalkenyl, cycloalkenylalkynyl, heterocyclyl, heterocyclylalkyl, heterocyclylalkenyl, heterocyclylalkynyl, aryl, arylalkyl, arylalkenyl, arylalkynyl, heteroaryl, heteroarylalkyl, heteroarylalkenyl, or heteroarylalkynyl;
wherein said alkyl, alkenyl, alkynyl, cycloalkyl, cycloalkenyl, heterocyclyl, aryl and heteroaryl groups are unsubstituted or substituted with one to four substituents, which may be the same or different and are independently selected from Re;
• Re is a group selected from alkyl, -OH, -ORc, -NO₂, halogen, -S(O)Rc, -S(O)₂Rc,-SRc, -S(O)₂ORc, -S(O)NRcRc, -S(O)₂NRcRc, -NRcRc, -O(CRcRc)mNRcRc,-C(O)Rc, -CO₂Rc, -CO₂(CRcRc)mCONRcRc, -OC(O)Rc, -CN, -C(O)NRcRc,-NRcC(O)Rc, -OC(O)NRcRc, -NRcC(O)ORc, -NRcC(O)NRcRc, -CRc(N-ORc),-CFH₂, - CF₂H, -Ra, or -CF₃ ; wherein if two or more Rc groups are present these may be the same or different;
and any pharmaceutically acceptable salt thereof as well as any compound resulting from the esterification, with any alcohol, of the carboxylic group in the case where G is such a carboxylic group and any pharmaceutically acceptable salt thereof

2. A compound according to claim 1, wherein:
• each alkyl moiety in R1, R2, R6, Ra, Rb, Rd or Re has 1 to 20 carbon atoms;
• each alkenyl moiety in R1, R2, R6, Rb and Rd has 2 to 20 carbon atoms;
• each alkynyl moiety in R1, R2, R6 and Rd has 2 to 20 carbon atoms;
• each aryl moiety is a monocyclic or polycyclic C₆₋₁₄ aryl moiety;
• each heteroaryl moiety is a 5- to 14- membered ring system comprising at least one heteroaromatic ring and containing at least one heteroatom selected from O, S and N;
• each cycloalkyl moiety has 3 to 7 carbon atoms;
• each cycloalkenyl moiety has 4 to 7 carbon atoms; and
• each heterocyclyl moiety is a monocyclic or polycyclic C₃-C₁₄ carbocyclic ring system in which one or more of the carbon atoms are replaced by a heteroatom selected from N, O and S.

3. A compound according to claim 2, wherein:
• each alkyl moiety has 1 to 6 carbon atoms;
• each alkenyl moiety has 2 to 6 carbon atoms; and
• each alkynyl moiety has 2 to 6 carbon atoms.

4. A compound according to any preceding claim wherein G is a COOH group as well as any compound resulting from the esterification, with an alcohol, of the COOH group.

5. A compound according to any preceding claim wherein R3 and R4 are hydrogen atoms.

6. A compound according any preceding claim wherein R1 and R2 are independently selected from hydrogen atoms and alkyl, cycloalkyl, heterocyclylalkyl, aryl, arylalkyl, heteroarylalkyl groups, wherein said alkyl, alkenyl, and alkynyl, cycloalkyl, heterocyclyl, aryl and heteroaryl groups or moieties are unsubstituted or substituted;
or R1 and R2 form, together with the nitrogen atom to which they are attached, either a 5- to 8- membered monocyclic heterocyclic ring system wherein said ring system comprises from 1 to 4 heteroatoms selected from nitrogen, oxygen and sulphur and is unsubstituted or substituted.

7. A compound according to any preceding claim wherein R5 is selected from the group consisting of 6- to 14- membered monocyclic or polycyclic aryl and 5- to 14-membered monocyclic or polycyclic heteroaryl groups comprising from 1 to 5 heteroatoms selected from nitrogen, oxygen and sulphur wherein said aryl and heteroaryl groups or moieties are unsubstituted or substituted

8. A compound according to claim 7 wherein said aryl or heteroaryl groups are unsubstituted or substituted by one or more halogen atoms.

9. A compound according to any preceding claim wherein L1 is a group selected from - NH-, -O- and -NHCO-.

10. A compound according to any preceding claim wherein R5-L1- is selected from the group comprising benzamide, isonicotinamide, 2,6-naphthyridin-1-ylamino, 2,7-naphthyridin-1-ylamino; 2,6-naphthyridin-1-yloxy and 2,7-naphthyridin-1-yloxy wherein said groups are unsubstituted or substituted.

11. A compound according to any preceding claim wherein n is zero.

12. A compound according to any preceding claim which is one of:
• (*2S*)-2-{[(tert-butylamino)sulfonyl]amino}-3-(4-[(3,5-dichloroisonicotinoyl)amino]phenyl}propionic acid
• Methyl (2*S*)-2-(*N*-benzylaminosulfonylamino)-3-[4-(2,6-dichlorobenzoylamino)phenyl]propionate
• (2*S*)-2-(*N*-benzylaminosulfonylamino)-3-[4-(2,6-dichlorobenzoylamino)phenyl]propionic acid
• Methyl (2*S*)-3-{4-[(2,6-dichlorobenzoyl)amino]phenyl}-2-{[(dimethytarnino)sulfonyl]amino} propionate
• (2*S*)-3-{4-[(2,6-dichlorobenzoyl)amino]phenyl}-2-{[(dimethylamino)sulfonyl]amino} propionic acid
• Methyl (2*S*)-3-{4-[(3,5-dichloroisonicotinoyl)amino]phenyl}-2-{[(dimethylamino)sulfonyl]amino}propionate
• (2*S*)-3-(4-[(3,5-dichloroisonicotinoyl)amino]phenyl)-2-{[(dimethylamino)sulfonyl]amino}propionic acid
• Methyl (2*S*)-3-(4-{[1-(2,6-dichlorophenyl)methanoyl]amino}phenyl)-2-(pipendine-1-sulfonylamino)propionate
• (2*S*)-3-(4-([1-(2,6-dichlorophenyl)methanoyl]amino)phenyl)-2-(piperidine-1-sulfonylamino)propionic acid
• Methyl (2*S*)-3-{4-[(3,5-dichloroisonicotinoyl)amino]phenyl}-2-{[(diisobutylamino)sulfonyl]amino}propionate
• (2*S*)-3-{4-[(3,5-dichloroisonicotinoyl)amino]phenyl}-2-{[(diisobutylamino)sulfonyl]amino}propionic acid
• Methyl (2*S*)-2-({[benzyl(ethyl)amino]sulfonyl}amino)-3-{4-[(3,5-dichloroisonicotinoyl)aminolphenyl}propionate
• (2*S*)-2-{[(benzylethylamino)sulfonyl]amino}-3-{4-[(3,5-dichloroisonicotinoyl)amino]phenyl}propionic acid
• Methyl (2*S*)-3-{4-[(3,5-dichloroisonicotinoyl)amino]phenyl}-2-{[(dibutylamino)sulfonyl]amino}propionate
• (2*S*)-3-{4-[(3,5-dichloroisonicotinoyl)amino]phenyl}-2-{[(dibutylamino)sulfonyl]amino}propionic acid
• Methyl (2*S*)-3-{4-[(3,5-dichloroisonicotinoyl)amino]phenyl}-2-({[[2-(3,4-dimethoxyphenyl)ethyl]isobutylamino]sulfonyl}amino)propionate
• (2*S*)-3-{4-[(3,5-dichloroisonicotinoyl)amino]phenyl)-2-({[[2-(3,4-dimethoxyphenyl)ethyl]isobutylamino]sulfonyl)amino)propionic acid
• Methyl (2*S*)-2-({[bis(thien-2-ylmethyl)amino]sulfonyl}amino)-3-{4-[(3,5-dichloroisonicotinoyl)amino]phenyl}propionate
• (2*S*)-2-({[bis(thien-2-ylmethyl)amino]sulfonyl}amino)-3-{4-[(3,5-dichloroisonicotinoyl)amino]phenyl}propionic acid
• Methyl (2*S*)-3-{4-[(3,5-dichloroisonicotinoyl)amino]phenyl)-2-({{methyl(2-pyridin-2-ylethyl)amino]sulfonyl}amino)propionate
• (2*S*)-3-{4-[(3,5-dichloroisonicotinoyl)amino]phenyl)-2-({[methyl(2-pyridin-2-ylethyl)amino]sulfonyl}amino)propionic acid
• Methyl (2*S*)-2-{[(cyclohexytmethylamino)sulfonyl]amino}-3-{4-[(3,5-dichloroisonicotinoyl)amino]phenyl}propionate
• (2*S*)-2-{[(cyclohexylmethylamino)sulfonyl]amino}-3-{4-[(3,5-dichloroisonicotinoyl)amino]phenyl}propionic acid
• Methyl (2*S*)-3-{4-[(3,5-dichloroisonicotinoyl)amino]phenyl}-2-({[(3-methylbutyl)(thien-2-ylmethyl)amino]sulfonyl}amino)propionate
• (2*S*)-3-{4-[(3,5-dichloroisonicotinoyl)amino]phenyl}-2-({[(3-methylbutyl)(thien-2-ylmethyl)amino]sulfony}amino)propionic acid
• Methyl (2*S*)-3-{4-[(3,5-dichloroisonicotinoyl)amino]phenyl}-2-[(piperidin-1-ylsulfonyl)amino]propionate
• (2*S*)-3-{4-[(3,5-dichloroisonicotinoyl)amino]phenyl}-2-[(piperidin-1-ylsulfonyl)amino]propionic acid
• Methyl (2*S*)-2-[(azepan-1-ylsulfonyl)amino]-3-{4-[(3,5-dichloroisonicotinoyl)amino]phenyl}propionate
• (2*S*)-2-[(azepan-1-ylsulfonyl)amino]-3-{4-[(3,5-dichloroisonicotinoyl)amino]phenyl}propionic acid
• Methyl (2*S*)-3-{4-[(3,5-dichloroisonicotinoyl)amino]phenyl}-2-[(morpholin-4-ylsulfonyl)amino]propionate
• (2*S*)-3-{4-[(3,5-dichtoroisonicotinoyl)amino]phenyl}-2-{(morpholin-4-ylsulfonyt)amino]propionic acid
• Methyl (2*S*)-3-{4-[(3,5-dichloroisoriicotinoyl)amino]phenyf}-2-[(thiomorpholin-4-ylsulfonyl)amino]propionate
• (2*S*)-3-{4-[(3,5-dichioroisonicotinoyl)amino]phenyl}-2-[(thiomorpholin-4-ylsulfonyl)amino]propionic acid
• Methyl (2*S*)-2-{[(dimethylamino)sulfonyl]amino)-3-[4-(2,6-naphthyridin-1-yloxy)phenyl]propionate
• (2*S*)-2-{[(dimethylamino)sulfonyl]amino)-3-[4-(2,6-naphthyridin-1-yloxy)phenyl]propionic acid
• Methyl (2*S*)-2-{[(diisobutylamino)sulfonyl]amino}-3-[4-(2,6-naphthyridin-1-yloxy)phenyl]propionate
• (2*S*)-2-{[(diisobutylamino)sulfonyl]amino)-3-[4-(2,6-naphthyridin-1-yloxy)phenyl]propionic acid
• Methyl (2*S*)-2-{[(diisobutylamino)sulfonyl]amino}-3-[4-(2,6-naphthyridin-1-ylamino)phenyl]propionate
• (2*S*)-2-{[(diisobutylamino)sulfonyl]amino}-3-[4-(2,6-naphthyridin-1-ylamino)phenyl]propionic acid
• Methyl (2*S*)-3-{4-[(2,6-dichlorobenzoyl)amino]phenyl}-2-{[(2,6-dimethylpiperidin-1-yl)sulfonyl]amino}propionate
• (2*S*)-3-{4-[(2,6-dichlorobenzoyl)amino]phenyl}-2-{[(2,6-dimethylpiperidin-1-yl)sulfonyl]amino}propionic acid
• Methyl (2*S*)-3-{4-[(3,5-dichloroisonicotinoyl)amino]phenyl}-2-{[(diisopropylamino)sulfonyl]amino}propionate
• (2*S*)-3-{4-[(3,5-dichloroisonicotinoyl)amino]phenyl}-2-{[(diisopropylamino)sulfonyl]amino}propionic acid
• Methyl (2*S*)-3-{4-[(3,5-dichloroisonicotinoyl)aminolphenyl}-2-{[(2,6-dimethylpiperidin-1-yl)sulfonyl]amino}propionate
• (2*S*)-3-{4-[(3,5-dichloroisonicotinoyl)amino]phenyl}-2-{((2,6-dimethylpiperidin-1-yl)sulfonyl]amino}propionic acid
• Methyl (2*S*)-2-{[(dimethylamino)sulfonyl]amino)-3-[4-(2,6-naphthyndin-1-ylamino)phenyl]propionate
• (2*S*)-2-{[(dimethylamino)sulfonyl]amino}-3-[4-(2,6-naphthyridin-1-ylamino)phenyl]propionic acid
• Methyl (2*S*)-2-{[(diisopropylamino)sulfonyl]amino}-3-[4-(2,6-naphthyridin-1-ylamino)phenyl]propionate
• (2*S*)-2-{[(diisopropylamino)sulfonyl]amino}-3-[4-(2,6-naphthyridin-1-ylamino)phenyl]propionic acid
• Methyl (2*S*)-2-({[cyclohexyl(isopropyl)amino]sulfonyl}amino)-3-[4-(2,6-naphthyridin-1-ylamino)phenyl]propionate
• (2*S*)-2-({[cyclohexyl(isopropyl)amino]sulfonyl}amino)-3-[4-(2,6-naphthyridin-1-ylamino)phenyl]propionic acid
• Methyl (2*S*)-2-{[(diisopropylamino)sulfonyl]amino}-3-[4-(2,6-naphthyridin-1-yloxy)phenyl]propionate
• (2*S*)-2-{[(diisopropylamino)sulfonyl]amino}-3-[4-(2,6-naphthyridin-1-yloxy)phenyl]propionic acid
• Methyl (2*S*)-2-{[(diisopropylamino)sulfonyl]amino]-3-[4-(2,6-naphthyridin-1-ylamino)phenyl]propionate
• (2*S*)-2-{[(diisopropylamino)sulfonyl]amino}-3-[4-(2,6-naphthyridin-1-ylamino)phenyl]propionic acid
• Methyl (2*S*)-2-{[(2,6-dimethylpiperidin-1-yl)sulfonyl]amino}-3-[4-(2,6-naphthyridin-1-ylamino)phenyl]propionate
• (2*S*)-2-{[(2,6-dimethylpiperidin-1-yl)sulfony]amino}-3-[4-(2,6-naphthyridin-1-ylamino)phenyl]propionic acid
• Methyl (2*S*)-2-{[(2,6-dimethylpiperidin-1-yl)sulfonyl]amino}-3-[4-(2,6-naphthyridin-1-yloxy)phenyl]propionate
• (2*S*)-2-{[(2,6-dimethylpiperidin-1-yl)sulfonyl]amino)-3-[4-(2,6-naphthyridin-1-yloxy)phenyl]propionic acid
• Methyl (2*S*)-2-({[benzyl(isopropyl)amino]sulfonyl}amino)-3-{4-[(2,6-dichlorobenzoyl)amino]phenyl}propionate
• (2*S*)-2-({[benzyl(isopropyl)aminolsulfonyl]amino)-3-{4-[(2,6-dichlorobenzoyl)amino]phenyl}propionic acid
• Methyl (2*S*)-2-({[benzyl(isopropyl)amino]sulfonyl}amino)-3-{4-[(3,5-dichloroisonicotinoyl)amino]phenyl}propionate
• (2*S*)-2-({[benzyl(isopropyl)amino]sulfonyl}amino)3-{4-[(3,5-dichloroisonicotinoyl)aminolphenyl)propionic acid
• Methyl (2*S*)-2-({[isopropyl(thien-2-ylmethyl)amino]sulfonyl}amino}-3-{4-[(3,5-dichloroisonicotinoyl)amino]phenyl}propionate
• (2*S*)-2-({[isopropyl(thien-2-ylmethyl)amino]sulfonyl}amino)-3-{4-[(3,5-dichloroisonicotinoyl)amino]phenyl)propionic acid
• Methyl (2*S*)-2-({[isopropyl(thien-2-ylmethyl)amino]sulfonyl}amino)-3-{4-[(3,5-dichlorobenzoyl)amino]phenyl}propionate
• (2*S*)-2-({[isopropyl(thien-2-ylmethyl)amino]sulfonyl}amino)-3-{4-[(3,5-dichlorobenzoyl)amino]phenyl}propionic acid
• Methyl (2*S*)-3-{4-(2,6-dichloroisonicotinoyl)amino]phenyl}-2-[({isobutyl[(1S)-1-phenylethyl]amino}sulfonyl)amino]propionate
• (2*S*)-3-{4-[(2,6-dichloroisonicotinoyl)amino]phenyl}-2-[({isobutyl[(1*S)-*1-phenylethyl]amino)sulfonyl)amino]propionic acid
• Methyl (2*S*)-2-({[cyclopentyl(isopropyl)amino]sulfonyl}amino )-3-{4-[(3,5-dichloroisonicotinoyl)amino]phenyl}propionate
• (2*S*)-2-({[cyclopentyl(isopropyl)amino]sulfonyl}amino)-3-{4-[(3,5-dichloroisonicotinoyl)amino]phenyl}propionic acid
• Methyl (2*S*)-3-{4-[(3,5-dichloroisonicotinoyl)amino]phenyl}-2-({[isobutyl(isopropyl)amino]sulfonyl}amino)propionate
• (2*S*)-3-{4-[(3,5-dichloroisonicotinoyl)amino]phenyl}-2-({[isobutyl(isopropyl)amino]sulfonyl}amino)propionic acid
• Methyl (2*S*)-2-({[cyclohexyl(isopropyl)amino)sulfonyl}amino)-3-[4-(2,6-naphthyridin-1-yloxy)phenyl]propionate
• (2*S*)-2-({[cyclohexyl(isopropyl)amino]sulfonyl}amino)-3-[4-(2,6-naphthyridin-1-yloxy)phenyl]propionic acid
• Methyl (2*S*)-3-(4-[(3,5-dichloroisonicotinoyl)amino]phenyl}-2-[({isobutyl[(1R)-1-phenylethyl]amino}sulfonyl)amino]propionate
• (2*S*)-3-{4-[(3,5-dichloroisonicotinoyl)amino]phenyl)-2-[({isobutyl](1R)-1-phenylethyl]amino}sulfonyl)amino]propionic acid
• Methyl (2*S*)-2-({[methyl(phenyl)amino]sulfonyl}amino)-3-{4-[(2,6-dichlorobenzoyl)amino]phenyl}propionate
• (2*S*)-2-({[methy)(phenyl)amino]sufonyl}amino)-3-{4-[(2,6-dichlorobenzoyl)amino]phenyl}propionic acid
• Methyl (2*S*)-({[2-(phenylsulfonyl)phenyl]amino}sulfonyl)amino]-3-{4-[(3,5-dichloroisonicotinoyl)amino]phenyl}propionate
• (2*S*)-({[2-(phenylsulfonyl)phenyl]amino}sulfonyl)amino]-3-{4-[(3,5-dichloroisonicotinoyl)amino]phenyl}propionic acid

13. A process for producing a compound of formula I as defined in any one of claims 1 to 12, which process comprises reacting an amine of formula (III): 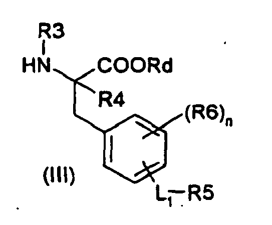 wherein Rd, R3, R4, R5, R6, L1 and n are as defined in any one of claims 1 to 12 with a corresponding sulfamoyl chloride of formula (IV): 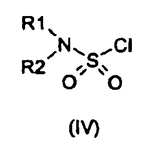 wherein R1 and R2 are as defined in any one of claims 1 to 12.

14. A process for producing a compound of formula I as defined in any one of claims 1 to 12, which process comprises reacting an amine of formula (II a): 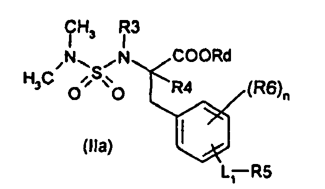 wherein Rd, R3, R4, R5, R6, L1 and n are as defined in any one of claims 1 to 12 with an amine of formula (V) 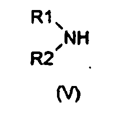 wherein R1 and R2 are as defined in any one of claims 1 to 12.

15. A process for producing a compound of formula I as defined in any one of claims 1 to 12, which process comprises reacting an amine of formula (III): 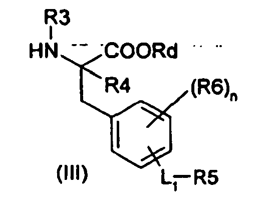 wherein R3, R4, R5, R6, Rd, L1 and n are as defined in any one of claims 1 to 12 with a sulfamide of formula (VII): 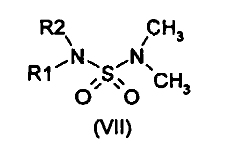 wherein R1 and R2 are as defined in any one of claims 1 to 12.

16. A process for producing a compound of formula as defined in any one of claims 1 to 12, which process comprises the steps of:
(a) reacting an amine of formula (III): 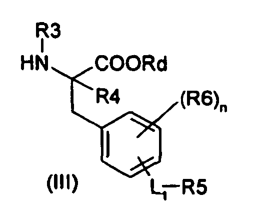 wherein R3, R4, R5, R6, Rd, L1 and n are as defined in any one of claims 1 to 12 with tert-butanol and chlorosulfonyl isocyanate to yield the sulfamide of formula (VIII); and 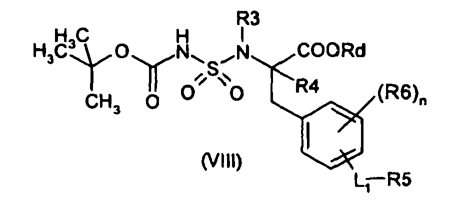
(b) reacting the sulfamide of formula (VIII) with an alcohol of formula (IX).
R2-OH (IX)

17. A process for producing a compound of formula 1 as defined in any one of claims 1 to 12, which process comprises reacting an amine of formula (X): 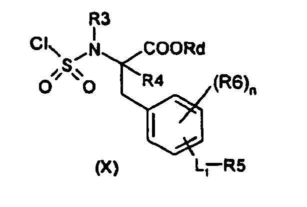 wherein Rd, R3, R4, R5, R6, L1 and n are as defined in any one of claims 1 to 10 with an amine of formula M 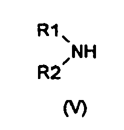 wherein R1 and R2 are as defined in any one of claims 1 to 10.

18. A compound of formula I as defined in any one of claims 1 to 12 or a pharmaceutically acceptable salt thereof for use in a method of treating the human or animal body.

19. Use of a compound of formula I as defined in any one of claims 1 to 12 or a pharmaceutically acceptable salt thereof in the manufacture of a medicament for the treatment of a pathological condition or disease which is multiple sclerosis, asthma, allergic rhinitis, allergic conjunctivitis, an inflammatory lung disease, rheumatoid arthritis, polydermatomyositis, septic arthritis, type I diabetes, rejection following organ transplantation, restenosis, rejection following autologous bone marrow transplantation, inflammatory sequelae of viral infections, atopic dermatitis, myocarditis, inflammatory bowel disease including ulcerative colitis and Crohn's disease, contact dermal hypersensitivity, psoriasis, tumor metastasis, atherosclerosis and cerebral ischemia.

20. A pharmaceutical composition comprising an effective amount of a compound as defined in any one of claims 1 to 12, or a pharmaceutically acceptable salt thereof, together with a pharmaceutically acceptable carrier.

## Patentansprüche

1. Verbindung der Formel (I): 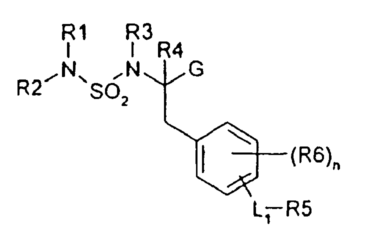 worin:
• G eine COOH-Gruppe oder eine Tetrazolyl-Gruppe ist;
• R1 und R2 unabhängig ausgewählt sind aus Wasserstoffatomen und Alkyl-, Alkenyl-, Alkinyl-, Cycloalkyl-, Cycloalkylalkyl-, Cycloalkylalkenyl-, Cycloalkylalkinyl-, Cycloalkenyl-, Cycloalkenylalkyl-, Cycloalkenylalkenyl-, Cylcoalkenylalkinyl-, Heterocyclyl-, Heterocyclylalkyl-, Heterocyclylalkenyl-, Heterocyclylalkinyl-, Aryl-, Arylalkyl-, Arylalkenyl-, Arylalkinyl-, Heteroaryl-, Heteroarylalkyl-, Heteroarylalkenyl- oder Heteroarylalkinylgruppen;
oder R1 und R2 bilden zusammen mit dem Stickstoffatom, an das sie gebunden sind, entweder ein 3- bis 14-gliedriges monocyclisches oder polycyclisches heterocyclisches Ringsystem oder eine 5- bis 14-gliedrige Heteroarylgruppe, wobei diese Gruppen 1 bis 5 Heteroatome umfassen, ausgewählt aus Stickstoff, Sauerstoff und Schwefel;
wobei die Alkyl-, Alkenyl- und Alkinylgruppen oder -reste unsubstituiert oder substituiert sind mit einem bis vier Substituenten, die gleich oder verschieden sein können und unabhängig ausgewählt sind aus Ra;
und wobei die Cycloalkyl-, Heterocyclyl-, Aryl- und Heteroarylgruppen oder -reste unsubstituiert oder substituiert sind mit eins bis vier Substituenten, die gleich oder verschieden sein können und unabhängig ausgewählt sind aus Rb;
• R3 und R4 unabhängig ausgewählt sind aus Wasserstoffatomen und Alkylgruppen mit 1 bis 6 Kohlenstoffatomen;
• R5 ausgewählt ist aus der Gruppe, bestehend aus 6- bis 14-gliedrigen monocyclischen oder polycyclischen Arylgruppen und 5- bis 14-gliedrigen monocyclischen oder polycyclischen Heteroarylgruppen, umfassend 1 bis 5 Heteroatome, ausgewählt aus Stickstoff, Sauerstoff und Schwefel;
wobei die Aryl- und Heteroarylgruppen oder -reste unsubstituiert oder substituiert sind mit eins bis vier Substituenten, die gleich oder verschieden sein können und unabhängig ausgewählt sind aus Rb;
• R6 eine Gruppe ist, ausgewählt aus -OH, -ORc, -NO₂, Halogen, -S (O) Rc, -S(O)₂Rc, -SRc, -S(O)₂ORc, -S (O) NRcRc, -S(O)₂NRcRc, -NRcRc, -O(CRcRc) mNRcRc, -C (O) Rc, -CO₂Rc, -CO₂(CRcRc)mCONRcRc, -OC (O) Rc, -CN, -C (O) NRcRc, -NRcC(O)Rc, -OC(O)NRcRc, -NRcC(O)ORc, -NRcC(O)NRcRc, -CRc(N-ORc), -CFH₂, -CF₂H, -Ra, -CF₃, Alkyl, Alkenyl und Alkinyl;
• n eine ganze Zahl von 0 bis 3 ist;
• Ra eine Gruppe ist, ausgewählt aus Alkyl, -OH, -ORc, -NO₂, Halogen, -S (O) Rc, -S(O)₂Rc, -SRc, -S(O)₂ORc, -S (O) NRcRc, -S(O)₂NRcRc, -NRcRc, -O(CRcRc)mNRcRc, -C (O) Rc, -CO₂Rc, -CO₂(CRcRc)mCONRcRc, -OC(O)Rc, -CN, -C(O)NRcRc, -NRcC(O)Rc, -OC(O)NRcRC, -NRcC(O)ORC, -NRcC(O)NRcRc, -CRc(N-ORc), -CFH₂, -CF₂H, -Ra oder -CF₃; wobei, wenn zwei oder mehr Rc-Gruppen vorliegen, diese gleich oder verschieden sein können;
• Rb eine Gruppe ist, ausgewählt aus -OH, -ORd, -NO₂, Halogen, -S(O)Rd, -S(O)₂Rd, -SRd, -S(O)₂ORd, -S (O) NRdRd, -S(O)₂NRdRd, -NRdRd, -O (CRdRd) mNRdRd, -C (O) Rd, -CO₂Rd, -CO₂(CRdRd)mCONRdRd, -OC(O)Rd, -CN, -C(O)NRdRd, -NRdC(O)Rd, -OC(O)NRdRd, -NRdC(O)ORd, -NRdC(O)NRdRd, -CRd(N-ORd), -CFH₂, -CF₂H, -Ra, -CF₃, Alkyl, Alkenyl, C₂₋₄-Alkinyl, Cycloalkyl, Cycloalkylalkyl, Heterocyclyl, Heterocyclylalkyl, Aryl, Arylalkyl, Heteroaryl oder Heteroarylalkyl; wobei die Alkyl-, Alkenyl-, Alkinyl-, Cycloalkyl-, Heterocyclyl-, Aryl- und Heteroarylgruppen oder -reste unsubstituiert oder substituiert sind mit eins bis vier Substituenten, die gleich oder verschieden sein können und unabhängig ausgewählt sind aus Ra;
• L1 entweder eine direkte Bindung ist oder eine Gruppe, ausgewählt aus der Gruppe, bestehend aus -N(Rc)-, -O-, -N(Rc)CO-, -CON(Rc)-, -O(CO)N(Rc)- und -N(Rc)(CO)O-;
• Rc ein Wasserstoffatom oder eine Alkylgruppe mit 1 bis 4 Kohlenstoffatomen ist;
• Rd Alkyl, Alkenyl, Alkinyl, Cycloalkyl, Cycloalkylalkyl, Cylcoalkylalkenyl, Cycloalkylalkinyl, Cycloalkenyl, Cycloalkenylalkyl, Cycloalkenylalkenyl, Cycloalkenylalkinyl, Heterocyclyl, Heterocyclylalkyl, Heterocyclylalkenyl, Heterocyclylalkinyl, Aryl, Arylalkyl, Arylalkenyl, Arylalkinyl, Heteroaryl, Heteroarylalkyl, Heteroarylalkenyl oder Heteroarylalkinyl ist;
wobei die Alkyl-, Alkenyl-, Alkinyl-, Cycloalkyl-, Cycloalkenyl-, Heterocyclyl-, Aryl- und Heteroarylgruppen unsubstituiert oder substituiert sind mit eins bis vier Substituenten, die gleich oder verschieden sein können und unabhängig ausgewählt sind aus Re;
• Re eine Gruppe ist, ausgewählt aus Alkyl, -OH, -ORc, -NO₂, Halogen, -S(O)Rc, -S(O)₂Rc, -SRc, -S(O)₂ORc, -S(O)NRcRc, -S(O)₂NRcRc, -NRcRc, -O(CRcRc)mNRcRc, -C(O)Rc, -CO₂Rc, -CO₂(CRcRc)mCONRcRc, -OC(O)Rc, -CN, -C(O)NRcRc, -NRcC(O)Rc, -OC(O)NRcRc, -NRcC(O)ORc, -NRcC(O)NRcRc, -CRc(N-ORc), -CFH₂, -CF₂H, -Ra oder -CF₃; wobei, wenn zwei oder mehrere Rc-Gruppen vorliegen, diese gleich oder verschieden sein können;
und jedes pharmazeutisch verträgliche Salz davon, ebenso wie jede Verbindung, die aus der Veresterung der Carboxylgruppe für den Fall, in dem G eine solche Carboxylgruppe ist, mit einem jeden Alkohol resultiert, und jedes pharmazeutisch verträgliche Salz davon.

2. Verbindung nach Anspruch 1, worin:
• jeder Alkylrest in R1, R2, R6, Ra, Rb, Rd oder Re 1 bis 20 Kohlenstoffatome aufweist;
• jeder Alkenylrest in R1, R2, R6, Rb und Rd 2 bis 20 Kohlenstoffatome aufweist;
• jeder Alkinylrest in R1, R2, R6 und Rd 2 bis 20 Kohlenstoffatome aufweist;
• jeder Arylrest ein monocyclischer oder polycyclischer C₆₋₁₄-Arylrest ist;
• jeder Heteroarylrest ein 5- bis 14-gliedriges Ringsystem ist, umfassend mindestens einen heteroaromatischen Ring und enthaltend mindestens ein Heteroatom, ausgewählt aus O, S und N;
• jeder Cycloalkylrest 3 bis 7 Kohlenstoffatome aufweist;
• jeder Cycloalkenylrest 4 bis 7 Kohlenstoffatome aufweist; und
• jeder Heterocyclylrest ein monocyclisches oder polycyclisches C₃-C₁₄-carbocyclisches Ringsystem ist, in dem ein oder mehrere Kohlenstoffatome durch ein Heteroatom ersetzt sind, ausgewählt aus N, O und S.

3. Verbindung nach Anspruch 2, worin:
• jeder Alkylrest 1 bis 6 Kohlenstoffatome aufweist;
• jeder Alkenylrest 2 bis 6 Kohlenstoffatome aufweist; und
• jeder Alkinylrest 2 bis 6 Kohlenstoffatome aufweist.

4. Verbindung nach einem der vorstehenden Ansprüche, worin G eine COOH-Gruppe ist ebenso wie jede Verbindung, die aus der Veresterung der COOH-Gruppe mit einem Alkohol resultiert.

5. Verbindung nach einem der vorstehenden Ansprüche, worin R3 und R4 Wasserstoffatome sind.

6. Verbindung nach einem der vorstehenden Ansprüche, worin R1 und R2 unabhängig ausgewählt sind aus Wasserstoffatomen und Alkyl-, Cycloalkyl-, Heterocyclylalkyl-, Aryl-, Arylalkyl-, Heteroarylalkylgruppen, wobei die Alkyl-, Alkenyl- und Alkinyl-, Cylcloalkyl-, Heterocyclyl-, Aryl- und Heteroarylgruppen oder -reste unsubstituiert oder substituiert sind;
oder R1 und R2 bilden zusammen mit dem Stickstoffatom, an das sie gebunden sind, ein 5- bis 8-gliedriges monocyclisches heterocyclisches Ringsystem, wobei das Ringsystem 1 bis 4 Heteroatome umfasst, ausgewählt aus Stickstoff, Sauerstoff und Schwefel, und unsubstituiert oder substituiert ist.

7. Verbindung nach einem der vorstehenden Ansprüche, worin R5 ausgewählt ist aus der Gruppe, bestehend aus 6- bis 14-gliedrigen monocyclischen oder polycyclischen Aryl- und 5-bis 14-gliedrigen monocyclischen oder polycyclischen Heteroarylgruppen, umfassend 1 bis 5 Heteroatome, ausgewählt aus Stickstoff, Sauerstoff und Schwefel, wobei die Aryl- und Heteroarylgruppen oder -reste unsubstituiert oder substituiert sind.

8. Verbindung nach Anspruch 7, worin die Aryl- oder Heteroarylgruppen unsubstituiert oder substituiert sind durch ein oder mehrere Halogenatom(e).

9. Verbindung nach einem der vorstehenden Ansprüche, worin L1 eine Gruppe ist, ausgewählt aus -NH-, -O- und -NHCO-.

10. Verbindung nach einem der vorstehenden Ansprüche, worin R5-L1- ausgewählt ist aus der Gruppe, umfassend Benzamid, Isonicotinamid, 2,6-Naphthyridin-1-ylamino, 2,7-Naphthyridin-1-ylamino, 2,6-Naphthyridin-1-yloxy und 2,7-Naphthyridin-1-yloxy, wobei diese Gruppen unsubstituiert oder substituiert sind.

11. Verbindung nach einem der vorstehenden Ansprüche, worin n gleich null ist.

12. Verbindung nach einem der vorstehenden Ansprüche, wobei es sich um eine der folgenden Verbindungen handelt:
• (2*S*)-2-{[(tert-Butylamino)sulfonyl]amino}-3-{4-[(3,5-dichlorisonicotinoyl)amino]phenyl}propionsäure
• Methyl-(2*S*)-2-(N-benzylaminosulfonylamino)-3-[4-(2,6-dichlorbenzoylamino)phenyl]propionat
• (2*S*)-2-(N-Benzylaminosulfonylamino)-3-[4-(2,6-dichlorbenzoylamino)phenyl]propionsäure
• Methyl-(2*S*)-3-{4-[(2,6-dichlorbenzoyl)amino]phenyl}-2-{[(dimethylamino)sulfonyl]amino}propionat
• (2*S*)-3-{4-[(2,6-Dichlorbenzoyl)amino]phenyl}-2-{[(dimethylamino)sulfonyl]amino}propionsäure
• Methyl-(2*S*)-3-{4-[(3,5-dichlorisonicotinoyl)-amino]phenyl}-2-{[(dimethylamino)sulfonyl]amino}propionat
• (2*S*)-3-{4-[(3,5-Dichlorisonicotinoyl)amino]phenyl}-2-{[(dimethylamino)sulfonyl]amino}propionsäure
• Methyl-(2*S*)-3-(4-{[1-(2,6-dichlorphenyl)methanoyl]amino}-phenyl)-2-(piperidin-1-sulfonylamino)propionat
• (2*S*)-3-(4-{[1-(2,6-Dichlorphenyl)methanoyl]amino}phenyl)-2-(piperidin-1-sulfonylamino)propionsäure
• Methyl-(2*S*)-3-{4-[(3,5-dichlorisonicotinoyl)amino]-phenyl}-2-{[(diisobutylamino)sulfonyl]amino}propionat
• (2*S*)-3-{4-[(3,5-Dichlorisonicotinoyl)amino]phenyl}-2-{[(diisobutylamino)sulfonyl]amino}propionsäure
• Methyl-(2*S*)-2-({[benzyl(ethyl)amino]sulfonyl}amino)-3-{4-[(3,5-dichlorisonicotinoyl)amino]phenyl}propionat
• (2*S*)-2-{[(Benzylethylamino)sulfonyl]amino}-3-{4[(3,5-dichlorisonicotinoyl)amino]phenyl}propionsäure
• Methyl-(2*S*)-3-{4-((3,5-dichlorisonicotinoyl)amino]-phenyl}-2-{[(dibutylamino)sulfonyl]amino}propionat
• (2*S*)-3-{4-[(3,5-dichlorisonicotinoyl)amino]phenyl}-2-{[(dibutylamino)sulfonyl]amino}propionsäure
• Methyl-(2*S*)-3-{4-[(3,5-dichlorisonicotinoyl)amino]-phenyl}-2-({[[2-(3,4-dimethoxyphenyl)ethyl]isobutylamino]sulfonyl}amino)propionat
• (2*S*)-3-{4-[(3,5-Dichlorisonicotinoyl)amino]phenyl}-2-({[[2-(3,4-dimethoxyphenyl)ethyl]isobutylamino]sulfonyl}amino)propionsäure
• Methyl-(2*S*)-2-({[bis(thien-2-ylmethyl)amino]sulfonyl}amino)-3-{4-[(3,5-dichlorisonicotinoyl)amino]-phenyl}propionat
• (2*S*)-2-({[Bis(thien-2-ylmethyl)amino]sulfonyl}amino)-3-{4-[(3,5-dichlorisonicotinoyl)amino]phenyl}propionsäure
• Methyl-(2*S*)-3-{4-[(3,5-dichlorisonicotinoyl)amino]-phenyl}-2-({[methyl(2-pyridin-2-ylethyl)amino]sulfonyl}amino)propionat
• (2*S*)-3-{4-[(3,5-Dichlorisonicotinoyl)amino]phenyl}-2-({[methyl(2-pyridin-2-ylethyl)amino]sulfonyl}amino)propionsäure
• Methyl-(2*S*)-2-{[(cyclohexylmethylamino)sulfonyl]amino}-3-{4-[(3,5-dichlorisonicotinoyl)amino]phenyl}propionat
• (2*S*)-2-{[(Cyclohexylmethylamino)sulfonyl]amino}-3-{4-[(3,5-dichlorisonicotinoyl)amino]phenyl}propionsäure
• Methyl-(2*S*)-3-{4-[(3,5-dichlorisonicotinoyl)amino]-phenyl}-2-({[(3-methylbutyl)(thien-2-ylmethyl)amino]-sulfonyl}amino)propionat
• (2*S*)-3-{4-[(3,5-Dichlorisonicotinoyl)amino]phenyl}-2-({[(3-methylbutyl)(thien-2-ylmethyl)amino]-sulfonyl}amino)propionsäure
• Methyl-(2S)-3-{4-[(3,5-dichlorisonicotinoyl)amino]-phenyl}-2-[(piperidin-1-ylsulfonyl)amino]propionat
• (2S)-3-{4-[(3,5-Dichlorisonicotinoyl)amino]phenyl}-2-[(piperidin-1-ylsulfonyl)amino]propionsäure
• Methyl-(2S)-2-[(azepan-1-ylsulfonyl)amino]-3-{4-[(3,5-dichlorisonicotinoyl)amino]phenyl}propionat
• (2*S*)-2-[(Azepan-1-ylsulfonyl)amino]-3-{4-[(3,5-dichlorisonicotinoyl)amino]phenyl}propionsäure
• Methyl-(2S)-3-{4-[(3,5-dichlorisonicotinoyl)amino]-phenyl}-2-[(morpholin-4-ylsulfonyl)amino]propionat
• (2*S*)-3-{4-[(3,5-Dichlorisonicotinoyl)amino]phenyl}-2-[(morpholin-4-ylsulfonyl)amino]propionsäure
• Methyl-(2*S*)-3-{4-[(3,5-dichlorisonicotinoyl)amino]-phenyl}-2-[(thiomorpholin-4-ylsulfonyl)amino]propionat
• (2*S*)-3-{4-[(3,5-Dichlorisonicotinoyl)amino]phenyl}-2-[(thiomorpholin-4-ylsulfonyl)amino]propionsäure
• Methyl-(2*S*)-2-{[(dimethylamino)sulfonyl]amino}-3-[4-(2,6-naphthyridin-1-yloxy)phenyl]propionat
• (2*S*)-2-{[(Dimethylamino)sulfonyl]amino}-3-[4-(2,6-naphthyridin-1-yloxy)phenyl]propionsäure
• Methyl-(2*S*)-2-{[(diisobutylamino)sulfonyl]amino}-3-[4-(2,6-naphthyridin-1-yloxy)phenyl]propionat
• (2*S*)-2-{[(Diisobutylamino)sulfonyl]amino}-3-[4-(2,6-naphthyridin-1-yloxy)phenyl]propionsäure
• Methyl-(2*S*)-2-{[(diisobutylamino)sulfonyl]amino}-3-[4-(2,6-naphthyridin-1-ylamino)phenyl]propionat
• (2S)-2-{[(Diisobutylamino)sulfonyl]amino}-3-[4-(2,6-naphthyridin-1-ylamino)phenyl]propionsäure
• Methyl-(2*S*)-3-{4-[(2,6-dichlorbenzoyl)amino]phenyl}-2-{[(2,6-dimethylpiperidin-1-yl)sulfonyl]amino}propionat
• (2S)-3-{4-[(2,6-Dichlorbenzoyl)amino]phenyl}-2-([(2,6-dimethylpiperidin-1-yl)sulfonyl]amino}propionsäure
• Methyl-(2*S*)-3-(4-[(3,5-dichlorisonicotinoyl)amino]-phenyl}-2-{[(diisopropylamino)sulfonyl]amino}propionat
• (2*S*)-3-(4-[(3,5-Dichlorisonicotinoyl)amino] phenyl)-2-{[(diisopropylamino)sulfonyl]amino}propionsäure
• Methyl-(2*S*)-3-(4-[(3,5-dichlorisonicotinoyl)amino]-phenyl}-2-{[(2,6-dimethylpiperidin-1-yl)sulfonyl]-amino}propionat
• (2*S*)-3-(4-[(3,5-Dichlorisonicotinoyl)amino]phenyl)-2-{[(2,6-dimethylpiperidin-1-yl)sulfonyl]amino}propionsäure
• Methyl-(2*S*)-2-{[(dimethylamino)sulfonyl]amino}-3-[4-(2,6-naphthyridin-1-ylamino)phenyl]propionat
• (2*S*)-2-{[(Dimethylamino)sulfonyl]amino}-3-[4-(2,6-naphthyridin-1-ylamino)phenyl]propionsäure
• Methyl-(2*S*)-2-{[(diisopropylamino)sulfonyl]amino}-3-[4-(2,6-naphthyridin-1-ylamino)phenyl]propionat
• (2*S*)-2-{[(Diisopropylamino)sulfonyl)amino}-3-[4-(2,6-naphthyridin-1-ylamino)phenyl]propionsäure
• Methyl-(2*S*)-2-({[cyclohexyl(isopropyl)amino]sulfonyl}amino)-3-[4-(2,6-naphthyridin-1-ylamino)phenyl]propionat
• (2*S*)-2-({[Cyclohexyl(isopropyl)amino]sulfonyl}amino)-3-[4-(2,6-naphthyridin-1-ylamino)phenyl]propionsäure
• Methyl-(2*S*)-2-{[(diisopropylamino)sulfonyl]amino}-3-[4-(2,6-naphthyridin-1-yloxy)phenyl]propionat
• (2*S*)-2-{[(Diisopropylamino)sulfonyl]amino}-3-[4-(2,6-naphthyridin-1-yloxy)phenyl]propionsäure
• Methyl-(2*S*)-2-{[(diisopropylamino)sulfonyl)amino}-3-[4-(2,6-naphthyridin-1-ylamino)phenyl]propionat
• (2*S*)-2-{[(Diisopropylamino)sulfonyl]amino}-3-[4-(2,6-naphthyridin-1-ylamino)phenyl]propionsäure
• Methyl-(2*S*)-2-{[(2,6-dimethylpiperidin-1-yl)sulfonyl]-amino}-3-[4-(2,6-naphthyridin-1-ylamino)phenyl]propionat
• (2*S*)-2-{[(2,6-Dimethylpiperidin-1-yl)sulfonyl]amino}-3-[4-(2,6-naphthyridin-1-ylamino)phenyl]propionsäure
• Methyl-(2*S*)-2-{[(2,6-dimethylpiperidin-1-yl)sulfonyl]-amino}-3-[4-(2,6-naphthyridin-1-yloxy)phenyl]propionat
• (2*S*)-2-{[(2,6-Dimethylpiperidin-1-yl)sulfonyl]amino}-3-[4-(2,6-naphthyridin-1-yloxy)phenyl]propionsäure
• Methyl-(2*S*)-2-({[benzyl(isopropyl)amino]sulfonyl}amino)-3-{4-[(2,6-dichlorbenzoyl)amino]phenyl}propionat
• (2*S*)-2-({[Benzyl(isopropyl)amino]sulfonyl}amino)-3-{4-[(2,6-dichlorbenzoyl)amino]phenyl}propionsäure
• Methyl-(2*S*)-2-({[benzyl(isopropyl)amino]sulfonyl}amino)-3-{4-[(3,5-dichlorisonicotinoyl)amino]phenyl}propionat
• (2*S*)-2-({[Benzyl(isopropyl)aminolsulfonyl}amino)-3-{4-[(3,5-dichlorisonicotinoyl)amino]phenyl}propionsäure
• Methyl-(2*S*)-2-({[isopropyl(thien-2-ylmethyl)amino]-sulfonyl}amino)-3-{4-[(3,5-dichlorisonicotinoyl)-amino]phenyl}propionat
• (2*S*)-2-({[Isopropyl(thien-2-ylmethyl)amino]sulfonyl}amino)-3-{4-[(3,5-dichlorisonicotinoyl)amino]-phenyl}propionsäure
• Methyl-(2*S*)-2-({[isopropyl(thien-2-ylmethyl)amino]-sulfonyl}amino)-3-{4-[(3,5-dichlorbenzoyl)amino]-phenyl}propionat
• (2*S*)-2-({[Isopropyl(thien-2-ylmethyl)amino]sulfonyl}amino)-3-{4-[(3,5-dichlorbenzoyl)amino]phenyl}propionsäure
• Methyl-(2*S*)-3-{4-[(2,6-dichlorisonicotinoyl)amino]-phenyl)-2-[({isobutyl[(1*S*)-1-phenylethyl]amino}sulfonyl)-amino]propionat
• (2*S*)-3-{4-[(2,6-Dichlorisonicotinoyl)amino]phenyl}-2-[({isobutyl[(1*S*)-1-phenylethyl]amino}sulfonyl)-amino]propionsäure
• Methyl-(2*S*)-2-({[cyclopentyl(isopropyl)amino]sulfonyl}amino)-3-{4-[(3,5-dichlorisonicotinoyl)amino]-phenyl}propionat
• (2*S*)-2-({[Cyclopentyl(isopropyl)amino]sulfonyl}amino)-3-{4-[(3,5-dichlorisonicotinoyl)amino]phenyl}propionsäure
• Methyl-(2S)-3-{4-[(3,5-dichlorisonicotinoyl)amino]-phenyl}-2-({[isobutyl(isopropyl)amino]sulfonyl}amino)propionat
• (2*S*)-3-{4-[(3,5-Dichlorisonicotinoyl)amino]phenyl}-2-({[isobutyl(isopropyl)amino]sulfonyl}amino)propionsäure
• Methyl-(2*S*)-2-({[cyclohexyl(isopropyl)amino]sulfonyl}amino)-3-[4-(2,6-naphthyridin-1-yloxy)phenyl]propionat
• (2*S*)-2-({[Cyclohexyl(isopropyl)amino]sulfonyl}amino)-3-[4-(2,6-naphthyridin-1-yloxy)phenyl]propionsäure
• Methyl-(2*S*)-3-{4-[(3,5-dichlorisonicotinoyl)amino]phenyl)-2-[({(isobutyl[(1R)-1-phenylethyl]amino}sulfonyl)-amino]propionat
• (2*S*)-3-{4-[(3,5-Dichlorisonicotinoyl)amino]phenyl}-2-[({isobutyl[(1R)-1-phenylethyl]amino}sulfonyl)-amino]propionsäure
• Methyl-(2*S*)-2-({[methyl(phenyl)amino]sulfonyl}amino)-3-{4-[(2,6-dichlorbenzoyl)amino]phenyl}propionat
• (2*S*)-2-({[Methyl(phenyl)amino]sulfonyl}amino)-3-{4-[(2,6-dichlorbenzoyl)amino]phenyl}propionsäure
• Methyl-(2*S*)-({[2-(phenylsulfonyl)phenyl]amino}sulfonyl)-amino]-3-{4-[(3,5-dichlorisonicotinoyl)amino]-phenyl}propionat
• (2*S*)-({[2-(Phenylsulfonyl)phenyl]amino}sulfonyl)amino]-3-{4-[(3,5-dichlorisonicotinoyl)amino]phenyl}propionsäure

13. Verfahren zur Herstellung einer Verbindung der Formel (I), wie in einem der Ansprüche 1 bis 12 definiert, wobei das Verfahren die Umsetzung eines Amins der Formel (III): 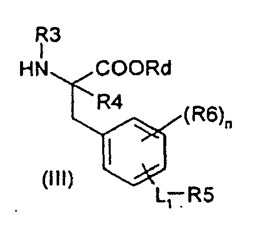 worin Rd, R3, R4, R5, R6, L1 und n wie in einem der Ansprüche 1 bis 12 definiert sind, mit einem entsprechenden Sulfamoylchlorid der Formel (IV): 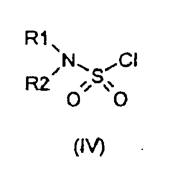 worin R1 und R2 wie in einem der Ansprüche 1 bis 12 definiert sind, umfasst.

14. Verfahren zur Herstellung einer Verbindung der Formel (I), wie in einem der Ansprüche 1 bis 12 definiert, wobei das Verfahren die Umsetzung eines Amins der Formel (IIa): 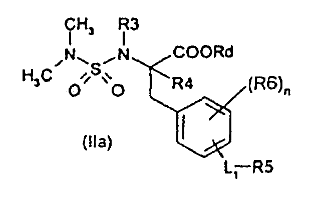 worin Rd, R3, R4, R5, R6, L1 und n wie in einem der Ansprüche 1 bis 12 definiert sind, mit einem Amin der Formel (V) 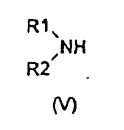 worin R1 und R2 wie in einem der Ansprüche 1 bis 12 definiert sind, umfasst.

15. Verfahren zur Herstellung einer Verbindung der Formel (I), wie in einem der Ansprüche 1 bis 12 definiert, wobei das Verfahren die Umsetzung eines Amins der Formel (III): 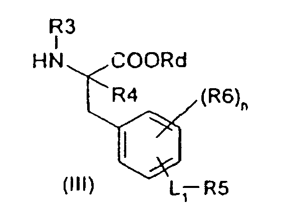 worin R3, R4, R5, R6, Rd, L1 und n wie in einem der Ansprüche 1 bis 12 definiert sind, mit einem Sulfamid der Formel (VII) 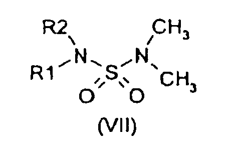 worin R1 und R2 wie in einem der Ansprüche 1 bis 12 definiert sind, umfasst.

16. Verfahren zur Herstellung einer Verbindung der Formel (I), wie in einem der Ansprüche 1 bis 12 definiert, wobei das Verfahren die Schritte umfasst :
(a) Umsetzung eines Amins der Formel (III) : 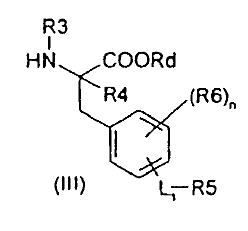 worin R3, R4, R5, R6, Rd, L1 und n wie in einem der Ansprüche 1 bis 12 definiert sind, mit tert-Butanol und Chlorsulfonylisocyanat, um das Sulfamid der Formel (VIII) zu erhalten; und 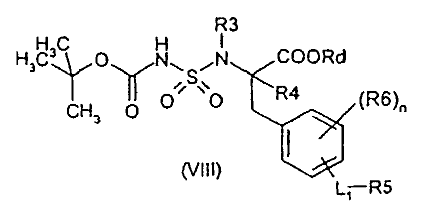
(b) Umsetzung des Sulfamids der Formel (VIII) mit einem Alkohol der Formel (IX)
R2-OH (IX)

17. Verfahren zur Herstellung einer Verbindung der Formel (I), wie in einem der Ansprüche 1 bis 12 definiert, wobei das Verfahren die Umsetzung eines Amins der Formel (X): 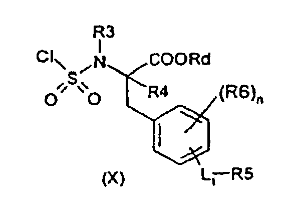 worin Rd, R3, R4, R5, R6, L1 und n wie in einem der Ansprüche 1 bis 10 definiert sind, mit einem Amin der Formel (V) 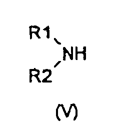 worin R1 und R2 wie in einem der Ansprüche 1 bis 10 definiert sind, umfasst.

18. Verbindung der Formel (I), wie in einem der Ansprüche 1 bis 12 definiert, oder ein pharmazeutisch verträgliches Salz davon, zur Verwendung in einem Verfahren zur Behandlung des menschlichen oder tierischen Körpers.

19. Verwendung einer Verbindung der Formel (I), wie in einem der Ansprüche 1 bis 12 definiert, oder eines pharmazeutisch verträglichen Salzes davon, zur Herstellung eines Medikaments zur Behandlung eines Krankheitszustandes oder einer Erkrankung, nämlich Multiple Sklerose, Asthma, allergische Rhinitis, allergische Konjunktivitis, eine entzündliche Lungenerkrankung, rheumatoide Arthritis, Polydermatomyositis, septische Arthritis, Typ I-Diabetes, Abstoßung infolge einer Organstransplantation, Restenose, Abstoßung infolge autologer Knochenmarkstransplantation, entzündliche Folgeerscheinungen von viralen Infektionen, atopische Dermatitis, Myokarditis, entzündliche Darmkrankheit einschließlich eitrige Kolitis und Crohnsche Krankheit, Kontakthautüberempfindlichkeit, Psoriasis, Tumormetastasen, Atherosklerose und cerebrale Ischämie.

20. Pharmazeutische Zusammensetzung, umfassend eine wirksame Menge einer Verbindung, wie in einem der Ansprüche 1 bis 12 definiert, oder eines pharmazeutisch verträglichen Salzes davon, zusammen mit einem pharmazeutisch verträglichen Trägerstoff.

## Revendications

1. Composé de formule (I) : 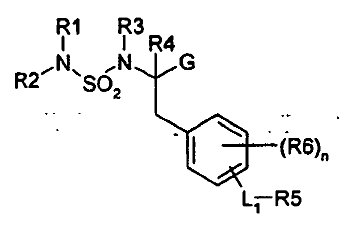 dans laquelle :
• G est un groupe COOH ou un groupe tétrazolyle ;
• R1 et R2 sont indépendamment choisis parmi les atomes d'hydrogène et les groupes alkyles, alcényles, alcynyles, cycloalkyles, cycloalkylalkyles, cycloalkylalcényles, cycloalkylalcynyles, cycloalcényles, cycloalcénylalkyles, cycloalcénylalcényles, cycloalcénylalcynyles, hétérocyclyles, hétérocyclylalkyles, hétérocyclylalcényles, hétérocyclylalcynyles, aryles, arylalkyles, arylalcényles, arylalcynyles, hétéroaryles, hétéroarylalkyles, hétéroarylalcényles ou hétéroarylalcynyles ;
ou R1 et R2 forment, conjointement avec l'atome d'azote auquel ils sont attachés, soit un système à noyau hétérocyclique monocyclique ou polycyclique à 3 à 14 chaînons, soit un groupe hétéroaryle à 5 à 14 chaînons dans lequel lesdits groupes comprennent de 1 à 5 hétéroatomes choisis parmi l'azote, l'oxygène et le soufre ;
où lesdits groupes ou fragments alkyles, alcényles et alcynyles sont non substitués ou substitués par un à quatre substituants, qui peuvent être identiques ou différents et sont indépendamment choisis parmi Ra ;
et où lesdits groupes ou fragments cycloalkyles, hétérocyclyles, aryles et hétéroaryles sont non substitués ou substitués par un à quatre substituants, qui peuvent être identiques ou différents et sont indépendamment choisis parmi Rb ;
• R3 et R4 sont indépendamment choisis parmi les atomes d'hydrogène et les groupes alkyles ayant de 1 à 6 atomes de carbone ;
• R5 est choisi dans le groupe constitué par les groupes aryles monocycliques ou polycycliques à 6 à 14 chaînons et les groupes hétéroaryles monocycliques ou polycycliques à 5 à 14 chaînons comprenant de 1 à 5 hétéroatomes choisis parmi l'azote, l'oxygène et le soufre ;
où lesdits groupes ou fragments aryles et hétéroaryles sont non substitués ou substitués par un à quatre substituants, qui peuvent être identiques ou différents et sont indépendamment choisis parmi Rb ;
• R6 est un groupe choisi parmi -OH, -ORc, -NO₂, halogène, -S(O)Rc, -S(O)₂Rc. -SRc, - S(O)₂ORc, -S(O)NRcRc -S(O)₂NRcRc, -NRcRc, -O(CRcRc)mNRcRc, -C(O)Rc, **-**CO₂Rc, -CO₂(CRcRc)mCONRcRc, -OC(O)Rc, -CN, -C(O)NRcRc, -NRcC(O)Rc, **-**OC(O)NRcRc, -NRcC(O)ORc, -NRcC(O)NRcRc, -CRc(N-ORc), -CFH₂, -CF₂H, -Ra, -CF₃, alkyle, alcényle et alcynyle ;
• n est un entier de 0 à 3
• Ra est un groupe choisi parmi alkyle, -OH, -ORc, -NO₂, halogène, -S.(O)Rc, -S(O)₂Rc, - SRc, -S(O)₂ORc, S(O)NRcRc, -S(O)₂NRcRc, -NRcRc, -O(CRcRc)mNRcRc, -C(O)Rc, -CO₂Rc, -CO₂(CRcRc)mCONRcRc, -OC(O)Rc, -CN, -C(O)NRcRc, -NRcC(O)Rc, - OC(O)NRcRc, -NRcC(O)ORc, -NRcC(O)NRcRc, -CRc(N-0Rc), -CFH₂, - CF₂H, -Ra, ou -CF₃ ; où si deux groupes Rc ou plus sont présents, ils peuvent être identiques ou différents ;
• Rb est un groupe choisi parmi -OH, -ORd. -NO₂, halogène, -S(O)Rd, -S(O)₂Rd ,- SRd, _ S(O)₂ORd,- S(O)NRdRd, -S(O)₂NRdRd, -NRdRd, - O(CRdRd)mNRdRd, -C(O)Rd, - CO₂Rd, -CO₂(CRdRd)mCONRdRd, -OC(O)Rd, -CN, -C(O)NRdRd, -NRdC(O)Rd, -OC(O)NRdRd, -NRdC(O)ORd, -NRdC(O)NRdRd, -CRd(N-ORd), -CFH₂, -CF₂H, -Ra, -CF₃ , alkyle, alcényle, alcynyle en C₂₋₄, cycloalkyle, cycloalkylalkyle, hétérocyclyle, hétérocyclylalkyle, aryle, arylalkyle, hétéroaryle ou hétéroarylalkyle ; où lesdits groupes ou fragments alkyles, alcényles, alcynyles, cycloalkyles, hétérocyclyles, aryles et hétéroaryles sont non substitués ou substitués par un à quatre substituants qui peuvent être identiques ou différents et sont indépendamment choisis parmi Ra ;
• L1 est soit une liaison directe soit un groupe choisi dans le groupe constitué par -N(Rc)-, - O-, -N(Rc)CO-, -CON(Rc)-, -O(CO)N(Rc)- et -N(Rc)(CO)O-;
• Rc est un atome d'hydrogène ou un groupe alkyle ayant de 1 à 4 atomes de carbone ;
• Rd est aryle, alcényle, alcynyle, cycloalkyle, cycloalkylalkyle, cycloalkylalcényle, cycloalkylalcynyle, cycloalcényle, cycloalcénylalkyle, cycloalcénylalcényle, cycloalcénylalcynyle, hétérocyclyle, hétérocyclylalkyle, hétérocyclylalcényle, hétérocyclylalcynyle, aryle, arylalkyle, arylalcényle, arylalcynyle, hétéroaryle, hétéroarylalkyle, hétéroarylalcényle ou hétéroarylalcynyle ; où lesdits groupes alkyles, alcényles, alcynyles, cycloalkyles, cycloalcényles, hétérocyclyles, aryles et hétéroaryles sont non substitués ou substitués par un à quatre substituants, qui peuvent être identiques ou différents et sont indépendamment choisis parmi Re ;
• Re est un groupe choisi parmi alkyle, **-**OH, -ORc, -NO₂. halogène, -S(O)Rc, -S(O)₂Rc, -SRc, -S(O)₂ORc, -S(O)NRcRc, -S(O)₂NRcRc, -NRcRc, -O(CRcRc)mNRcRc, -C(O)Rc, -CO₂Rc, -CO₂(CRcRc)mCONRcRc. -OC(O)Rc, -CN, -C(O)NRcRc, - NRcC(O)Rc, -OC(O)NRcRc, -NRcC(O)ORc. -NRcC(O)NRcRc, -CRc(N-ORc), CFH₂, -CF₂H, -Ra, ou -CF₃ ; où si deux groupes Rc ou plus sont présents, ceux-ci peuvent être identiques ou différents ;
et tout sel pharmaceutiquement acceptable de celui-ci ainsi que tout composé résultant de l'estérification, avec tout alcool, du groupe carboxylique dans le cas où G est un tel groupe carboxylique et tout sel pharmaceutiquement acceptable de celui-ci.

2. Composé selon la revendication 1, dans lequel :
• chaque groupe alkyle dans R1, R2, R6, Ra, Rb, Rd ou Re a 1 à 20 atomes de carbone ;
• chaque groupe alcényle dans R1, R2, R6, Rb et Rd a 2 à 20 atomes de carbone ;
• chaque groupe alcynyle dans R1, R2, R6 et Rd a 2 à 20 atomes de carbone ;
• chaque groupe aryle est un groupe aryle en C₆₋₁₄ monocyclique ou polycyclique ;
• chaque groupe hétéroaryle est un système cyclique à 5 à 14 chaînons comprenant au moins un cycle hétéro-aromatique et contenant au moins un hétéroatome choisi parmi O, S et N ;
• chaque groupe cycloalkyle a 3 à 7 atomes de carbone ;
• chaque groupe cycloalcényle a 4 à 7 atomes de carbone ; et
• chaque groupe hétérocyclyle est un système à noyau carbocyclique en C₃-C₁₄ monocyclique ou polycyclique dans lequel un ou plusieurs des atomes de carbone sont remplacés par un hétéroatome choisi parmi N, O et S.

3. Composé selon la revendication 2, dans lequel :
• chaque groupe alkyle a 1 à 6 atomes de carbone ;
• chaque groupe alcényle a 2 à 6 atomes de carbone ; et
• chaque groupe alcynyle a 2 à 6 atomes de carbone.

4. Composé selon l'une quelconque des revendications précédentes, dans lequel G est un groupe COOH ainsi que tout composé résultant de l'estérification, avec un alcool, du groupe COOH.

5. Composé selon l'une quelconque des revendications précédentes, dans lequel R3 et R4 sont des atomes d'hydrogène.

6. Composé selon l'une quelconque des revendications précédentes, dans lequel R1 et R2 sont indépendamment choisis parmi les atomes d'hydrogène et les groupes alkyles, cycloalkyles, hétérocyclylalkyles, aryles, arylalkyles, hétéroarylalkyles, dans lesquels lesdits groupes ou fragments alkyles, alcényles et alcynyles, cycloalkyles, hétérocyclyles, aryles et hétéroaryles sont non substitués ou substitués ;
ou R1 et R2 forment, conjointement avec l'atome d'azote auquel ils sont attachés, un système à noyau hétérocyclique monocyclique à 5 à 8 chaînons, dans lequel ledit système à noyau comprend de 1 à 4 hétéroatomes choisis parmi l'azote, l'oxygène et le soufre et est non substitué ou substitué.

7. Composé selon l'une quelconque des revendications précédentes, dans lequel R5 est choisi dans le groupe constitué par les groupes aryles monocycliques ou polycycliques à 6 à 14 chaînons et les groupes hétéroaryles monocycliques ou polycycliques à 5 à 14 chaînons, comprenant de 1 à 5 hétéroatomes choisis parmi l'azote, l'oxygène et le soufre, dans lequel lesdits groupes ou fragments aryles et hétéroaryles sont non substitués ou substitués.

8. Composé selon la revendication 7, dans lequel lesdits groupes aryles ou hétéroaryles sont non substitués ou substitués par un ou plusieurs atomes d'halogène.

9. Composé selon l'une quelconque des revendications précédentes, dans lequel L1 est un groupe choisi parmi -NH-, -O- et -NHCO-.

10. Composé selon l'une quelconque des revendications précédentes, dans lequel R5-L1- est choisi dans le groupe comprenant le benzamide, l'isonicotinamide, le 2,6-naphtyridin-1-ylamino, le 2,7-naphtyridin-1-ylamino ; le 2,6-naphtyridin-1-yloxy et le 2,7-naphtyridin-1-yloxy, dans lequel lesdits groupes sont non substitués ou substitués.

11. Composé selon l'une quelconque des revendications précédentes, dans lequel n est zéro.

12. Composé selon l'une quelconque des revendications précédentes, qui est l'un parmi :
• l'acide (2*S*)-2-{[(tert-butylamino)sulfonyl]amino}-3-{4-[(3,5-dichloroisonicotinoyl)amino]phényl}propionique
• le (2*S*)-2-(*N*-benzylaminosulfonamino)-3-[4-(2,6-dichlorobenzoylamino)phényl]propionate de méthyle
• l'acide (2*S*)-2-(*N*-benzylaminosulfonamino)-3-[4-(2,6-dichlorobenzoylamino)phényl]propionique
• le (2*S*)-3-{4-[(2,6-dichlorobenzoyl)amino]phényl}-2-{[(diméthylamino)sulfonyl]amino}propionate de méthyle
• l'acide (2S)-3-{4-[(2,6-dichlorobenzoyl)amino]-phényl}-2-{[(diméthylamino)sulfonyl]amino}propionique
• le (2*S*)-3-{4-[(3,5-dichloroisonicotinoyl)amino]-phényl}-2-{[(diméthylamino)sulfonyl]amino}propionate de méthyle
• l'acide (2*S*)-3-{4-[(3,5-dichloroisonicotinoyl)amino]-phényl}-2-{[(diméthylamino)sulfonyl]amino}propionique
• le (2*S*)-3-(4-{[1-(2,6-dichlorophényl)méthanoyl]-amino}phényl)-2-(pipéridine-1-sulfonylamino)-propionate de méthyle
• l'acide (2*S*)-3-(4-{[1-(2,6-dichlorophényl)méthanoyl]-amino}phényl)-2-(pipéridine-1-sulfonylamino)-propionique
• le (2*S*)-3-{4-[(3,5-dichloroisonicotinoyl)amino]-phényl}-2-{[(diisobutylamino)sulfonyl]amino}propionate de méthyle
• l'acide (2*S*)-3-{4-[(3,5-dichloroisonicotinoyl)amino]-phényl}-2-{[(diisobutylamino)sulfonyl]amino}propionique
• le (2*S*)-2-({[benzyl(éthyl)amino]sulfonyl}amino)-3-{4-[(3,5-dichloroisonicotinoyl)amino]phényl}propionate de méthyle
• l'acide (2*S*)-2-{[(benzyléthylamino)sulfonyl]amino}-3-{4-[(3,5-dichloroisonicotinoyl)amino]phényl}propionique
• le (2*S*)-3-{4-[(3,5-dichloroisonicotinoyl)amino]-phényl}-2-{[(dibutylamino)sulfonyl]amino}propionate de méthyle
• l'acide (2*S*)-3-{4-[(3,5-dichloroisonicotinoyl)amino]-phényl}-2-{[(dibutylamino)sulfonyl]amino}propionique
• le (2*S*)-3-{4-[(3,5-dichloroisonicotinoyl)amino]-phényl}-2-({[[2-(3,4-diméthoxyphényl)éthyl]isobutylamino]sulfonyl}amino)propionate de méthyle
• l'acide (2*S*)-3-{4-[(3,5-dichloroisonicotinoyl)amino]-phényl}-2-({[[2-(3,4-diméthoxyphényl)éthyl]isobutylamino]sulfonyl}amino)propionique
• le (2*S*)-2-({[bis(thién-2-ylméthyl)amino]sulfonyl}amino)-3-{4-[(3,5-dichloroisonicotinoyl)amino]-phényl}propionate de méthyle
• l'acide (2*S*)-2-({[bis(thién-2-ylméthyl)amino]-sulfonyl}amino)-3-{4-[(3,5-dichloroisonicotinoyl)-amino]phényl}propionique
• le (2*S*)-3-{4-[(3,5-dichloroisonicotinoyl)amino]-phényl}-2-({[méthyl(2-pyridin-2-yléthyl)amino]-sulfonyl}amino)propionate de méthyle
• l'acide (2*S*)-3-{4-[(3,5-dichloroisonicotinoyl)amino]-phényl}-2-({[méthyl(2-pyridin-2-yléthyl)amino]-sulfonyl}amino)propionique
• le (2*S*)-2-{[(cyclohexylméthylamino)sulfonyl]amino}-3-{4-[(3,5-dichloroisonicotinoyl)amino]phényl}propionate de méthyle
• l'acide (2*S*)-2-{[(cyclohexylméthylamino)sulfonyl]-amino}-3-{4-[(3,5-dichloroisonicotinoyl)amino]-phényl}propionique
• le (2*S*)-3-{4-[(3,5-dichloroisonicotinoyl)amino]-phényl}-2-({[(3-méthylbutyl)(thién-2-ylméthyl)amino]-sulfonyl}amino)propionate de méthyle
• l'acide (2*S*)-3-{4-[(3,5-dichloroisonicotinoyl)amino]-phényl}-2-({[(3-méthylbutyl)(thién-2-ylméthyl)amino]-sulfonyl}amino)propionique
• le (2*S*)-3-{4-[(3,5-dichloroisonicotinoyl)amino]-phényl}-2-[(pipéridin-1-ylsulfonyl)amino]propionate de méthyle
• l'acide (2*S*)-3-{4-[(3,5-dichloroisonicotinoyl)amino]-phényl}-2-[(pipéridin-1-ylsulfonyl)amino]propionique
• le (2*S*)-2-[(azépan-1-ylsulfonyl)amino]-3-{4-[(3,5-dichloroisonicotinoyl)amino]phényl}propionate de méthyle
• l'acide (2*S*)-2-[(azépan-1-ylsulfonyl)amino]-3-{4-[(3,5-dichloroisonicotinoyl)amino]phényl}propionique
• le (2*S*)-3-{4-[[3,5-dichloroisonicotinoyl)amino]-phényl}-2-[(morpholin-4-ylsulfonyl)amino]propionate de méthyle
• l'acide (2*S*)-3-{4-[[3,5-dichloroisonicotinoyl)amino]-phényl}-2-[(morpholin-4-ylsulfonyl)amino]propionique
• le (2*S*)-3-{4-[(3,5-dichloroisonicotinoyl)amino]-phényl}-2-[(thiomorpholin-4-ylsulfonyl)amino]-propionate de méthyle
• l'acide (2*S*)-3-{4-[(3,5-dichloroisonicotinoyl)amino]-phényl}-2-[(thiomorpholin-4-ylsulfonyl)amino]-propionique
• le (2*S*)-2-{[(diméthylamino)sulfonyl]amino}-3-[4-(2,6-naphtyridin-1-yloxy)phényl]propionate de méthyle
• l'acide (2*S*)-2-{[(diméthylamino)sulfonyl]amino}-3-[4-(2,6-naphtyridin-1-yloxy)phényl]propionique
• le (2*S*)-2-{[(diisobutylamino)sulfonyl]amino}-3-[4-(2,6-naphtyridin-1-yloxy)phényl]propionate de méthyle
• l'acide (2*S*)-2-{[(diisobutylamino)sulfonyl]amino}-3-[4-(2,6-naphtyridin-1-yloxy)phényl]propionique
• le (2*S*)-2-{[(diisobutylamino)sulfonyl]amino}-3-[4-(2,6-naphtyridin-1-ylamino)phényl]propionate de méthyle
• l'acide (2*S*)-2-{[(diisobutylamino)sulfonyl]amino}-3-[4-(2,6-naphtyridin-1-ylamino)phényl]propionique
• le (2*S*)-3-{4-[(2,6-dichlorobenzoyl)amino]phényl}-2-{[(2,6-diméthylpipéridin-1-yl)sulfonyl]amino}propionate de méthyle
• l'acide (2*S*)-3-{4-[(2,6-dichlorobenzoyl)amino]-phényl}-2-{[(2,6-diméthylpipéridin-1-yl)sulfonyl]-amino}propionique
• le (2*S*)-3-{4-[(3,5-dichloroisonicotinoyl)amino]-phényl}-2-{[(diisopropylamino)sulfonyl]amino}propionate de méthyle
• l'acide (2*S*)-3-{4-[(3,5-dichloroisonicotinoyl)amino]-phényl}-2-{[(diisopropylamino)sulfonyl]amino}propionique
• le (*2S*)-3-{4-[(3,5-dichloroisonicotinoyl)amino]-phényl}-2-{[(2,6-diméthylpipéridin-1-yl)sulfonyl]-amino}propionate de méthyle
• l'acide (2*S*)-3-{4-[(3,5-dichloroisonicotinoyl)amino]-phényl}-2-{[(2,6-diméthylpipéridin-1-yl)sulfonyl]-amino}propionique
• le (2*S*)-2-{[(diméthylamino)sulfonyl]amino}-3-[4-(2,6-naphtyridin-l-ylamino)phényl]propionate de méthyle
• l'acide (2*S*)-2-{[(diméthylamino)sulfonyl]amino}-3-[4-(2,6-naphtyridin-1-ylamino)phényl]propionique
• le (2*S*)-2-{[(diisopropylamino)sulfonyl]amino}-3-[4-(2,6-naphtyridin-1-ylamino)phényl]propionate de méthyle
• l'acide (2*S*)-2-{[(diisopropylamino)sulfonyl]amino}-3-[4-(2,6-naphtyridin-1-ylamino)phényl]propionique
• le (2*S*)-2-({[cyclohexyl(isopropyl)amino]sulfonyl}amino)-3-[4-(2,6-naphtyridin-1-ylamino)phényl]-propionate de méthyle
• l'acide (2*S*)-2-({[cyclohexyl(isopropyl)amino]-sulfonyl}amino)-3-[4-(2,6-naphtyridin-1-ylamino)-phényl]propionique
• le (2*S*)-2-{[(diisopropylamino)sulfonyl]amino}-3-[4-(2,6-naphtyridin-1-yloxy)phényl]propionate de méthyle
• l'acide (2*S*)-2-{[(diisopropylamino)sulfonyl]amino}-3-[4-(2,6-naphtyridin-1-yloxy)phényl]propionique
• le (2*S*)-2-{[(diisopropylamino)sulfonyl]amino}-3-[4-(2,6-naphtyridin-1-ylamino)phényl]propionate de méthyle
• l'acide (2*S*)-2-{[(diisopropylamino)sulfonyl]amino}-3-[4-(2,6-naphtyridin-1-ylamino)phényl]propionique
• le (2*S*)-2-{[(2,6-diméthylpipéridin-1-yl)sulfonyl]-amino}-3-[4-(2,6-naphtyridin-1-ylamino)phényl]-propionate de méthyle
• l'acide (2*S*)-2-{[(2,6-diméthylpipéridin-1-yl)sulfonyl]amino}-3-[4-(2,6-naphtyridin-1-ylamino)-phényl]propionique
• le (2*S*)-2-{[(2,6-diméthylpipéridin-1-yl)sulfonyl]-amino}-3-[4-(2,6-naphtyridin-1-yloxy)phényl]-propionate de méthyle
• l'acide (2*S*)-2-{[(2,6-diméthylpipéridin-1-yl)sulfonyl]amino}-3-[4-(2,6-naphtyridin-1-yloxy)-phényl]propionique
• le (2*S*)-2-({[benzyl(isopropyl)amino]sulfonyl}amino)-3-{4-[(2,6-dichlorobenzoyl)amino]phényl}proprionate de méthyle
• l'acide (2*S*)-2-(([benzyl(isopropyl)amino]sulfonyl)amino)-3-{4-[(2,6-dichlorobenzoyl)amino]phényl}proprionique
• le (2*S*)-2-({[benzyl(isopropyl)amino]sulfonyl}amino)-3-{4-[(3,5-dichloroisonicotinoyl)amino]phényl}propionate de méthyle
• l'acide (2*S*)-2-({[benzyl(isopropyl)amino]sulfonyl}amino)-3-{4-[(3,5-dichloroisonicotinoyl)amino]-phényl}propionique
• le (2*S*)-2-({[isopropyl(thién-2-ylméthyl)amino)-sulfonyl}amino]-3-{4-[(3,5-dichloroisonicotinoyl)-amino]phényl}propionate de méthyle
• l'acide (2*S*)-2-({[isopropyl(thién-2-ylméthyl)amino]-sulfonyl}amino)-3-{4-[(3,5-dichloroisonicotinoyl)-amino]phényl}propionique
• le (2*S*)-2-({[isopropyl(thién-2-ylméthyl)amino]-sulfonyl}amino)-3-{4-[(3,5-dichlorobenzoyl)amino]-phényl}propionate de méthyle
• l'acide (2*S*)-2-({[isopropyl(thién-2-ylméthyl)amino]-sulfonyl}amino)-3-{4-[(3,5-dichlorobenzoyl)amino]-phényl}propionique
• le (2*S*)-3-{4-[(2,6-dichloroisonicotinoyl)amino]-phényl}-2-[({isobutyl[(1S)-1-phényléthyl]amino}sulfonyl)amino]propionate de méthyle
• l'acide (2*S*)-3-{4-[(2,6-dichloroisonicotinoyl)amino]-phényl}-2-[({isobutyl[(1S)-1-phényléthyl]amino}sulfonyl)amino]propionique
• le (2*S*)-2-({[cyclopentyl(isopropyl)amino]sulfonyl}amino]-3-{4-[(3,5-dichloroisonicotinoyl)amino]-phényl}propionate de méthyle
• l'acide (2*S*)-2-({[cyclopentyl(isopropyl)amino]-sulfonyl}amino]-3-{4-[(3,5-dichloroisonicotinoyl)-amino]phényl}propionique
• le (2*S*)-3-{4-[(3,5-dichloroisonicotinoyl)amino]-phényl}-2-({[isobutyl(isopropyl)amino]sulfonyl}amino)propionate de méthyle
• l'acide (2*S*)-3-{4-[(3,5-dichloroisonicotinoyl)amino]-phényl}-2-({[isobutyl(isopropyl)amino]sulfonyl}amino)propionique
• le (2*S*)-2-({[cyclohexyl(isopropyl)amino]sulfonyl)-amino)-3-[4-(2,6-naphtyridin-1-yloxy)phényl]-propionate de méthyle
• l'acide (2*S*)-2-({[cyclohexyl(isopropyl)amino]-sulfonyl}amino)-3-[4-(2,6-naphtyridin-1-yloxy)-phényl]propionique
• le (2*S*)-3-{4-[(3,5-dichloroisonicotinoyl)amino]-phényl}-2-[({isobutyl [(1R)-1-phényléthyl]amino}sulfonyl)amino]propionate de méthyle
• l'acide (2*S*)-3-{4-[(3,5-dichloroisonicotinoyl)amino]-phényl}-2-[({isobutyl[(1R)-1-phényléthyl]amino}sulfonyl)amino]propionique
• le (2*S*)-2-({[méthyl(phényl)amino]sulfonyl)amino)-3-{4-[(2,6-dichlorobenzoyl)amino]phényl}propionate de méthyle
• l'acide (2*S*)-2-({[méthyl(phényl)amino]sulfonyl}amino)-3-{4-[(2,6-dichlorobenzoyl)amino]phényl}propionique
• le (2*S*)-({[2-(phénylsulfonyl)phényl]amino}sulfonyl)-amino]-3-{4-[(3,5-dichloroisonicotinoyl)amino]-phényl}propionate de méthyle
• l'acide (2*S*)-({[2-(phénylsulfonyl)phényl]amino}sulfonyl)amino]-3-{4-[(3,5-dichloroisonicotinoyl)amino]phényl}propionique

13. Procédé pour préparer un composé de formule I tel que défini dans l'une quelconque des revendications 1 à 12, lequel procédé comprend la réaction d'une amine de formule (III) : 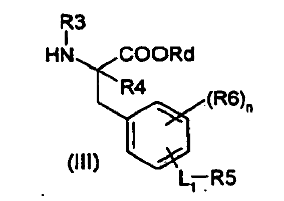 dans laquelle Rd, R3, R4, R5, R6, L1 et n sont tels que définis dans l'une quelconque des revendications 1 à 12, avec un chlorure de sulfamoyle de formule (IV) correspondant : 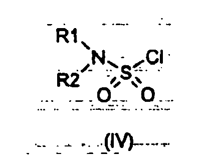 dans laquelle R1 et R2 sont tels que définis dans l'une quelconque des revendications 1 à 12.

14. Procédé pour préparer un composé de formule I tel que défini dans l'une quelconque des revendications 1 à 12, lequel procédé comprend la réaction d'une amine de formule (IIa) : 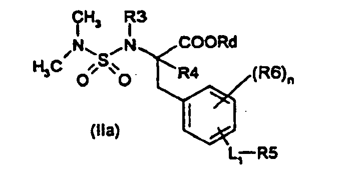 dans laquelle Rd, R3, R4, R5, R6, L1 et n sont tels que définis dans l'une quelconque des revendications 1 à 12, avec une amine de formule (V) 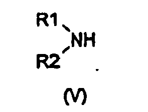 dans laquelle R1 et R2 sont tels que définis dans l'une quelconque des revendications 1 à 12.

15. Procédé pour préparer un composé de formule I tel que défini dans l'une quelconque des revendications 1 à 12, lequel procédé comprend la réaction d'une amine de formule (III) : 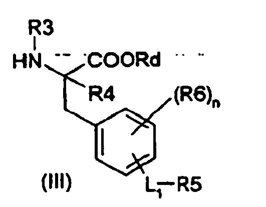 dans laquelle R3, R4, R5, R6, Rd, L1 et n sont tels que définis dans l'une quelconque des revendications 1 à 12, avec un sulfamide de formule (VII) _{:} 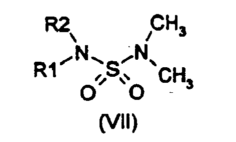 dans laquelle R1 et R2 sont tels que définis dans l'une quelconque des revendications 1 à 12.

16. Procédé pour préparer un composé de formule I tel que défini dans l'une quelconque des revendications 1 à 12, lequel procédé comprend les étapes consistant:
(a) à faire réagir une amine de formule (III) 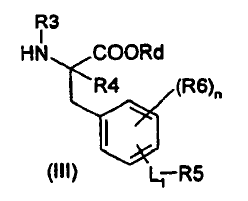 dans laquelle R3, R4, R5, R6, Rd, L1 et n sont tels que définis dans l'une quelconque des revendications 1 à 12, avec du tert-butanol et de l'isocyanate de chlorosulfonyle pour donner le sulfamide de formule (VIII) ; et 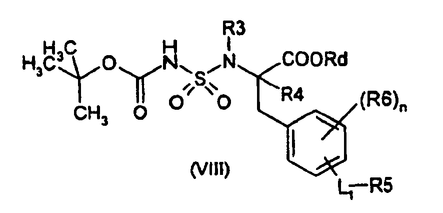
(b) à faire réagir le sulfamide de formule (VIII) avec un alcool de formule (IX).
R2-OH (IX)

17. Procédé pour préparer un composé de formule I tel que défini dans l'une quelconque des revendications 1 à 12, lequel procédé comprend la réaction d'une amine de formule (X) : 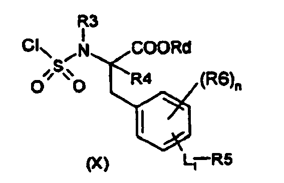 dans laquelle Rd, R3, R4, R5, R6, L1 et n sont tels que définis dans l'une quelconque des revendications 1 à 10, avec une amine de formule (V) 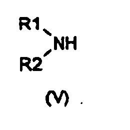 dans laquelle R1 et R2 sont tels que définis dans l'une quelconque des revendications 1 à 10.

18. Composé de formule I tel que défini dans l'une quelconque des revendications 1 à 12 ou sel pharmaceutiquement acceptable de celui-ci pour une utilisation dans un procédé de traitement du corps humain
ou animal.

19. Utilisation d'un composé de formule I tel que défini dans l'une quelconque des revendications 1 à 12 ou d'un sel pharmaceutiquement acceptable de celui-ci, dans la fabrication d'un médicament pour le traitement d'une affection pathologique ou d'une maladie qui est une sclérose en plaques, un asthme, une rhinite allergique, une conjonctivite allergique, une maladie pulmonaire inflammatoire, une arthrite rhumatoïde, une polydermatomyosite, une arthrite septique, un diabète de type I, un rejet faisant suite à une transplantation d'organe, une resténose, un rejet faisant suite à une transplantation de moelle osseuse autologue, des séquelles inflammatoires d'infections virales, une dermatite atopique, une myocardite, une maladie intestinale inflammatoire incluant une recto-colite hémorragique et la maladie de Crohn, une hypersensibilité dermique de contact, un psoriasis, une métastase tumorale, une athérosclérose et une ischémie cérébrale.

20. Composition pharmaceutique comprenant une quantité efficace d'un composé tel que défini dans l'une quelconque des revendications 1 à 12, ou d'un sel pharmaceutiquement acceptable de celui-ci, conjointement avec un véhicule pharmaceutiquement acceptable.
